# EUROPEAN PATENT APPLICATION

(11) **EP 3 366 125 A1**
(43) Date of publication of application: **29.08.2018**
(21) Application number: 18153154.2
(22) Date of filing: 07.01.2011
(51) Int. Cl.: A01K 67/027, C12N 15/85, C07K 16/00

(54) **ANIMAL MODELS AND THERAPEUTIC MOLECULES**

(30) Priority: 17.06.2010 US 355666 P; 07.07.2010 WO PCT/GB2010/051122
(62) Divisional of application: 11705964.2
(71) Applicant: Kymab Limited, Cambridge CB22 3AT (GB)
(72) Inventor: BRADLEY, Allan, Cambridge, Cambridgeshire CB22 3AT (GB); LEE, E-Chiang, Cambridge, Cambridgeshire CB22 3AT (GB); LIANG, Qi, Cambridge, Cambridgeshire CB22 3AT (GB); WANG, Wei, Cambridge, Cambridgeshire CB22 3AT (GB)
(74) Representative: CMS Cameron McKenna Nabarro Olswang LLP

(57) **Abstract**

The invention discloses methods for the generation of chimeric human - non human antibodies and chimaeric antibody chains, antibodies and antibody chains so produced, and derivatives thereof including fully humanised antibodies; compositions comprising said antibodies, antibody chains and derivatives, as well as cells, non-human mammals and vectors suitable for use in said methods.

SEQUENCE LISTING

<110>  Kymab Limited

<120>  Animal models and therapeutic molecules

<130>  P/25077.42

<150> EP11705964.0
<151> 07.01.2011

<150> PCT/GB2011/050019
<151> 07.01.2011

<150> US61/355,666
<151> 17.06.2010

<150> PCT/GB2010/051122
<151> 07.07.2010

<160>  17

<170>  PatentIn version 3.5

<210>  1
<211>  4272
<212>  DNA
<213>  Rattus norvegicus

<400>  1
agatctgccc atctcaggct agttaaatta gttcatccca gtttggccca acttacccca      60

tctagagtag cgaaactaat ctgagcctag ctaagtccag tttagtttaa tgtagcccag     120

cttggcacag gctaatacat actgctacag tttggtctag cctaccctaa ttaagctgat     180

ccaggcctgg gtagacctag ctcatctcag cccagttaag gttatccagt acatctcttt     240

ccagttcagc tcaggttacc ataccttatc tcaattcagc tcagctagtg taattcatct     300

tagttcatcc cctacccctc tagactccct gttgatctta actcagttta gacatggcca     360

acaaagcctg gcccaactca ggccaggtta gtgtagctca gcataagcag tctagccttg     420

ctcagtctag ctcacccttc ctcatctaaa ttcaactcag ctatgccggc cctgcagcag     480

gtcccctcag ctcacccaag tccaaccagt tcagtctggc tcatttaagt cttgacaatc     540

cccaattcat cccagctcag cttagcataa ctcaggcagt ccattcttag cccaacccag     600

tttagcccag tttatcccag ttcatcctgg ctgtactcag tgcaactcga ttcatgttct     660

cccaggccac ctcagcccag ttcatggtag ctcatctgag cccaacttat cccagctcat     720

cccaaaccac ctcacctaag ccctgctcag cctagctcat ctgagcctag ttcaacctct     780

ctcatcctgc cagctagccc agtttagtcc acatcatctt gcaaagctca accagcccaa     840

gtcagccggg tccagctcat tcatgtccaa accagctcag tcatgctcat cctaactcag     900

cctcaccatc atccacatca gctagcccag ttcagctgag ctcatcccag cccacttcaa     960

tcacagctca tttaagtaca gctcacccca gctctattta gctcaagcta gcttatttag    1020

cctacttcat cccagctcag cccagccaac tcaactcatc ctagctcagc taaaccctgc 1080

tcagctcacc caagcaaagc tgactccaac ccagatcctt tcagctcagc tcacccagct 1140

caggccagct cacccatccc agctcaccca gcttagctca cccagcccag ctcagcccag 1200

ctcacccagc ccagctcagc ccagctcacc cagcccagct cagcccagct cagctcagct 1260

cagctcagct cagctcagct cacccagctc agctcagcca gctcagctca ccccagctca 1320

gtccagctca gttcagctca ccccagctca gctcacccaa ctcagctcac tcaactcagc 1380

tcacccaact cagctcagct cagttcaccc agctcagctc acccagccca gcacagctca 1440

tctcacccag ctcagctcac ccagcccagc tcaccccagc tcaccccagc tcagctcagc 1500

tcaccccagc tcagcccagc tcagctcacc cagctcagct cacccaactc agctcagctc 1560

agttcaccca gctcagctca cccagcccag cacagctcat ctcacccagc ccagctcacc 1620

ccagctcacc ccagctcagc tcagctcagc ccagctcacc cagctcagct cagctcaccc 1680

cagctcagct cacccagctc agctcaccca gcccagctca gctcagctca ccccagctca 1740

gcccagctca gctcacccag ctcagctcac ccagcccagc tcaccccagc tcaccccagc 1800

tcagtccagc tcagttcagc tcacccagct cagctcaccc aactcaactc agctcagttc 1860

acccagctca gctcagctca ccccagctca ccccagctca cccagctcag ttcagctcac 1920

cccagctcag ttcacccagc tcagctcacc cagcccagct cagcccagct caccccagct 1980

cagctcaacc agatcagctc agcccagctc accctagttt agttcaccca gcccagctca 2040

ccccagctca gctcacccca actcagctca cccagctcat cccagctcag ccagctaatc 2100

ccagctcagc tcaccccagc tcagctcacc cagctcagct cacccaactc agctcacccc 2160

agctcacccc agctcatccc agctcatccc agttcagacc tgttcagctc atctcacccc 2220

agctcagctc accccagttc agctcaccta gcccaactca ccccagctca gtccagctca 2280

gttcagctca ccccaactca tctcacccag ctcagctcac cccagctcat cccagctcag 2340

ctcaccccag ttcagccctg ttcagctcat ctcacccagc tcagctcatc cagcccagct 2400

caccccagct caccccagct cagtccagct cagttcagct cacccagctc agctcaccca 2460

actcaactca gctcagttca cccagctcag ctcagctcac cccagctcac ccagctcagt 2520

tcagctcacc ccagctcagt tcacccagct cagctcaccc agcccagctc aaccagatca 2580

gctcagccca gctcacccta gtttagttca cccagcccag ctcaccccag ctcagctcac 2640

cccaactcag ctcacctagc tcatcccagc tcagctcacc ccagctcagc tcaccccagc 2700

tcatctcacc ccagctcagc tcacccagct catcccagct cagctcagcc cagctcatcc 2760

cagccctgct catcccagct cagctcagct cagcccagct cagcccagct cagcccagct 2820

cagcccagct cagcccagct cagctcaacc cagctcagct cacccagccc agctcagccc 2880

agctcaccca gctcagctca ccccagctca gctcacccca gctcatctca cccagctcag 2940

ctcacccagc tcagcccagc tcagctcagc tcacccagct catctcaccc agctcagctc 3000

accccagctc atcccagctc agctcacccc agttcagccc tgttcagctc atctcacccc 3060

agctcagctc acccagttca gctcatccca gcccatccca gctcagctca gcccagctca 3120

gcccagctca gcccagccca gcccagccca gctcagctca gcccagctca gcccagctca 3180

gtccagctca gcttagccca gcccagctca gctcagccca gctcagccca gctcagccca 3240

gctcagctca cccagctcac cccagctcag cccagctcag cccagctcag ctcacccagc 3300

tcaccccacc ccagctcacc ccagttcagc ccagctcagc ccagctcagc ccagcccagc 3360

ccagcccagc ccagcccagc tcagcccagc tcagctcagc ccagcccagc tcagctcagc 3420

ccagctcagc ccagctcatc ccagctcagc tcaccccagc tcagcccagc tcagcccagc 3480

tcagctcacc cagctcaccc caccccagct caccccagtt cagcccagct catccagctc 3540

agctcacccc cagctctgct cacccagctc agctcagctt acccagctca gctcaactca 3600

cccagctcag ctcacccagc tcagctcagc tcaccccagc ccagctcagc tcagctcacc 3660

ccagctctgc tcacccagct cagctcagct cacctcagct ctgctcaccc agctcagctc 3720

aaccacctca ggtcagccca gctcacccca gcttacccca gctcacccag ctcagctcag 3780

ctcacccagc tcagctcacc cagctcagct caccccagct taccccagct caccccagct 3840

cagctaaccc agctcagctc acccagctca gctcacccag ctcagctcat cccagctcac 3900

cccagctacc acagagtagc tcatgctagc tcagctcacc ccagcacaac acagcccaac 3960

acagctcagt tcagagcagt ccagtagagt ttagctccaa tcagcccaga tcaagacaat 4020

tcattccaat ttggctatct tggttaagtc agcctagttt agcttagccg gcctagctca 4080

attcagctca ttgcagtcta cctcgttcct gctcaagtcc agctttggct acctcagagt 4140

aatcatctca gcttagcaca tttttgaagg gctcagggaa gcctacacat ctcagtccaa 4200

ctgtgcttaa ctagagccta gcttcctagc caggctgtca accttgttca ctaaattttg 4260

ctcagcaagc tt 4272

<210> 2
<211> 22190
<212> DNA
<213> Artificial Sequence

<220>
<223> Tragetting Vector (long version)

<400> 2
gcggccgcaa cctgggcaaa tgggagctta gcaacaatgt agggggctgg acctagactt 60

cctacacatt tgtagcagat gtgcagcttg gtcttcatgt gtgtattacc ctaacatttg 120

gagcaggagc tgtctctgac tctgttgcct gccattggat ccccttcccc tgcttgggct 180

gccttgtttg gccttagtag gaaaggatgt gcttagtcct gctgtgactt gatgtcccta 240

ggcagaatga taccccaggg gggctcccca tctctgagga gatgggcaaa gggtaatggt 300

tggagggact tgtgaggctg ggactgggag gagagaaagg agacagctgt aactggaatg 360

atgttaagtg aacaaatgaa tggatagatt agatagacag atagacagac agacagacag 420

acagacagac agacagacag acagatagaa agatagatag ataaggggaa aaagaaacgt 480

agctgagcaa gccagagaga gcaagccaaa taagcagcat tcctccatga cttttccttc 540

agctcctgcc tatgagtctg ccttgacttc cctcagtgat tggttgtaag ttaaaaggtg 600

aaataaaccc tttctttgac aagttgcttt tggttctgat ttttatcaca gcaagagaaa 660

atcaaactag aacaaacatg tatttttcct ggcacatgtc catagtaagg cagaaatgat 720

cttcagacct agaccataga tactacagag agcagaagtg tagataggtg gacttactgt 780

atgattgtaa tccaagtaaa tctacatagc tagagagcta gaggaaaggc caaagcttcc 840

tctgggaggt cagatcctgt cgcactgtag ccaataaggc atattgcatc acaggaaagg 900

actaagaccc aggctggcaa tagtgtctgt atcttaacta gacctctcta gtgagtgagg 960

aaggaagttt gtgagagccc agactgtggg ctcggaaggt acctgccatg cccctgttag 1020

taactgagta ctacagcagg agcaggtgtt ctctagaaag cctgagacaa ctctacttct 1080

tctctcaaga gaccacctaa tacaggcctg agagaacaga ctctggaaat agatgggact 1140

taaggagcta agatctagag ctcatctaca gagcagaatc ccagccaaga gaacaaagaa 1200

tactggctct ctctcctgtt ccctactcct agagttctaa aacacactat agggaaggga 1260

gcctctagac ctccgtccat tccccatctt gctcattcca tcttcccatg tccccaggtc 1320

tccaagccac agacactacc tttcctattc acccaccttt ctgtgtccct aggtccccag 1380

gccatagtca cctcccccca cacacacccc actcaccctg ccccatctat gcccctagat 1440

gcttacttac cagagtcttt tgtctgacgt ggggctacaa gcatctatgc tccctaagca 1500

cctactgctg acctgtagga cccagctctg aaccaactca tataagtaaa tacagactct 1560

cccctgtctt aggatggcct cctggatcag gaggagacca ctgccaaaga accttctctc 1620

agagcactga actcctcccc tgtaccactt aggacagacc tgagacctat tattactgat 1680

taccagagct ctggcagtga ccacggagga gataggtcca ccctggacac aggaaacaca 1740

gcagcagaga tactgctcca tcacaacagt agagtgacac tttagacttt aatttgggtc 1800

actttcctgc tgcagaggtg ggatcagaaa gcaaagagca gtatgagtgc ctgataggca 1860

cccaagtaca ctatagagta ctcatggtga ataaggtacc tccatggctt cccagggagg 1920

ggcactgccc cacccccacc atcacagacc tttctccata gttgataact cagacacaag 1980

tgaatgacag atggacctcc atctactctt attttaaaaa gaagacaaac cccacaggct 2040

cgagaacttt agcgactgtt ttgagagaaa tcattggtcc ctgactcaag agatgactgg 2100

cagattgggg atcagaatac ccatactctg tggctagtgt gaggtttaag cctcagagtc 2160

cctgtggtct ctgactggtg caaggttttg actaagcgga gcaccacagt gctaactggg 2220

accacggtga cacgtggctc aacaaaaacc ttctgtttgg agctctccag gggcagcctg 2280

agctatgagg aagtagagag gcttgagaaa tctgaggaag aaaagagtag atctgagagg 2340

aaaggtagct ttctggaggt caggagacag tgcagagaag aacgagttac tgtggacagg 2400

tcttagatgg ggaaagaatg agcaaatgca agcatcagaa gggtggatgc aatgtcctgc 2460

caaggactta ccaagaggat ccccggacag agcaggcagg tggagttgac tgagaggaca 2520

gggtaggtgc aggtccctct ctcgtttcct ttctccttct cctgtttcct tcctctcttg 2580

tcacaggtct cactatgcta gccaaggcta gcctgaaaga ttaccatcct acagatgggc 2640

ccatccagtt gagttaaggt ggagatctct ccaaacatct gagtttctga ggcttggatg 2700

ccactgggga cgccaaggga ctttggctgg ggtttggttg gccccagatg aaggctact 2760

tcactgggtc tataattact ctgatgtcta ggaccagggg gctcaggtca ctcaggtcag 2820

gtgagtcctg catctgggga ctgtggggtt caggtgtcct aaggcaggat gtggagagag 2880

ttttagtata ggaacagagg cagaacagag actgtgctac tggtacttcg atgtctgggg 2940

cgcagggacc acggtcaccg tctcctcagg taagctggct tttttctttc tgcacattcc 3000

attctgaaat gggaaaagat attctcagat ctccccatgt caggccatct gccacactct 3060

gcatgctgca gaagcttttc tgtaaggata gggtcttcac tcccaggaaa agaggcagtc 3120

agaggctagc tgcctgtgga acagtgacaa tcatggaaaa taggcattta cattgttagg 3180

ctacatgggt agatgggttt ttgtacaccc actaaagggg tctatgatag tgtgactact 3240

ttgactactg gggccaaggc accactctca cagtctcctc aggtgagtcc ttacaacctc 3300

tctcttctat tcagcttaaa tagattttac tgcatttgtt gggggggaaa tgtgtgtatc 3360

tgaatttcag gtcatgaagg actagggaca ccttgggagt cagaaagggt cattgggagc 3420

cctggctgat gcagacagac atcctcagct cccagacttc atggccagag atttataggg 3480

atcctggcca gcattgccgc taggtccctc tcttctatgc tttctttgtc cctcactggc 3540

ctccatctga gatcatcctg gagccctagc caaggatcat ttattgtcag gggtctaatc 3600

attgttgtca caatgtgcct ggtttgctta ctggggccaa gggactctgg tcactgtctc 3660

tgcaggtgag tcctaacttc tcccattcta aatgcatgtt ggggggattc tgagccttca 3720

ggaccaagat tctctgcaaa cgggaatcaa gattcaaccc ctttgtccca aagttgagac 3780

atgggtctgg gtcagggact ctctgcctgc tggtctgtgg tgacattaga actgaagtat 3840

gatgaaggat ctgccagaac tgaagcttga agtctgaggc agaatcttgt ccagggtcta 3900

tcggactctt gtgagaatta ggggctgaca gttgatggtg acaatttcag ggtcagtgac 3960

tgtctggttt ctctgaggtg aggctggaat ataggtcacc ttgaagacta aagaggggtc 4020

caggggcttc tgcacaggca gggaacagaa tgtggaacaa tgacttgaat ggttgattct 4080

tgtgtgacac caggaattgg cataatgtct gagttgccca ggggtgattc tagtcagact 4140

ctggggtttt tgtcgggtat agaggaaaaa tccactattg tgattactat gctatggact 4200

actggggtca aggaacctca gtcaccgtct cctcaggtaa gaatggcctc tccaggtctt 4260

tatttttaac ctttgttatg gagttttctg agcattgcag actaatcttg gatatttgtc 4320

cctgagggag ccggctgaga gaagttggga aataaactgt ctagggatct cagagccttt 4380

aggacagatt atctccacat ctttgaaaaa ctaagaatct gtgtgatggt gttggtggag 4440

tccctggatg atgggatagg gactttggag gctcatttga gggagatgct aaaacaatcc 4500

tatggctgga gggatagttg gggctacgcg tttttaaccc tagaaagata gtctgcgtaa 4560

aattgacgca tgcattcttg aaatattgct ctctctttct aaatagcgcg aatccgtcgc 4620

tgtgcattta ggacatctca gtcgccgctt ggagctcccg tgaggcgtgc ttgtcaatgc 4680

ggtaagtgtc actgattttg aactataacg accgcgtgag tcaaaatgac gcatgattat 4740

cttttacgtg acttttaaga tttaactcat acgataatta tattgttatt tcatgttcta 4800

cttacgtgat aacttattat atatatattt tcttgttata gatatcgcta gtggatccgg 4860

ctggttcttt ccgcctcaga aggtactttt tttttttttt tttttttttt tttttttttt 4920

tttttttttt tttttttttt ttttttaaat ttttgggaat ttattgattt gcatttaaaa 4980

gggaactgct gacaaagatt cactggtaat aatttgaaca agttggaaaa tacagtcaac 5040

attactgaaa cactactaaa ataattccag gacagaacaa aacttcttag atgctgtctt 5100

tgatgtgaaa attgactgct tcttactttt ctaacacacg gtggtataat taacaatatt 5160

caatcacttc tattctttcc tgcatatata aaaattaaaa taccaattaa aaaactaata 5220

tatcttctct ttatttctta cagatatgag ttcaatgttt cactcaatag tgctgtggtt 5280

taagagaatt ttttcattta caagttaaac aacaatccgc ccaaagggaa ctgatagtct 5340

ataggctcat agtgcaaata aacagtttag gaatgcagca actgacattt ctaaagtaca 5400

aaacagataa aattcttaga agatacatgc aaaaagctct actaagcaga tggccacaga 5460

actagaacat tgataatttt actggcgatg tcaataggac tccagatgtt tccaaactca 5520

acttgaactc tcatcttagg ctttgtattt tgcttttcca gtttcactaa tgacacaaac 5580

atgattcaaa tccctgaagt attcattata gtcaagggca tatcctacaa caaacttgtc 5640

tggaatttca aatccaacaa agtctggctt atatccaaca cttcgtgggg tccttttcac 5700

cagcaagctt gcgaccttga ccatctttgg attatactgc ctgaccaagg aaagcaaagt 5760

ctgcattgtt ttgccagtgt caattatatc ttccacaatc aagacattct ttccagttaa 5820

agttgagaga tcatctccac caattacttt tatgtcccct gttgactggt cattacaata 5880

gctcttcagt ctgataaaat ctacagtcat aggaatggat ctatcactat ttctattcag 5940

tgctttgatg taatccagca ggtcagcaaa gaatttatag ccccccttga gcacacagag 6000

ggctacaatg tgatggcctc ccatctcctt catcacatct cgagcaagac gttcagtcct 6060

acagaaataa aatcaggaat ttaatagaaa gtttcataca ttaaacttta taacaaacac 6120

ctcttagtca ttaaacttcc acaccaacct gggcaatata gtgagacccc atgcctgcaa 6180

aaaaaaaaaa attagccagg catggtagca tgtacctgta gtcccagcta cttgagaggt 6240

gaggtgggaa aatcacttta gtgcaggatg ttgaggctgg agtgaactgt gattgtgcca 6300

ctgcactcca gcctggacaa tagagcaaga ccttgtctca aaaaaatgca ttaaaaattt 6360

tttttaaatc ttccacgtaa cacatccttt gccctcatgt ttcataaggt aaaaaatttg 6420

ataccttcaa aaaaaccaag cataccacta tcataatttt ttttaaatgc aaataaaaac 6480

aagataccat tttcacctat cagactggca ggttctgatt aaatgaaatt tcttggataa 6540

tatacaatat taagagagac tgtagaaact gggccagtgg ctcatgcctg taatcccagc 6600

actttgggag gctgggtaac atggcgaacc ctgtttctac aaaataaaaa tattagctgg 6660

gagtggtggc gcacacctat agtcccagct actcaggagg ctgaggtgga aggatcgctt 6720

gaacccagga ggttgagact gcagtgaact gtgatcattc tgctgcactg caccccagcc 6780

tgggcaacag agaccttgtc tcaaaaaaaa aaaaaaaaga gacaaattgt gaagagaaag 6840

gtactctcat ataacatcag gagtataaaa tgattcaact tcttagagga aaatttggca 6900

ataccaaaat attcaataaa ctctttcccc ttgacccaga aattccactt gaataaagct 6960

gaacaagtac caaacatgta aaagaatgtt tcttctagta cagtcggtaa gaacaaaata 7020

gtgtctatca atagtggact ggttaaatca gttatggtat ctccataaga cagaatgcta 7080

tgcaaccttt aaaatatatt agatagctct agacagtgga tcccctcgag ggacctaata 7140

acttcgtata gcatacatta tacgaagtta tattaagggt tattgaatat gtcgactaga 7200

cacactaata ttaaaagtgt ccaataacat ttaaaactat actcatacgt taaaatataa 7260

atgtatatat gtacttttgc atatagtata catgcatagc cagtgcttga gaagaaatgt 7320

gtacagaagg ctgaaaggag agaactttag tcttcttgtt tatggcctcc atagttagaa 7380

tattttataa cacaaatatt ttgatattat aattttaaaa taaaaacaca gaatagccag 7440

acatacaatg caagcattca ataccaggta aggtttttca ctgtaattga cttaacagaa 7500

aattttcaag ctagatgtgc ataataataa aaatctgacc ttgccttcat gtgattcagc 7560

cccagtccat taccctgttt aggactgaga aatgcaagac tctggctaga gttccttctt 7620

ccatctccct tcaatgttta ctttgttctg gtccctacag agtcccacta taccacaact 7680

gatactaagt aattagtaag gccctcctct tttattttta ataaagaaga ttttagaaag 7740

catcagttat ttaataagtt ggcctagttt atgttcaaat agcaagtact cagaacagct 7800

gctgatgttt gaaattaaca caagaaaaag taaaaaacct cattttaaga tcttacttac 7860

ctgtccataa ttagtccatg gggaataaac accctttcca aatcctcagc ataatgatta 7920

ggtatgcaaa ataaatcaag gtcataacct ggttcatcat cactaatcac gacgccaggg 7980

ctgcgggtcg ccataacgga gccggccggc gcgcgggctg aataacttcg tataatgtgt 8040

actatacgaa gttatttgtt caggaggagg aagccggtgg cggagcagag gaggaggcgg 8100

aggcgcagca agaccccccc ccccctgcag gtcgaaaggc ccggagatga ggaagaggag 8160

aacagcgcgg cagacgtgcg cttttgaagc gtgcagaatg ccgggcctcc ggaggacctt 8220

cgggcgcccg ccccgcccct gagcccgccc ctgagcccgc ccccggaccc accccttccc 8280

agcctctgag cccagaaagc gaaggagcaa agctgctatt ggccgctgcc ccaaaggcct 8340

acccgcttcc attgctcagc ggtgctgtcc atctgcacga gactagtgag acgtgctact 8400

tccatttgtc acgtcctgca cgacgcgagc tgcggggcgg gggggaactt cctgactagg 8460

ggaggagtag aaggtggcgc gaaggggcca ccaaagaacg gagccggttg gcgcctaccg 8520

gtggatgtgg aatgtgtgcg aggccagagg ccacttgtgt agcgccaagt gcccagcggg 8580

gctgctaaag cgcatgctcc agactgcctt gggaaaagcg cctcccctac ccggtagata 8640

tctataacaa gaaaatatat atataataag ttatcacgta agtagaacat gaaataacaa 8700

tataattatc gtatgagtta aatcttaaaa gtcacgtaaa agataatcat gcgtcatttt 8760

gactcacgcg gtcgttatag ttcaaaatca gtgacactta ccgcattgac aagcacgcct 8820

cacgggagct ccaagcggcg actgagatgt cctaaatgca cagcgacgga ttcgcgctat 8880

ttagaaagag agagcaatat ttcaagaatg catgcgtcaa ttttacgcag actatctttc 8940

tagggttaaa agaattcgat atcaagctta tcgatgtagt tggagatttt cagtttttag 9000

aataaaagta ttagttgtgg aatatacttc aggaccacct ctgtgacagc atttatacag 9060

tatccgatgc atagggacaa agagtggagt ggggcacttt ctttagattt gtgaggaatg 9120

ttccgcacta gattgtttaa aacttcattt gttggaagga gagctgtctt agtgattgag 9180

tcaagggaga aaggcatcta gcctcggtct caaaagggta gttgctgtct agagaggtct 9240

ggtggagcct gcaaaagtcc agctttcaaa ggaacacaga agtatgtgta tggaatatta 9300

gaagatgttg cttttactct taagttggtt cctaggaaaa atagttaaat actgtgactt 9360

taaaatgtga gagggttttc aagtactcat ttttttaaat gtccaaaatt tttgtcaatc 9420

aatttgaggt cttgtttgtg tagaactgac attacttaaa gtttaaccga ggaatgggag 9480

tgaggctctc tcatacccta ttcagaactg acttttaaca ataataaatt aagtttaaaa 9540

tatttttaaa tgaattgagc aatgttgagt tggagtcaag atggccgatc agaaccagaa 9600

cacctgcagc agctggcagg aagcaggtca tgtggcaagg ctatttgggg aagggaaaat 9660

aaaaccacta ggtaaacttg tagctgtggt ttgaagaagt ggttttgaaa cactctgtcc 9720

agccccacca aaccgaaagt ccaggctgag caaaacacca cctgggtaat ttgcatttct 9780

aaaataagtt gaggattcag ccgaaactgg agaggtcctc ttttaactta ttgagttcaa 9840

ccttttaatt ttagcttgag tagttctagt ttccccaaac ttaagtttat cgacttctaa 9900

aatgtattta gaattcattt tcaaaattag gttatgtaag aaattgaagg actttagtgt 9960

ctttaatttc taatatattt agaaaacttc ttaaaattac tctattattc ttccctctga 10020

ttattggtct ccattcaatt cttttccaat acccgaagca tttacagtga ctttgttcat 10080

gatctttttt agttgtttgt tttgccttac tattaagact ttgacattct ggtcaaaacg 10140

gcttcacaaa tctttttcaa gaccactttc tgagtattca ttttaggaga aatacttttt 10200

ttttaaatga atgcaattat ctagacttat ttcagttgaa catgctggtt ggtggttgag 10260

aggacactca gtcagtcagt gacgtgaagg gcttctaagc cagtccacat gctctgtgtg 10320

aactccctct ggccctgctt attgttgaat gggccaaagg tctgagacca ggctgctgct 10380

gggtaggcct ggactttggg tctcccaccc agacctggga atgtatggtt gtggcttctg 10440

ccacccatcc acctggctgc tcatggacca gccagcctcg gtggctttga aggaacaatt 10500

ccacacaaag actctggacc tctccgaaac caggcaccgc aaatggtaag ccagaggcag 10560

ccacagctgt ggctgctgct cttaaagctt gtaaactgtt tctgcttaag agggactgag 10620

tcttcagtca ttgctttagg gggagaaaga gacatttgtg tgtcttttga gtaccgttgt 10680

ctgggtcact cacatttaac tttccttgaa aaactagtaa aagaaaaatg ttgcctgtta 10740

accaataatc atagagctca tggtattttg aggaaatctt agaaaacgtg tatacaattg 10800

tctggaatta tttcagttaa gtgtattagt tgaggtactg atgctgtctc tacttcagtt 10860

atacatgtgg gtttgaattt tgaatctatt ctggctcttc ttaagcagaa aatttagata 10920

aaatggatac ctcagtggtt tttaatggtg ggtttaatat agaaggaatt taaattggaa 10980

gctaatttag aatcagtaag gagggaccca ggctaagaag gcaatcctgg gattctggaa 11040

gaaaagatgt ttttagtttt tatagaaaac actactacat tcttgatcta caactcaatg 11100

tggtttaatg aatttgaagt tgccagtaaa tgtacttcct ggttgttaaa gaatggtatc 11160

aaaggacagt gcttagatcc aaggtgagtg tgagaggaca ggggctgggg tatggatacg 11220

cagaaggaag gccacagctg tacagaattg agaaagaata gagacctgca gttgaggcca 11280

gcaggtcggc tggactaact ctccagccac agtaatgacc cagacagaga aagccagact 11340

cataaagctt gctgagcaaa atttagtgaa caaggttgac agcctggcta ggaagctagg 11400

ctctagttaa gcacagttgg actgagatgt gtaggcttcc ctgagccctt caaaaatgtg 11460

ctaagctgag atgattactc tgaggtagcc aaagctggac ttgagcagga acgaggtaga 11520

ctgcaatgag ctgaattgag ctaggccggc taagctaaac taggctgact taaccaagat 11580

agccaaattg gaatgaattg tcttgatctg ggctgattgg agctaaactc tactggactg 11640

ctctgaactg agctgtgttg ggctgtgttg tgctggggtg agctgagcta gcatgagcta 11700

ctctgtggta gctggggtga gctgggatga gctgagctgg gtgagctgag ctgggtgagc 11760

tgagctgggt tagctgagct ggggtgagct ggggtaagct ggggtgagct gagctgggtg 11820

agctgagctg ggtgagctga gctgagctgg gtgagctggg gtaagctggg gtgagctggg 11880

ctgacctgag gtggttgagc tgagctgggt gagcagagct gaggtgagct gagctgagct 11940

gggtgagcag agctggggtg agctgagctg agctgggctg gggtgagctg agctgagctg 12000

ggtgagctga gctgggtgag ttgagctgag ctgggtaagc tgagctgagc tgggtgagca 12060

gagctggggg tgagctgagc tggatgagct gggctgaact ggggtgagct ggggtggggt 12120

gagctgggtg agctgagctg ggctgagctg ggctgagctg gggtgagctg agctgggatg 12180

agctgggctg agctgggctg agctgagctg ggctgggctg agctgagctg ggctgagctg 12240

ggctgggctg ggctgggctg ggctgggctg agctgggctg agctgggctg aactggggtg 12300

agctggggtg gggtgagctg ggtgagctga gctgggctga gctgggctga gctggggtga 12360

gctgggtgag ctgagctggg ctgagctggg ctgagctggg ctgagctgag ctgggctggg 12420

ctaagctgag ctggactgag ctgggctgag ctgggctgag ctgagctggg ctgggctggg 12480

ctgggctgag ctgggctgag ctgggctgag ctgagctggg atgggctggg atgagctgaa 12540

ctgggtgagc tgagctgggg tgagatgagc tgaacagggc tgaactgggg tgagctgagc 12600

tgggatgagc tggggtgagc tgagctgggt gagatgagct gggtgagctg agctgagctg 12660

ggctgagctg ggtgagctga gctgggtgag atgagctggg gtgagctgag ctggggtgag 12720

ctgagctggg tgagctgggc tgagctgggc tgggtgagct gagctgggtt gagctgagct 12780

gggctgagct gggctgagct gggctgagct gggctgagct gggctgagct gagctgagct 12840

gggatgagca gggctgggat gagctgggct gagctgagct gggatgagct gggtgagctg 12900

agctggggtg agatgagctg gggtgagctg agctggggtg agctgagctg ggatgagcta 12960

ggtgagctga gttggggtga gctgagctgg ggtgagctgg gctgggtgaa ctaaactagg 13020

gtgagctggg ctgagctgat ctggttgagc tgggctgggt gagctgagct gggtgaactg 13080

agctggggtg agctgaactg agctgggtga gctggggtga gctgagctga gctgggtgaa 13140

ctgagctgag ttgagttggg tgagctgagc tgggtgagct gaactgagct ggactgagct 13200

ggggtgagct ggggtgagct gggctggatg agctgagctg ggtgagatga gctgaacagg 13260

gctgaactgg ggtgagctga gctgggatga gctggggtga gctgagctgg gtgagatgag 13320

ttggggtgag ctgaactgag ctggactgag ctggggtgag ttgggctagg tgagctgaac 13380

tggggtgagc tgagctgggg tgagatgagc tgaacaggtc tgaactggga tgagctggga 13440

tgagctgggg tgagctgggg tgagctgagt tgggtgagct gagctgggtg agctgagctg 13500

gggtgagctg agctgggatt agctggctga gctgggatga gctgggtgag ctgagttggg 13560

gtgagctgag ctggggtgag ctgggctggg tgaactaaac tagggtgagc tgggctgagc 13620

tgatctggtt gagctgagct ggggtgagct gggctgagct gggctgggtg agctgagctg 13680

ggtgaactga gctggggtga gctgaactga gctgggtgag ctggggtgag ctggggtgag 13740

ctgagctgag ctgggtgaac tgagctgagt tgagttgggt gagctgagct gggtgagctg 13800

aactgagctg gactgagctg gggtgagctg gggtgagctg ggctgggtga gctgagctgg 13860

gtgagctgag ctgggctgag ctggggtgag ctgagctgag ctgggctggg tgagctgagc 13920

tgggtgagct gagctggggt gagctgagct gagctgggtg agctgggctg agctgagctg 13980

agctggggtg agctggggtg agctgggctg ggtgagatga gctgtgctgg gctgggtgag 14040

ctgagctggg tgaactgagc tgagctgagt tgggtgagct gagctgggtg agctgagctg 14100

ggctgagctg gggtgagctg agctgagctg gggtgagctg gggtgagctg ggctgggtga 14160

gctgagctgg gtgagatgag ctgtgctggg ctgggtgagc tgagctgggt gaactgagct 14220

gagctgagtt gggtgagctg agttgagtga gctgagttgg gtgagctgag ctggggtgag 14280

ctgaactgag ctggactgag ctggggtgag ctgagctggc tgagctgagc tgggtgagct 14340

gagctgagct gagctgagct gagctgagct gggctgagct gggctgggtg agctgggctg 14400

agctgggctg ggtgagctgg gctgagctgg gctgggtgag ctaagctggg tgagctggga 14460

tgggtgagct ggcctgagct gggtgagctg agctgaaagg atctgggttg gagtcagctt 14520

tgcttgggtg agctgagcag ggtttagctg agctaggatg agttgagttg gctgggctga 14580

gctgggatga agtaggctaa ataagctagc ttgagctaaa tagagctggg gtgagctgta 14640

cttaaatgag ctgtgattga agtgggctgg gatgagctca gctgaactgg gctagctgat 14700

gtggatgatg gtgaggctga gttaggatga gcatgactga gctggtttgg acatgaatga 14760

gctggacccg gctgacttgg gctggttgag ctttgcaaga tgatgtggac taaactgggc 14820

tagctggcag gatgagagag gttgaactag gctcagatga gctaggctga gcagggctta 14880

ggtgaggtgg tttgggatga gctgggataa gttgggctca gatgagctac catgaactgg 14940

gctgaggtgg cctgggagaa catgaatcga gttgcactga gtacagccag gatgaactgg 15000

gataaactgg gctaaactgg gttgggctaa gaatggactg cctgagttat gctaagctga 15060

gctgggatga attggggatt gtcaagactt aaatgagcca gactgaactg gttggacttg 15120

ggtgagctga ggggacctgc tgcagggccg gcatagctga gttgaattta gatgaggaag 15180

ggtgagctag actgagcaag gctagactgc ttatgctgag ctacactaac ctggcctgag 15240

ttgggccagg ctttgttggc catgtctaaa ctgagttaag atcaacaggg agtctagagg 15300

ggtaggggat gaactaagat gaattacact agctgagctg aattgagata aggtatggta 15360
acctgagctg aactggaaag agatgtactg gataacctta actgggctga gatgagctag 15420
gtctacccag gcctggatca gcttaattag ggtaggctag accaaactgt agcagtatgt 15480
attagcctgt gccaagctgg gctacattaa actaaactgg acttagctag gctcagatta 15540
gtttcgctac tctagatggg gtaagttggg ccaaactggg atgaactaat ttaactagcc 15600
tgagatgggc agatctgaat gagcagagct gggatgaact gaatgagttt caccaggcct 15660
ggaccagtta ggctaggacc tcgttctata gaggcagact gtgtgctaca gtggagtttc 15720
aagatgattc catgagtcct ccccgccccc aacataaccc accttcctcc taccctacaa 15780
gcctgtctgg tgtgtaaatc ccagctttgt gtgctgatac agaagcctga gcccctcccc 15840
cacctccacc tacctattac tttgggatga gaatagttct cccagccagt gtctcagagg 15900
gaagccaagc aggacaggcc caaggctact tgagaagcca ggatctaggc ctctccctga 15960
gaacgggtgt tcatgcccct agagttggct gaagggccag atccacctac tctagaggca 16020
tctctccctg tctgtgaagg cttccaaagt cacgttcctg tggctagaag gcagctccat 16080
agccctgctg cagtttcgtc ctgtatacca ggttcaccta ctaccatatc tagccctgcc 16140
tgccttaaga gtagcaacaa ggaaatagca gggtgtagag ggatctcctg tctgacagga 16200
ggcaagaaga cagattctta cccctccatt tctcttttat ccctctctgg tcctcagaga 16260
gtcagtcctt cccaaatgtc ttccccctcg tctcctgcga gagccccctg tctgataaga 16320
atctggtggc catgggctgc ctggcccggg acttcctgcc cagcaccatt tccttcacct 16380
ggaactacca gaacaacact gaagtcatcc agggtatcag aaccttccca acactgagga 16440
cagggggcaa gtacctagcc acctcgcagg tgttgctgtc tcccaagagc atccttgaag 16500
gttcagatga atacctggta tgcaaaatcc actacggagg caaaaacaaa gatctgcatg 16560
tgcccattcc aggtaagaac caaaccctcc cagcaggggt gcccaggccc aggcatggcc 16620
cagagggagc agcggggtgg ggcttaggcc aagctgagct cacaccttga cctttcattc 16680
cagctgtcgc agagatgaac cccaatgtaa atgtgttcgt cccaccacgg gatggcttct 16740
ctggccctgc accacgcaag tctaaactca tctgcgaggc cacgaacttc actccaaaac 16800
cgatcacagt atcctggcta aaggatggga agctcgtgga atctggcttc accacagatc 16860
cggtgaccat cgagaacaaa ggatccacac cccaaaccta caaggtcata agcacactta 16920
ccatctctga aatcgactgg ctgaacctga atgtgtacac ctgccgtgtg gatcacaggg 16980
gtctcacctt cttgaagaac gtgtcctcca catgtgctgc cagtgagtgg cctgggataa 17040
gcccaatgcc tagccctccc agattaggga agtcctccta caattatggc caatgccacc 17100
cagacatggt catttgctcc ttgaactttg gctccccaga gtggccaagg acaagaatga 17160

gcaataggca gtagaggggt gagaatcagc tggaaggacc agcatcttcc cttaagtagg 17220

tttgggggat ggagactaag cttttttcca acttcacaac tagatatgtc ataacctgac 17280

acagtgttct cttgactgca ggtccctcca cagacatcct aaccttcacc atccccccct 17340

cctttgccga catcttcctc agcaagtccg ctaacctgac ctgtctggtc tcaaacctgg 17400

caacctatga aaccctgaat atctcctggg cttctcaaag tggtgaacca ctggaaacca 17460

aaattaaaat catggaaagc catcccaatg gcaccttcag tgctaagggt gtggctagtg 17520

tttgtgtgga agactggaat aacaggaagg aatttgtgtg tactgtgact cacagggatc 17580

tgccttcacc acagaagaaa ttcatctcaa aacccaatgg taggtatccc cccttccctt 17640

cccctccaat tgcaggaccc ttcctgtacc tcatagggag ggcaggtcct cttccaccct 17700

atcctcacta ctgtcttcat ttacagaggt gcacaaacat ccacctgctg tgtacctgct 17760

gccaccagct cgtgagcaac tgaacctgag ggagtcagcc acagtcacct gcctggtgaa 17820

gggcttctct cctgcagaca tcagtgtgca gtggcttcag agagggcaac tcttgcccca 17880

agagaagtat gtgaccagtg ccccgatgcc agagcctggg gccccaggct tctactttac 17940

ccacagcatc ctgactgtga cagaggagga atggaactcc ggagagacct atacctgtgt 18000

tgtaggccac gaggccctgc cacacctggt gaccgagagg accgtggaca agtccactgg 18060

taaacccaca ctgtacaatg tctccctgat catgtctgac acaggcggca cctgctattg 18120

accatgctag cgctcaacca ggcaggccct gggtgtccag ttgctctgtg tatgcaaact 18180

aaccatgtca gagtgagatg ttgcatttta taaaaattag aaataaaaaa aatccattca 18240

aacgtcactg gttttgatta tacaatgctc atgcctgctg agacagttgt gttttgcttg 18300

ctctgcacac accctgcata cttgcctcca ccctggccct tcctctacct tgccagtttc 18360

ctccttgtgt gtgaactcag tcaggcttac aacagacaga gtatgaacat gcgattcctc 18420

cagctacttc tagatatatg gctgaaagct tgcctaacct ggtgcaggca gcattcaggc 18480

acatatatag acacacatgc atttatacat agatatatag gtacacatgt gtagacacat 18540

acatgaatgt gtattcatgg acacacagac aaaggtacac atatatacac atgagttcat 18600

gcgcacacac atgcatggac acttacaaac gccttcagag acaaataggc atagacacac 18660

aaccactcac agaaacagat accaatatgc atggtcctgt gtacacagaa acagactata 18720

ggcaaatata cacaaataaa ctatatagat acaaagatat gcatatacac acatgtacag 18780

aaacatcttc acatgtgtac actaacatgt ggacaggtat agcacacaga tacacctgga 18840

ctctgaccag ggctgtaatc tccaaggctc acggctcaga gagcctacac taggctgggt 18900

cactgatact cctcaggagc ccactctatg attgggagag ataaccccag gtacaaagta 18960

tgcctatctg tctcaacacc atggggcaga agatactcca ctaaccaccc atgacagaaa 19020

gttagccttg gctgtgtctc cattaataga acacctcaga agaccaatgt gaaattgcct 19080

aacccactca cacccaccct gatctccagt tcaaaatgca gaaaacataa tgcagttgtc 19140

caaaagatgc cccaaccaca cacacacaca cacacacaca cacacacaca cacacacaca 19200

cacacataca cacacacaca ccatcaagga gcctctgtaa ggagtcacca cccaataaca 19260

ctgcctcttt gggctcatat cctggacatt cttcatattc atatccattt ggggcctagg 19320

ctttagatat ccccaagggc tcatctttac agggatcaga gatcccaata aatgccctgg 19380

tcccacagcc tccctcaggt atctgtctgt ttatctcttg gtacctttct tagacgttag 19440

gtggcacttt tcggggaaat gtgcgcggaa cccctatttg tttatttttc taaatacatt 19500

caaatatgta tccgctcatg agacaataac cctgataaat gcttcaataa tattgaaaaa 19560

ggaagagtat gagtattcaa catttccgtg tcgcccttat tccctttttt gcggcatttt 19620

gccttcctgt ttttgctcac ccagaaacgc tggtgaaagt aaaagatgct gaagatcagt 19680

tgggtgcacg agtgggttac atcgaactgg atctcaacag cggtaagatc cttgagagtt 19740

ttcgccccga agaacgtttt ccaatgatga gcacttttaa agttctgcta tgtggcgcgg 19800

tattatcccg tgttgacgcc gggcaagagc aactcggtcg ccgcatacac tattctcaga 19860

atgacttggt tgagtactca ccagtcacag aaaagcatct tacggatggc atgacagtaa 19920

gagaattatg cagtgctgcc ataaccatga gtgataacac tgcggccaac ttacttctga 19980

caacgatcgg aggaccgaag gagctaaccg cttttttgca caacatgggg gatcatgtaa 20040

ctcgccttga tcgttgggaa ccggagctga atgaagccat accaaacgac gagcgtgaca 20100

ccacgatgcc tgcagcaatg gcaacaacgt tgcgcaaact attaactggc gaactactta 20160

ctctagcttc ccggcaacaa ttaatagact ggatggaggc ggataaagtt gcaggaccac 20220

ttctgcgctc ggcccttccg gctggctggt ttattgctga taaatctgga gccggtgagc 20280

gtgggtctcg cggtatcatt gcagcactgg ggccagatgg taagccctcc cgtatcgtag 20340

ttatctacac gacggggagt caggcaacta tggatgaacg aaatagacag atcgctgaga 20400

taggtgcctc actgattaag cattggtaac tgtcagacca agtttactca tatatacttt 20460

agattgattt aaaacttcat ttttaattta aaaggatcta ggtgaagatc ctttttgata 20520

atctcatgac caaaatccct taacgtgagt tttcgttcca ctgagcgtca gaccccgtag 20580

aaaagatcaa aggatcttct tgagatcctt tttttctgcg cgtaatctgc tgcttgcaaa 20640

caaaaaaacc accgctacca gcggtggttt gtttgccgga tcaagagcta ccaactcttt 20700

ttccgaaggt aactggcttc agcagagcgc agataccaaa tactgtcctt ctagtgtagc 20760

cgtagttagg ccaccacttc aagaactctg tagcaccgcc tacatacctc gctctgctaa 20820

tcctgttacc agtggctgct gccagtggcg ataagtcgtg tcttaccggg ttggactcaa 20880

gacgatagtt accggataag gcgcagcggt cgggctgaac ggggggttcg tgcacacagc 20940

ccagcttgga gcgaacgacc tacaccgaac tgagatacct acagcgtgag ctatgagaaa 21000

gcgccacgct tcccgaaggg agaaaggcgg acaggtatcc ggtaagcggc agggtcggaa 21060

caggagagcg cacgagggag cttccagggg gaaacgcctg gtatctttat agtcctgtcg 21120

ggtttcgcca cctctgactt gagcgtcgat ttttgtgatg ctcgtcaggg gggcggagcc 21180

tatggaaaaa cgccagcaac gcggcctttt tacggttcct ggccttttgc tggccttttg 21240

ctcacatgtt ctttcctgcg ttatcccctg attctgtgga taaccgtatt accgcctttg 21300

agtgagctga taccgctcgc cgcagccgaa cgaccgagcg cagcgagtca gtgagcgagg 21360

aagcggaaga gcgcctgatg cggtattttc tccttacgca tctgtgcggt atttcacacc 21420

gcatatggtg cactctcagt acaatctgct ctgatgccgc atagttaagc cagtatacac 21480

tccgctatcg ctacgtgact gggtcatggc tgcgccccga cacccgccaa cacccgctga 21540

cgcgccctga cgggcttgtc tgctcccggc atccgcttac agacaagctg tgaccgtctc 21600

cgggagctgc atgtgtcaga ggttttcacc gtcatcaccg aaacgcgcga ggcagctgcg 21660

gtaaagctca tcagcgtggt cgtgaagcga ttcacagatg tctgcctgtt catccgcgtc 21720

cagctcgttg agtttctcca gaagcgttaa tgtctggctt ctgataaagc gggccatgtt 21780

aagggcggtt ttttcctgtt tggtcactga tgcctccgtg taagggggat ttctgttcat 21840

gggggtaatg ataccgatga aacgagagag gatgctcacg atacgggtta ctgatgatga 21900

acatgcccgg ttactggaac gttgtgaggg taaacaactg gcggtatgga tgcggcggga 21960

ccagagaaaa atcactcagg gtcaatgcca gcgcttcgtt aatacagatg taggtgttcc 22020

acagggtagc cagcagcatc ctgcgatgca gatccggaac ataatggtgc agggcgctga 22080

cttccgcgtt tccagacttt acgaaacacg gaaaccgaag accattcatg ttgttgctca 22140

ggtcgcagac gttttgcagc agcagtcgct tcacgttcgc tcgcgtatcg 22190

<210> 3
<211> 14130
<212> DNA
<213> Artificial Sequence

<220>
<223> Targetting vector (short version)

<400> 3
gcggccgcaa cctgggcaaa tgggagctta gcaacaatgt agggggctgg acctagactt 60

cctacacatg tgtaacagat gtgcagcttg gtcttcatgt gtgtattacc ctaacatttg 120

gagcaggagc tgtctctgac tctgttgcct gccattggat ccccttcccc tgcttgggct 180

gccttgtttg gccttagtag gaaaggatgt gcttagtcct gctgtgactt gatgtcccta 240

ggcagaatga taccccaggg gggctcccca tctctgagga gatgggcaaa gggtaatggt 300

tggagggact tgtgaggctg ggactgggag gagagaaagg agacagctgt aactggaatg 360

atgttaagtg aacaaatgaa tggatagatt agatagacag atagacagac agacagacag 420

acagacagac agacagacag acagacagat agaaagatag atagataagg ggaaaaagaa 480

acgtagctga gcaagccaga gagagcaagc caaataagca gcattcctcc atgacttttc 540

cttcagctcc tgcctatgag tctgccttga cttccctcag tgattggttg taagttaaaa 600

ggtgaaataa accctttctt tgacaagttg cttttggttc tgatttttat cacagcaaga 660

gaaaatcaaa ctagaacaaa catgtatttt tcctggcaca tgtccatagt aaggcagaaa 720

tgatcttcag acctagacca tagatactac agagagcaga agtgtagata ggtggactta 780

ctgtatgatt gtaatccaag taaatctaca tagctagaga gctagaggaa aggccaaagc 840

ttcctctggg aggtcagatc ctgtcgcact gtagccaata aggcatattg catcacagga 900

aaggactaag acccaggctg gcaatagtgt ctgtatctta actagatctc tctagtgagt 960

gaggaagtaa atttgtgaga gcccagactg tgggctcgga aggtacctgc catgcccctg 1020

ttagtaactg agtactacag caggagcagg tgttctctag aaagcctgag acaactctac 1080

ttcttctctc aagagaccac ctaatacagg cctgagagaa cagactctgg aaatagatgg 1140

gacttacgga gctaagatct agagctcatc tacagagcag aatcccagcc aagagaacaa 1200

agaatactga ctctctcctg ttccctactc ctagagttct aaaacacact ataggaaggg 1260

gagcctctag acctccgtcc attccccatc ttgctcattc catcttccca tgtccccagg 1320

tctccaagcc acagacacca cctttcctat tcacccacct ttctgtgtcc ctaggtcccc 1380

aggccatagt cacctccccc cacaccccgc tcaccctgcc ccatctatgc ccctagatgc 1440

ttacttacca gagtcttttg tctgacgtgg ggctacaagc atctatgctc cctaagcacc 1500

tactgctgac ctgtaggacc cagctctgaa ccaactcata taagtaaata cagactctcc 1560

cctgtcttag gatggccccc tgggtcagga ggagaccact gccaaggaac cttctcttag 1620

agcactgaac tcctcccctg taccacttag gacagacctg agacctatta ttactgatta 1680

ccagagctct ggcagtgacc acggaggaga tagatccacc ctggacacag gaaacacagc 1740

accagagata ctgcttcatc acaacagtag agtgacactt tagactttaa tttgggtcac 1800

tttcctgctg tagaggtggg atcagaaagc aaagagcagt atgagtgcct gataggcacc 1860

caagtacact atagagtact catggtgaat aaggtacctc catggcttcc cagggagggg 1920

cactgcccca cccccaccat cacagacctt tctccatagt tgataactca gacacaagtg 1980

aatgacagat ggacctccat ctgctcttat tttaaaaaga agacaaaccc cacaggctcg 2040

agaactttag cgactgtttt gagagaaatc attggtccct gactcaagag atgactggca 2100

gattggggat cagaataccc atactctgtg gctagtgtga ggtttaagcc tcagagtccc 2160

tgtggtctct gactggtgca aggttttgac taagcggagc accacagtgc taactgggac 2220

cacggtgaca cgtggctcaa caaaaacctt ctgtttggag ctctccaggg gcagcctgag 2280

ctatgaggaa gtagagaggc ttgagaaatc tgaggaagaa aagagtagat ctgagaggaa 2340

aggtagcttt ctggaggtca ggagacagtg cagagaagaa cgagttactg tggacaggtc 2400

ttagatgggg aaagaatgag caaatgcaag catcagaagg gtggatgcaa tgtcctgcca 2460

aggacttacc aagaggatcc ccggacagag caggcaggtg gagttgactg agaggacagg 2520

ataggtgcag gtccctctct tgtttccttt ctccttctcc tgtttccttc ttctcttgtc 2580

acaggtctca ctatgctagc caaggctagc ctgaaagatt accatcctac agatgggccc 2640

atccagttga attaaggtgg agatctctcc aaacatctga gtttctgagg cttggatgcc 2700

actggggacg ccaagggact ttgggatggg tttggttggc cccagatgaa gggctacttc 2760

actgggtcta taattactct gatgtctagg accagggggc tcaggtcact caggtcaggt 2820

gagtcctgca tctggggact gtggggttca ggtggcctaa ggcaggatgt ggagagagtt 2880

ttagtatagg aacagaggca gaacagagac tgtgctactg gtacttcgat gtctggggca 2940

cagggaccac ggtcaccgtc tcctcaggta agctggcttt tttctttctg cacattccat 3000

tctgaaacgg gaaaagatat tctcagatct ccccatgtca ggccatctgc cacactctgc 3060

atgctgcaga agcttttctg taaggatagg gtcttcactc ccaggaaaag aggcagtcag 3120

aggctagctg cctgtggaac agtgacaatc atggaaaata ggcatttaca ttgttaggct 3180

acatgggtag atgggttttt gtacacccac taaaggggtc tatgatagtg tgactacttt 3240

gactactggg gccaaggcac cactctcaca gtctcctcag gtgagtcctt acaacctctc 3300

tcttctattc agcttaaata gattttactg catttgttgg gggggaaatg tgtgtatctg 3360

aatttcaggt catgaaggac tagggacacc ttgggagtca gaaagggtca ttgggagccc 3420

tggctgacgc agacagacat cctcagctcc catacttcat ggccagagat ttatagggat 3480

cctggccagc attgccgcta ggtccctctc ttctatgctt tctttgtccc tcactggcct 3540

ccatctgaga tcatcctgga gccctagcca aggatcattt attgtcaggg gtctaatcat 3600

tgttgtcaca atgtgcctgg tttgcttact ggggccaagg gactctggtc actgtctctg 3660

caggtgagtc ctaacttctc ccattctaaa tgcatgttgg ggggattctg ggccttcagg 3720

accaagattc tctgcaaacg ggaatcaaga ttcaacccct ttgtcccaaa gttgagacat 3780

gggtctgggt cagggactct ctgcctgctg gtctgtggtg acattagaac tgaagtatga 3840

tgaaggatct gccagaactg aagcttgaag tctgaggcag aatcttgtcc agggtctatc 3900

ggactcttgt gagaattagg ggctgacagt tgatggtgac aatttcaggg tcagtgactg 3960

tctggtttct ctgaggtgag gctggaatat aggtcacctt gaagactaaa gaggggtcca 4020

ggggcttctg cacaggcagg gaacagaatg tggaacaatg acttgaatgg ttgattcttg 4080

tgtgacacca ggaattggca taatgtctga gttgcccagg ggtgattcta gtcagactct 4140

gggggttttg tcgggtatag aggaaaaatc cactattgtg attactatgc tatggactac 4200

tggggtcaag gaacctcagt caccgtctcc tcaggtaaga atggcctctc caggtcttta 4260

tttttaacct ttgttatgga gttttctgag cattgcagac taatcttgga tatttgtccc 4320

tgagggagcc ggctgagaga agttgggaaa taaactgtct agggatctca gagcctttag 4380

gacagattat ctccacatct ttgaaaaact aagaatctgt gtgatggtgt tggtggagtc 4440

cctggatgat gggataggga ctttggaggc tcatttgaag aagatgctaa aacaatccta 4500

tggctggagg gatagttggg gctacgcgtt tttaacccta gaaagatagt ctgcgtaaaa 4560

ttgacgcatg cattcttgaa atattgctct ctctttctaa atagcgcgaa tccgtcgctg 4620

tgcatttagg acatctcagt cgccgcttgg agctcccgtg aggcgtgctt gtcaatgcgg 4680

taagtgtcac tgattttgaa ctataacgac cgcgtgagtc aaaatgacgc atgattatct 4740

tttacgtgac ttttaagatt taactcatac gataattata ttgttatttc atgttctact 4800

tacgtgataa cttattatat atatattttc ttgttataga tatcgctagt ggatcctggt 4860

tctttccgcc tcagaaggta cttttttttt tttttttttt tttttttttt tttttttttt 4920

tttttttttt tttttttttt taaatttttg ggaatttatt gatttgcatt taaaagggaa 4980

ctgctgacaa agattcactg gtaataattt gaacaagttg gaaaatacag tcaacattac 5040

tgaaacacta ctaaaataat tccaggacag aacaaaactt cttagatgct gtctttgatg 5100

tgaaaattga ctgcttctta cttttctaac acacggtggt ataattaaca atattcaatc 5160

acttctattc tttcctgcat atataaaaat taaaatacca attaaaaaac taatatatct 5220

tctctttatt tcttacagat atgagttcaa tgtttcactc aatagtgctg tggtttaaga 5280

gaattttttc atttacaagt taaacaacaa tccgcccaaa gggaactgat agtctatagg 5340

ctcatagtgc aaataaacag tttaggaatg cagcaactga catttctaaa gtacaaaaca 5400

gataaaattc ttagaagata catgcaaaaa gctctactaa gcagatggcc acagaactag 5460

aacattgata attttactgg cgatgtcaat aggactccag atgtttccaa actcaacttg 5520

aactctcatc ttaggctttg tattttgctt ttccagtttc actaatgaca caaacatgat 5580

tcaaatccct gaagtattca ttatagtcaa gggcatatcc tacaacaaac ttgtctggaa 5640

tttcaaatcc aacaaagtct ggcttatatc caacacttcg tggggtcctt ttcaccagca 5700

agcttgcgac cttgaccatc tttggattat actgcctgac caaggaaagc aaagtctgca 5760

ttgttttgcc agtgtcaatt atatcttcca caatcaagac attctttcca gttaaagttg 5820

agagatcatc tccaccaatt acttttatgt cccctgttga ctggtcatta caatagctct 5880

tcagtctgat aaaatctaca gtcataggaa tggatctatc actatttcta ttcagtgctt 5940

tgatgtaatc cagcaggtca gcaaagaatt tatagccccc cttgagcaca cagagggcta 6000

caatgtgatg gcctcccatc tccttcatca catctcgagc aagacgttca gtcctacaga 6060
aataaaatca ggaatttaat agaaagtttc atacattaaa ctttataaca aacacctctt 6120
agtcattaaa cttccacacc aacctgggca atatagtgag accccatgcc tgcaaaaaaa 6180
aaaaaattag ccaggcatgg tagcatgtac ctgtagtccc agctacttga gaggtgaggt 6240
gggaaaatca ctttagtgca ggatgttgag gctggagtga actgtgattg tgccactgca 6300
ctccagcctg gacaatagag caagaccttg tctcaaaaaa atgcattaaa aatttttttt 6360
aaatcttcca cgtaacacat cctttgccct catgtttcat aaggtaaaaa atttgatacc 6420
ttcaaaaaaa ccaagcatac cactatcata atttttttta aatgcaaata aaaacaagat 6480
accattttca cctatcagac tggcaggttc tgattaaatg aaatttcttg gataatatac 6540
aatattaaga gagactgtag aaactgggcc agtggctcat gcctgtaatc ccagcacttt 6600
gggaggctgg gtaacatggc gaaccctgtt tctacaaaat aaaaatatta gctgggagtg 6660
gtggcgcaca cctatagtcc cagctactca ggaggctgag gtggaaggat cgcttgaacc 6720
caggaggttg agactgcagt gaactgtgat cattctgctg cactgcaccc cagcctgggc 6780
aacagagacc ttgtctcaaa aaaaaaaaaa aaagagacaa attgtgaaga gaaaggtact 6840
ctcatataac atcaggagta taaaatgatt caacttctta gaggaaaatt tggcaatacc 6900
aaaatattca ataaactctt tccccttgac ccagaaattc cacttgaata aagctgaaca 6960
agtaccaaac atgtaaaaga atgtttcttc tagtacagtc ggtaagaaca aaatagtgtc 7020
tatcaatagt ggactggtta aatcagttat ggtatctcca taagacagaa tgctatgcaa 7080
cctttaaaat atattagata gctctagaca gtggatcccc tcgagggacc taataacttc 7140
gtatagcata cattatacga agttatatta agggttattg aatatgtcga ctagacacac 7200
taatattaaa agtgtccaat aacatttaaa actatactca tacgttaaaa tataaatgta 7260
tatatgtact tttgcatata gtatacatgc atagccagtg cttgagaaga aatgtgtaca 7320
gaaggctgaa aggagagaac tttagtcttc ttgtttatgg cctccatagt tagaatattt 7380
tataacacaa atattttgat attataattt taaaataaaa acacagaata gccagacata 7440
caatgcaagc attcaatacc aggtaaggtt tttcactgta attgacttaa cagaaaattt 7500
tcaagctaga tgtgcataat aataaaaatc tgaccttgcc ttcatgtgat tcagccccag 7560
tccattaccc tgtttaggac tgagaaatgc aagactctgg ctagagttcc ttcttccatc 7620
tcccttcaat gtttactttg ttctggtccc tacagagtcc cactatacca caactgatac 7680
taagtaatta gtaaggccct cctcttttat ttttaataaa gaagatttta gaaagcatca 7740
gttatttaat aagttggcct agtttatgtt caaatagcaa gtactcagaa cagctgctga 7800
tgtttgaaat taacacaaga aaaagtaaaa aacctcattt taagatctta cttacctgtc 7860
cataattagt ccatggggaa taaacaccct ttccaaatcc tcagcataat gattaggtat 7920

gcaaaataaa tcaaggtcat aacctggttc atcatcacta atcacgacgc cagggctgcg 7980

ggtcgccata acggagccgg ccggcgcgcg ggctgaataa cttcgtataa tgtgtactat 8040

acgaagttat ttgttcagga ggaggaagcc ggtggcggag cagaggagga ggcggaggcg 8100

cagcaagacc cccccccccc tgcaggtcga aaggcccgga gatgaggaag aggagaacag 8160

cgcggcagac gtgcgctttt gaagcgtgca gaatgccggg cctccggagg accttcgggc 8220

gcccgccccg cccctgagcc cgcccctgag cccgcccccg gacccacccc ttcccagcct 8280

ctgagcccag aaagcgaagg agccaaagct gctattggcc gctgccccaa aggcctaccc 8340

gcttccattg ctcagcggtg ctgtccatct gcacgagact agtgagacgt gctacttcca 8400

tttgtcacgt cctgcacgac gcgagctgcg gggcgggggg gaacttcctg actaggggag 8460

gagtagaagg tggcgcgaag gggccaccaa agaacggagc cggttggcgc ctaccggtgg 8520

atgtggaatg tgtgcgaggc cagaggccac ttgtgtagcg ccaagtgccc agcggggctg 8580

ctaaagcgca tgctccagac tgccttggga aaagcgcctc ccctacccgg tagatatcta 8640

taacaagaaa atatatatat aataagttat cacgtaagta gaacatgaaa taacaatata 8700

attatcgtat gagttaaatc ttaaaagtca cgtaaaagat aatcatgcgt cattttgact 8760

cacgcggtcg ttatagttca aaatcagtga cacttaccgc attgacaagc acgcctcacg 8820

ggagctccaa gcggcgactg agatgtccta aatgcacagc gacggattcg cgctatttag 8880

aaagagagag caatatttca agaatgcatg cgtcaatttt acgcagacta tctttctagg 8940

gttaaaagaa ttcgtagttg gagattttca gtttttagaa taaaagtatt agctgcggaa 9000

tatacttcag gaccacctct gtgacagcat ttatacagta tccgatgcat agggacaaag 9060

agtggagtgg ggcactttct ttagatttgt gaggaatgtt ccacactaga ttgtttaaaa 9120

cttcatttgt tggaaggaga gctgtcttag tgattgagtc aagggagaaa ggcatctagc 9180

ctcggtctca aaagggtagt tgctgtctag agaggtctgg tggagcctgc aaaagtccag 9240

ctttcaaagg aacacagaag tatgtgtatg gaatattaga agatgttgct tttactctta 9300

agttggttcc taggaaaaat agttaaatac tgtgacttta aaatgtgaga gggttttcaa 9360

gtactcattt ttttaaatgt ccaaaatttt tgtcaatcaa tttgaggtct tgtttgtgta 9420

gaactgacat tacttaaagt ttaaccgagg aatgggagtg aggctctctc ataccctatt 9480

cagaactgac ttttaacaat aataaattaa gtttaaaata tttttaaatg aattgagcaa 9540

tgttgagttg gagtcaagat ggccgatcag aaccagaaca cctgcagcag ctggcaggaa 9600

gcaggtcatg tggcaaggct atttggggaa gggaaaataa aaccactagg taaacttgta 9660

gctgtggttt gaagaagtgg ttttgaaaca ctctgtccag ccccaccaaa ccgaaagtcc 9720

aggctgagca aaacaccacc tgggtaattt gcatttctaa aataagttga ggattcagcc 9780

gaaactggag aggtcctctt ttaacttatt gagttcaacc ttttaatttt agcttgagta 9840
gttctagttt ccccaaactt aagtttatcg acttctaaaa tgtatttaga attcattttc 9900
aaaattaggt tatgtaagaa attgaaggac tttagtgtct ttaatttcta atatatttag 9960
aaaacttctt aaaattactc tattattctt ccctctgatt attggtctcc attcaattct 10020
tttccaatac ccgaagcatt tacagtgact ttgttcatga tcttttttag ttgtttgttt 10080
tgccttacta ttaagacttt gacattctgg tcaaaacggc ttcacaaatc tttttcaaga 10140
ccactttctg agtattcatt ttaggagaaa tacttttttt ttaaatgaat gcaattatct 10200
agacttattt cggttgaaca tgctggttgg tggttgagag gacactcagt cagtcagtgg 10260
cgtgaagggc ttctaagcca gtccacatgc tctgtgtgaa ctccctctgg ccctgcttat 10320
tgttgaatgg gccaaaggtc tgagaccagg ctgctgctgg gtaggcctgg actttgggtc 10380
tcccacccag acctgggaat gtatggttgt ggcttctgcc acccatccac ctggctgctc 10440
atggaccagc cagcctcggt ggctttgaag gaacaattcc acacaaagac tctggacctc 10500
tccgaaacca ggcaccgcaa atggtaagcc agaggcagcc acagctgtgg ctgctgctct 10560
taaagcttgt aaactgtttc tgcttaagag ggactgagtc ttcagtcatt gctttagggg 10620
gagaaagaga catttgtgtg tcttttgagt accgttgtct gggtcactca catttaactt 10680
tccttgaaaa actagtaaaa gaaaaatgtt gcctgttaac caataatcat agagctcatg 10740
gtattttgag gaaatcttag aaaacgtgta tacaattgtc tggaattatt tcagttaagt 10800
gtattagttg aggtactgat gctgtctcta cttcagttat acatgtgggt ttgaattttg 10860
aatctattct ggctcttctt aagcagaaaa tttagataaa atggatacct cagtggtttt 10920
taatggtggg tttaatatag aaggaattta aattggaagc taatttagaa tcagtaagga 10980
gggacccagg ctaagaaggc aatcctggga ttctggaaga aaagatgttt ttagttttta 11040
tagaaaacac tactacattc ttgatctaca actcaatgtg gtttaatgaa tttgaagttg 11100
ccagtaaatg tacttcctgg ttgttaaaga atggtatcaa aggacagtgc ttagatccaa 11160
ggtgagtgtg agaggacagg ggctggggta tggatacgca gaaggaaggc cacagctgta 11220
cagaattgag aaagaataga gacctgcagt tgaggccagc aggtcggctg gactaactct 11280
ccagccacag taatgaccca gacagagaag gccagactca taaagcttta tcgataccgt 11340
cgacctcgag ggggggcccg gtacctttct tagacgtcag gtggcacttt tcggggaaat 11400
gtgcgcggaa cccctatttg tttatttttc taaatacatt caaatatgta tccgctcatg 11460
agacaataac cctgataaat gcttcaataa tattgaaaaa ggaagagtat gagtattcaa 11520
catttccgtg tcgcccttat tccctttttt gcggcatttt gccttcctgt ttttgctcac 11580
ccagaaacgc tggtgaaagt aaaagatgct gaagatcagt tgggtgcacg agtgggttac 11640
atcgaactgg atctcaacag cggtaagatc cttgagagtt ttcgccccga agaacgtttt 11700

ccaatgatga gcacttttaa agttctgcta tgtggcgcgg tattatcccg tgttgacgcc 11760

gggcaagagc aactcggtcg ccgcatacac tattctcaga atgacttggt tgagtactca 11820

ccagtcacag aaaagcatct tacggatggc atgacagtaa gagaattatg cagtgctgcc 11880

ataaccatga gtgataacac tgcggccaac ttacttctga caacgatcgg aggaccgaag 11940

gagctaaccg cttttttgca caacatgggg gatcatgtaa ctcgccttga tcgttgggaa 12000

ccggagctga atgaagccat accaaacgac gagcgtgaca ccacgatgcc tgcagcaatg 12060

gcaacaacgt tgcgcaaact attaactggc gaactactta ctctagcttc ccggcaacaa 12120

ttaatagact ggatggaggc ggataaagtt gcaggaccac ttctgcgctc ggcccttccg 12180

gctggctggt ttattgctga taaatctgga gccggtgagc gtgggtctcg cggtatcatt 12240

gcagcactgg ggccagatgg taagccctcc cgtatcgtag ttatctacac gacggggagt 12300

caggcaacta tggatgaacg aaatagacag atcgctgaga taggtgcctc actgattaag 12360

cattggtaac tgtcagacca agtttactca tatatacttt agattgattt aaaacttcat 12420

ttttaattta aaaggatcta ggtgaagatc ctttttgata atctcatgac caaaatccct 12480

taacgtgagt tttcgttcca ctgagcgtca gaccccgtag aaaagatcaa aggatcttct 12540

tgagatcctt tttttctgcg cgtaatctgc tgcttgcaaa caaaaaaacc accgctacca 12600

gcggtggttt gtttgccgga tcaagagcta ccaactcttt ttccgaaggt aactggcttc 12660

agcagagcgc agataccaaa tactgtcctt ctagtgtagc cgtagttagg ccaccacttc 12720

aagaactctg tagcaccgcc tacatacctc gctctgctaa tcctgttacc agtggctgct 12780

gccagtggcg ataagtcgtg tcttaccggg ttggactcaa gacgatagtt accggataag 12840

gcgcagcggt cgggctgaac ggggggttcg tgcacacagc ccagcttgga gcgaacgacc 12900

tacaccgaac tgagatacct acagcgtgag ctatgagaaa gcgccacgct tcccgaaggg 12960

agaaaggcgg acaggtatcc ggtaagcggc agggtcggaa caggagagcg cacgagggag 13020

cttccagggg gaaacgcctg gtatctttat agtcctgtcg ggtttcgcca cctctgactt 13080

gagcgtcgat ttttgtgatg ctcgtcaggg gggcggagcc tatggaaaaa cgccagcaac 13140

gcggcctttt tacggttcct ggccttttgc tggccttttg ctcacatgtt ctttcctgcg 13200

ttatcccctg attctgtgga taaccgtatt accgcctttg agtgagctga taccgctcgc 13260

cgcagccgaa cgaccgagcg cagcgagtca gtgagcgagg aagcggaaga gcgcctgatg 13320

cggtattttc tccttacgca tctgtgcggt atttcacacc gcatatggtg cactctcagt 13380

acaatctgct ctgatgccgc atagttaagc cagtatacac tccgctatcg ctacgtgact 13440

gggtcatggc tgcgccccga cacccgccaa cacccgctga cgcgccctga cgggcttgtc 13500

tgctcccggc atccgcttac agacaagctg tgaccgtctc cgggagctgc atgtgtcaga 13560

ggttttcacc gtcatcaccg aaacgcgcga ggcagctgcg gtaaagctca tcagcgtggt 13620

cgtgaagcga ttcacagatg tctgcctgtt catccgcgtc cagctcgttg agtttctcca 13680

gaagcgttaa tgtctggctt ctgataaagc gggccatgtt aagggcggtt ttttcctgtt 13740

tggtcactga tgcctccgtg taagggggat ttctgttcat gggggtaatg ataccgatga 13800

aacgagagag gatgctcacg atacgggtta ctgatgatga acatgcccgg ttactggaac 13860

gttgtgaggg taaacaactg gcggtatgga tgcggcggga ccagagaaaa atcactcagg 13920

gtcaatgcca gcgcttcgtt aatacagatg taggtgttcc acagggtagc cagcagcatc 13980

ctgcgatgca gatccggaac ataatggtgc agggcgctga cttccgcgtt tccagacttt 14040

acgaaacacg gaaaccgaag accattcatg ttgttgctca ggtcgcagac gttttgcagc 14100

agcagtcgct tcacgttcgc tcgcgtatcg 14130

<210> 4
<211> 3551
<212> DNA
<213> Mus musculus

<220>
<221> misc_feature
<222> (1399)..(1498)
<223> n is a, c, g, or t

<400> 4
aagcttgctg agcaaaatta agggaacaag gttgagagcc ctagtaagcg aggctctaaa 60

aagcatggct gagctgagat gggtgggctt ctctgagcgc ttctaaaatg cgctaaactg 120

aggtgattac tctgaggtaa gcaaagctgg gcttgagcca aaatgaagta gactgtaatg 180

aactggaatg agctgggccg ctaagctaaa ctaggctggc ttaaccgaga tgagccaaac 240

tggaatgaac ttcattaatc taggttgaat agagctaaac tctactgcct acactggact 300

gttctgagct gagatgagct ggggtgagct cagctatgct acgctgtgtt ggggtgagct 360

gatctgaaat gagctactct ggagtagctg agatggggtg agatggggtg agctgagctg 420

ggctgagctg gactgagctg agctagggtg agctgagctg ggtgagctga gctaagctgg 480

ggtgagctga gctgagcttg actgagctag ggtgagctgg actgagctgg ggtgagctga 540

gctgagctgg ggtaagctgg gatgagctgg ggtgagctga gctgagctgg agtgagctga 600

gctgggctga gctggggtga gctgggctgg gctgagctgg ggtgagctgg gctgagctgg 660

ggtgagctga gctggggtga gctgagctga gctggggtga gctgagctga gctggggtga 720

gctgagctgg ggtgagctga gctgagctgg gctgagctga ggtgagctga gctggggtga 780

gctgagctgg ggtgagctga gctgagctgg ggtaagctgg gatgagctgg ggtgagctga 840

gctgagctgg agtgagctga gctgggctga gctgggctga gctggggtga gctgagctgg 900

ggtgagctga gctgagctgg gctgagctga ggtgagctga gctggggtga gctgagctga 960

gctggggtga gctgagctga gctggggtga gctgagctgg ggtgagctga gctggggtga 1020

gctgagctga gctggggtga gctgagctgg ggtgagctga gctgagctgg ggtgagctga 1080

gctgagctgg ggtgagctga gctgagctga gctggggtga gctgagctga gctggggtga 1140

gctgagctga gctggggtga gctgagctgg ggtgagctgg gctgagctga gctgggctga 1200

gctgagctga gctgagctga gctggggtga gctgagctgg gctgagctgg ggtgagctgg 1260

gctgagctgg ggtgagctga gctggggtga gctgagctga gctggggtga gctgagctga 1320

gctggggtga gctgagctgg ggtgagctga gctgagctgg gctgagctga gctgagctgg 1380

ggtgagctga gctgagctnn nnnnnnnnnn nnnnnnnnnn nnnnnnnnnn nnnnnnnnnn 1440

nnnnnnnnnn nnnnnnnnnn nnnnnnnnnn nnnnnnnnnn nnnnnnnnnn nnnnnnnnag 1500

ctgagctgag ctgagctgag ctgagctggg gtgagctggg gtgagctgag ctggggtgag 1560

ctgagctgag ctggggtgag ctgagctgag ctgagctgag ctgagctgag ctgggtgagc 1620

tgagctgagc tgagctgggg tgagctgagc tggggtgagc tgagctgagc tggggtgagc 1680

tgagctgggg tgagctgagc tgagctgggg tgagctgggg tgagctgggg tgagctgggg 1740

tgagctgagc tgaactgggg tgagctgggc tgagctgggg tgagctgagc tgagctgggc 1800

tgagctgggg tgagctgggg tgagctgggg tgagctgagc tgagctaggg tgagctgagc 1860

tgagctaggg tgagctgagc tgagctgggg tgagctgagc tgagctgggg tgagctgagc 1920

tgagctgggg tgagctgagc tgagctgggg tgagctgagc tgagctgggg tgagcttggc 1980

tgagctgggg tgagctgggg tgagctgagc tggggtgagc tggggtaagc tgagctgagc 2040

tggggtgagc tgagctgagc tggggtgagc tggggtgagc tgagctgagc tgagctgggt 2100

gatctgagct gagctgagct gggtgagctg agctgagctg agctgggtga gctgagctga 2160

gctgagctga gctgggtgag ctgagctgag ctgagctgag ctgagctgag ctggggtgag 2220

ctgggctgag ctgagctgag ctggggtgag ctgagctgag ctgagctgag ctggggtgag 2280

ctgggctgag ctggggtgag ctgggctgag ctgagctggg tgagctgagc tgaactgagc 2340

tgagctgggt gagctgagct gagctgagct gggtgagctg agctgggctg agctgagctg 2400

ggtgagctga gctgaactga gctgagctgg gtgagctgag ctgagctgag ctgggtgagc 2460

tgagctgggg tgagctgagc tgagctgggg tgagctgagc tgagctgagc tgggtgagct 2520

gagctggggt gagctgagct gagctggggt gagctgagct gagctggggt gagctgagct 2580

gagctggggt gagctgagct gagctggggt gagctgagct agggtgaact gggctgggtg 2640

agctggagtg agctgagctg aggtgaactg gggtgagccg ggatgttttg agttgagctg 2700

gggtaagatg agctgaactg gggtaagatg ggatgagctg tggtgagggg agctggattg 2760

aactgagctg tgtgagctga gctggggtca gctgagcaag agtgagtaga gctggctggc 2820

cagaaccaga atcaattagg ctaagtgagc cagattgcgc tgggatcagc tgtactcaga 2880

tgagctggga tgaggtaggc tgggatgagc tgggctagct gacatggatt atgtgaggct 2940

gagctagcat gggctggcct agctgatgag ctaagcttga atgaacgggg ctgagctgga 3000

ctcagatgtg ctagactgag ctgtactgga tgatctggtg tagggtgatc tggactcaac 3060

tgggctggct gatgggatgc cccaggttga actaggctca gataagttag gctgagtagg 3120

gcctggttga gatggttcgg gatgagctgg gaaaagatgg actgggacca tgaactgggc 3180

tgagctgggt tgggagacca tgaattgagc tgaactgagt gcagctggga taaactgggt 3240

tgagctaaga atagactacc tgaattgtgc caaactgggc tgggatcaat tggaaattat 3300

caggatttag atgagccgga ctaaactatg ctgagctgga ctggttggat gtgttgaact 3360

ggcctgctgc tgggctggca tagctgagtt gaacttaaat gaggaaggat gagcaaggct 3420

agcctgcttg catagagctg aactttagcc tagcctgagc tggaccagcc tgagctgagt 3480

aggtctaaac tgagttaaaa atcaacaggg ataatttaac agctaattta acaagcctga 3540

ggtctgagat t 3551

<210> 5
<211> 2484
<212> DNA
<213> Mus musculus

<400> 5
aagcttgctg agcaaaatta agggaacaag gttgagagcc ctagtaagcg aggctctaaa 60

aagcacagct gagctgagat gggtgggctt ctctgagtgc ttctaaaatg cgctaaactg 120

aggtgattac tctgaggtaa gcaaagctgg gcttgagcca aaatgaagta gactgtaatg 180

aactggaatg agctgggccg ctaagctaaa ctaggctggc ttaaccgaga tgagccaaac 240

tggaatgaac ttcattaatc taggttgaat agagctaaac tctactgcct acactggact 300

gttctgagct gagatgagct ggggtgagct cagctatgct acgctgtgtt ggggtgagct 360

gatctgaaat gagatactct ggagtagctg agatggggtg agatggggtg agctgagctg 420

ggctgagcta gactgagctg agctagggtg agctgagctg ggtgagctga gctaagctgg 480

ggtgagctga gctgagcttg gctgagctag ggtgagctgg gctgagctgg ggtgagctga 540

gctgagctgg ggtaagctgg gatgagctgg ggtgagctga gctgagctgg agtgagctga 600

gctgggctga gctggggtga gctgggctga gctgggctga gctgggctga gctggggtga 660

gctgagctgg ggtgagctga gctgagctgg ggtgagctga gctgagctgg ggtgagctgg 720

ggtgagctga gctggggtga gctgagctga gctggggtga gctgagctgg ggtgagctga 780

gctgagctgg ggtgagctga gctgagctga gctgagctga gctggggtga gctgagctga 840

gctgagctgg ggtgagctgg ggtgagctga gctgagctgg agtgagctga gctgggctga 900

gctggggtga gctgggctga gctggggtga gctgagctga gctgagctga gctggggtga 960

gctgagctga gctggggtga gctgagctgg ggtgagctgg gctgagctga gctgagctga 1020

gctgagctga gctgagctga gctgagctga gctgagctga gctgagctga gctgagctga 1080

gctgagctgg ggtgagctga gctgagctgg gctgagctgg ggtgagctgg gctgagctgg 1140

gctgagctgg gctgagctgg ggtgagctga gctggggtga gctgagctga gctgggctga 1200

gctgagctga gctggggtga gctgagctga gctggggtga gctgagctga gctgagctgg 1260

ggtgagctga gctgggctga gcagggctga gctggggtga gctgagctga gctggggtga 1320

gctgggctga gctgggctga gctgagctga gctgggctga gctgggctga gctgggctga 1380

gctgggctga gctgggctga gctggggtga gctgagctga gctggggtga gctggggtga 1440

gctgagctgg ggtgagctga gctggggtga gctgagctga gctggggtga gctgagctgg 1500

ggtgagctga gctgagctgg ggtgagctga gctgagctgg ggtgagctga gctagggtga 1560

actgggctgg gtgagctgga gtgagctgag ctgaggtgaa ctggggtgag ccgggatgtt 1620

ttgagttgag ctggggtaag atgagctgaa ctggggtaaa ctgggatgag ctgtggtgag 1680

cggagctgga ttgaactgag ctgtgtgagc tgagctgggg tcagctgagc aagagtgagt 1740

agagctggct ggccagaacc agaatcaatt aggctaagtg agccagattg tgctgggatc 1800

agctgtactc agatgagctg ggatgaggta ggctgggatg agctgggcta gctgacatgg 1860

attatgtgag gctgagctag catgggctgg cctagctgat gagctaagct tgaatgagcg 1920

gggctgagct ggactcagat gtgctagact gagctgtact ggatgatctg gtgtagggtg 1980

atctggactc aactgggctg gctgatggga tgcgccaggt tgaactaggc tcagataagt 2040

taggctgagt agggcctggt tgagatggtt cgggatgagc tgggaaaaga tggactcgga 2100

ccatgaactg ggctgagctg ggttgggaga ccatgaattg agctgaactg agtgcagctg 2160

ggataaactg ggttgagcta agaatagact acctgaattg tgccaaactc ggctgggatc 2220

aattggaaat tatcaggatt tagatgagcc ggactaaact atgctgagct ggactggttg 2280

gatgtgttga actggcctgc tgctgggctg gcatagctga gttgaactta aatgaggaag 2340

gctgagcaag gctagcctgc ttgcatagag ctgaacttta gcctagcctg agctggacca 2400

gcctgagctg agtaggtcta aactgagtta aaaatcaaca gggataattt aacagctaat 2460

ttaacaagcc tgaggtctga gatt 2484

<210> 6
<211> 4515
<212> DNA
<213> Artificial Sequence

<220>
<223> 5′ homology arm of targetting vector

<400> 6
aacctgggca aatgggagct tagcaacaat gtagggggct ggacctagac ttcctacaca 60
tgtgtaacag atgtgcagct tggtcttcat gtgtgtatta ccctaacatt tggagcagga 120
gctgtctctg actctgttgc ctgccattgg atccccttcc cctgcttggg ctgccttgtt 180
tggccttagt aggaaaggat gtgcttagtc ctgctgtgac ttgatgtccc taggcagaat 240
gataccccag gggggctccc catctctgag gagatgggca aagggtaatg gttggaggga 300
cttgtgaggc tgggactggg aggagagaaa ggagacagct gtaactggaa tgatgttaag 360
tgaacaaatg aatggataga ttagatagac agatagacag acagacagac agacagacag 420
acagacagac agacagacag atagaaagat agatagataa ggggaaaaag aaacgtagct 480
gagcaagcca gagagagcaa gccaaataag cagcattcct ccatgacttt tccttcagct 540
cctgcctatg agtctgcctt gacttccctc agtgattggt tgtaagttaa aaggtgaaat 600
aaaccctttc tttgacaagt tgcttttggt tctgattttt atcacagcaa gagaaaatca 660
aactagaaca aacatgtatt tttcctggca catgtccata gtaaggcaga aatgatcttc 720
agacctagac catagatact acagagagca gaagtgtaga taggtggact tactgtatga 780
ttgtaatcca agtaaatcta catagctaga gagctagagg aaaggccaaa gcttcctctg 840
ggaggtcaga tcctgtcgca ctgtagccaa taaggcatat tgcatcacag gaaaggacta 900
agacccaggc tggcaatagt gtctgtatct taactagatc tctctagtga gtgaggaagt 960
aaatttgtga gagcccagac tgtgggctcg gaaggtacct gccatgcccc tgttagtaac 1020
tgagtactac agcaggagca ggtgttctct agaaagcctg agacaactct acttcttctc 1080
tcaagagacc acctaataca ggcctgagag aacagactct ggaaatagat gggacttacg 1140
gagctaagat ctagagctca tctacagagc agaatcccag ccaagagaac aaagaatact 1200
gactctctcc tgttccctac tcctagagtt ctaaaacaca ctatagggaa gggagcctct 1260
agacctccgt ccattcccca tcttgctcat tccatcttcc catgtcccca ggtctccaag 1320
ccacagacac cacctttcct attcacccac ctttctgtgt ccctaggtcc ccaggccata 1380
gtcacctccc cccacacccc gctcaccctg ccccatctat gcccctagat gcttacttac 1440
cagagtcttt tgtctgacgt ggggctacaa gcatctatgc tccctaagca cctactgctg 1500
acctgtagga cccagctctg aaccaactca tataagtaaa tacagactct cccctgtctt 1560
aggatggccc cctgggtcag gaggagacca ctgccaagga accttctctt agagcactga 1620
actcctcccc tgtaccactt aggacagacc tgagacctat tattactgat taccagagct 1680
ctggcagtga ccacggagga gatagatcca ccctggacac aggaaacaca gcaccagaga 1740
tactgcttca tcacaacagt agagtgacac tttagacttt aatttgggtc actttcctgc 1800
tgtagaggtg ggatcagaaa gcaaagagca gtatgagtgc ctgataggca cccaagtaca 1860

ctatagagta ctcatggtga ataaggtacc tccatggctt cccagggagg ggcactgccc 1920

cacccccacc atcacagacc tttctccata gttgataact cagacacaag tgaatgacag 1980

atggacctcc atctgctctt attttaaaaa gaagacaaac cccacaggct cgagaacttt 2040

agcgactgtt ttgagagaaa tcattggtcc ctgactcaag agatgactgg cagattgggg 2100

atcagaatac ccatactctg tggctagtgt gaggtttaag cctcagagtc cctgtggtct 2160

ctgactggtg caaggttttg actaagcgga gcaccacagt gctaactggg accacggtga 2220

cacgtggctc aacaaaaacc ttctgtttgg agctctccag gggcagcctg agctatgagg 2280

aagtagagag gcttgagaaa tctgaggaag aaaagagtag atctgagagg aaaggtagct 2340

ttctggaggt caggagacag tgcagagaag aacgagttac tgtggacagg tcttagatgg 2400

ggaaagaatg agcaaatgca agcatcagaa gggtggatgc aatgtcctgc caaggactta 2460

ccaagaggat ccccggacag agcaggcagg tggagttgac tgagaggaca ggataggtgc 2520

aggtccctct cttgtttcct ttctccttct cctgtttcct tcttctcttg tcacaggtct 2580

cactatgcta gccaaggcta gcctgaaaga ttaccatcct acagatgggc ccatccagtt 2640

gaattaaggt ggagatctct ccaaacatct gagtttctga ggcttggatg ccactgggga 2700

cgccaaggga ctttgggatg ggtttggttg gccccagatg aagggctact tcactgggtc 2760

tataattact ctgatgtcta ggaccagggg gctcaggtca ctcaggtcag gtgagtcctg 2820

catctgggga ctgtggggtt caggtggcct aaggcaggat gtggagagag ttttagtata 2880

ggaacagagg cagaacagag actgtgctac tggtacttcg atgtctgggg cacagggacc 2940

acggtcaccg tctcctcagg taagctggct tttttctttc tgcacattcc attctgaaac 3000

gggaaaagat attctcagat ctccccatgt caggccatct gccacactct gcatgctgca 3060

gaagcttttc tgtaaggata gggtcttcac tcccaggaaa agaggcagtc agaggctagc 3120

tgcctgtgga acagtgacaa tcatggaaaa taggcattta cattgttagg ctacatgggt 3180

agatgggttt ttgtacaccc actaaagggg tctatgatag tgtgactact ttgactactg 3240

gggccaaggc accactctca cagtctcctc aggtgagtcc ttacaacctc tctcttctat 3300

tcagcttaaa tagattttac tgcatttgtt gggggggaaa tgtgtgtatc tgaatttcag 3360

gtcatgaagg actagggaca ccttgggagt cagaaagggt cattgggagc cctggctgac 3420

gcagacagac atcctcagct cccatacttc atggccagag atttataggg atcctggcca 3480

gcattgccgc taggtccctc tcttctatgc tttctttgtc cctcactggc ctccatctga 3540

gatcatcctg gagccctagc caaggatcat ttattgtcag gggtctaatc attgttgtca 3600

caatgtgcct ggtttgctta ctggggccaa gggactctgg tcactgtctc tgcaggtgag 3660

tcctaacttc tcccattcta aatgcatgtt ggggggattc tgggccttca ggaccaagat 3720

tctctgcaaa cgggaatcaa gattcaaccc ctttgtccca aagttgagac atgggtctgg 3780

gtcagggact ctctgcctgc tggtctgtgg tgacattaga actgaagtat gatgaaggat 3840

ctgccagaac tgaagcttga agtctgaggc agaatcttgt ccagggtcta tcggactctt 3900

gtgagaatta ggggctgaca gttgatggtg acaatttcag ggtcagtgac tgtctggttt 3960

ctctgaggtg aggctggaat ataggtcacc ttgaagacta aagaggggtc caggggcttc 4020

tgcacaggca gggaacagaa tgtggaacaa tgacttgaat ggttgattct tgtgtgacac 4080

caggaattgg cataatgtct gagttgccca ggggtgattc tagtcagact ctggggtttt 4140

tgtcgggtat agaggaaaaa tccactattg tgattactat gctatggact actggggtca 4200

aggaacctca gtcaccgtct cctcaggtaa gaatggcctc tccaggtctt tatttttaac 4260

ctttgttatg gagttttctg agcattgcag actaatcttg gatatttgtc cctgagggag 4320

ccggctgaga gaagttggga aataaactgt ctagggatct cagagccttt aggacagatt 4380

atctccacat ctttgaaaaa ctaagaatct gtgtgatggt gttggtggag tccctggatg 4440

atgggatagg gactttggag gctcatttga agaagatgct aaaacaatcc tatggctgga 4500

gggatagttg gggct 4515

<210> 7
<211> 25
<212> DNA
<213> Artificial Sequence

<220>
<223> Oligonucleotide HV2-5

<400> 7
agatcacctt gaaggagtct ggtcc 25

<210> 8
<211> 25
<212> DNA
<213> Artificial Sequence

<220>
<223> Oligonucleotide HV4-4

<400> 8
tggtgaagcc ttcggagacc ctgtc 25

<210> 9
<211> 25
<212> DNA
<213> Artificial Sequence

<220>
<223> Oligonucleotide HV1-3

<400> 9
cactagctat gctatgcatt gggtg 25

<210>  10
<211>  25
<212>  DNA
<213>  Artificial Sequence

<220>
<223>  Oligonucleotide HV1-2

<400>  10
atggatcaac cctaacagtg gtggc                                          25


<210>  11
<211>  25
<212>  DNA
<213>  Artificial Sequence

<220>
<223>  Oligonucleotide HV6-1

<400>  11
ggaaggacat actacaggtc caagt                                          25


<210>  12
<211>  25
<212>  DNA
<213>  Artificial Sequence

<220>
<223>  Oligonucleotide Cμ

<400>  12
   taggtacttg ccccctgtcc tcagt                                         25


<210>  13
<211>  25
<212>  DNA
<213>  Artificial Sequence

<220>
<223>  Oligonucleotide KV1-9

<400>  13
agcccagtgt gttccgtaca gcctg                                          25


<210>  14
<211>  25
<212>  DNA
<213>  Artificial Sequence

<220>
<223>  Oligonucleotide KV1-8

<400>  14
atcctcattc tctgcatcta cagga                                          25


<210>  15

<211>  25
<212>  DNA
<213>  Artificial Sequence

<220>
<223>  Oligonucleotide KV1-6

<400>  15
ggtaaggatg gagaacactg gcagt                                             25


<210>  16
<211>  25
<212>  DNA
<213>  Artificial Sequence

<220>
<223>  Oligonucleotide KV1-5

<400>  16
ttagtagctg gttggcctgg tatca                                             25


<210>  17
<211>  25
<212>  DNA
<213>  Artificial Sequence

<220>
<223>  Oligonucleotide Ck

<400>  17
ctttgctgtc ctgatcagtc caact                                             25

## Description

### Background

The present invention relates inter alia to non-human animals and cells that are engineered to contain exogenous DNA, such as human immunoglobulin gene DNA, their use in medicine and the study of disease, methods for production of non-human animals and cells, and antibodies and antibody chains produced by such animals and derivatives thereof.

In order to get around the problems of humanizing antibodies a number of companies set out to generate mice with human immune systems. The strategy used was to knockout the heavy and light chain loci in ES cells and complement these genetic lesions with transgenes designed to express the human heavy and light chain genes. Although fully human antibodies could be generated, these models have several major limitations:
(i) The size of the heavy and light chain loci (each several Mb) made it impossible to introduce the entire loci into these models. As a result the transgenic lines recovered had a very limited repertoire of V-regions, most of the constant regions were missing and important distant enhancer regions were not included in the transgenes.
(ii) The very low efficiency of generating the large insert transgenic lines and the complexity and time required to cross each of these into the heavy and light chain knockout strains and make them homozygous again, restricted the number of transgenic lines which could be analysed for optimal expression.
(iii) Individual antibody affinities rarely reached those which could be obtained from intact (non-transgenic) animals.

WO2007117410 discloses chimaeric constructs for expressing chimaeric antibodies.

WO2010039900 discloses knock in cells and mammals having a genome encoding chimaeric antibodies.

The present invention provides, inter alia, a process for the generation in non-human mammals of antibodies that comprise a human Ig variable region, and further provides non-human animal models for the generation of such antibodies.

### Summary of the Invention

In one aspect the invention relates to a non-human mammal whose genome comprises:
(a) a plurality of human IgH V regions, one or more human D regions and one or more human J regions upstream of the host non-human mammal constant region; and
(b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region;
wherein the non-human mammal is able to produce a repertoire of chimaeric antibodies, or chimaeric light or heavy chains, having a non-human mammal constant region and a human variable region.

In one aspect the invention relates to non-human mammal whose genome comprises
(a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
(b) optionally one or more human IgH V regions, one or more human D regions and one or more human J regions upstream of the host non-human mammal constant region;
wherein the non-human mammal is able to produce a repertoire of chimaeric antibodies, or chimaeric light or heavy chains, having a non-human mammal constant region and a human variable region.

In one aspect the invention relates to non-human mammalian cell whose genome comprises
(a) a plurality of human IgH V regions, one or more human D regions and one or more human J regions upstream of the host non-human mammal constant region and
(b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region.

In one aspect the invention relates to a non-human mammalian cell whose genome comprises
(a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
(b) optionally one or more human IgH V regions, one or more human D regions and one or more human J regions upstream of the host non-human mammal constant region;

In a further aspect the invention relates to a method for producing a non-human cell or mammal comprising inserting into a non-human mammal cell genome, such as an ES cell genome;
(a) a plurality of human IgH V regions, one or more human D regions and one or more human J regions upstream of the host non-human mammal constant region; and
(b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region; respectively,
the insertion being such that the non-human cell or mammal is able to produce a repertoire of chimaeric antibodies having a non-human mammal constant region and a human variable region, wherein steps (a) and (b) can be carried out in either order and each of steps (a) and (b) can be carried out in a stepwise manner or as a single step.

Insertion may be by homologous recombination.

In a further aspect the invention relates to a method for producing an antibody or antibody chain specific to a desired antigen the method comprising immunizing a transgenic non-human mammal as disclosed herein with the desired antigen and recovering the antibody or antibody chain.

In a further aspect the invention relates to a method for producing a fully humanised antibody comprising immunizing a transgenic non-human mammal as disclosed herein with the desired antigen, recovering the antibody or cells producing the antibody and then replacing the non-human mammal constant region with a human constant region, for example by protein or DNA engineering.

In a further aspect the invention relates to humanised antibodies and antibody chains produced according to the present invention, both in chimaeric (for example, mouse-human) and fully humanised form, as well as fragments and derivatives of said antibodies and chains, and use of said antibodies, chains and fragments in medicine, including diagnosis.

In a further aspect the invention relates to use of a non-human mammal as described herein as a model for the testing of drugs and vaccines.

### Figures

Figures 1 - 8 show an iterative process for insertion of a series of human BACs into a mouse Ig locus.
Figures 9 - 18 show in more detail the process of figures 1-8 for the IgH and kappa locus
Figures 19 and 20 show the principles behind antibody generation in chimaeric mice.

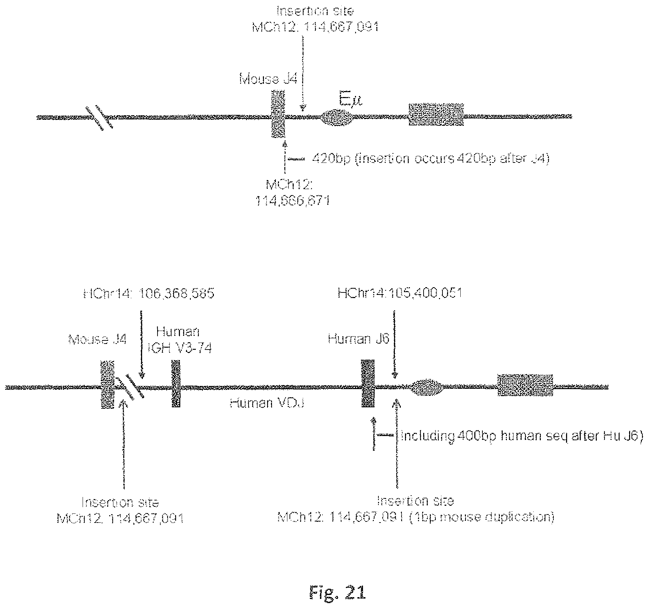

Figure 21 shows a possible insertion site for the human DNA in a mouse chromosome.
Figures 22 - 26 disclose an alternative iterative process for insertion of a series of human BACs into a mouse Ig locus.
Figure 27 - 29 illustrate a mechanism for inversion of the host VDJ region
Figure 30 illustrates proof of principle for insertion of a plasmid using an RMCE approach.
Figure 31 illustrates sequential RMCE - Integration into Landing Pad
Figure 32 illustrates confirmation of Successful Insertion into Landing Pad
Figure 33 illustrates PCR Confirmation of 3' End Curing
Figure 34 illustrates insertion of BAC#1 and PCR Diagnostics
Fig.35. illustrates JH and JK usage
Fig. 36. illustrates DH usage
Fig. 37. illustrates the distribution of CDR-H3 length in human VDJCµ transcripts from chimera mice
Fig. 38. illustrates the distribution of nucleotide numbers of deletion and insertion in IGH-VDI or IGK-VJ junctions

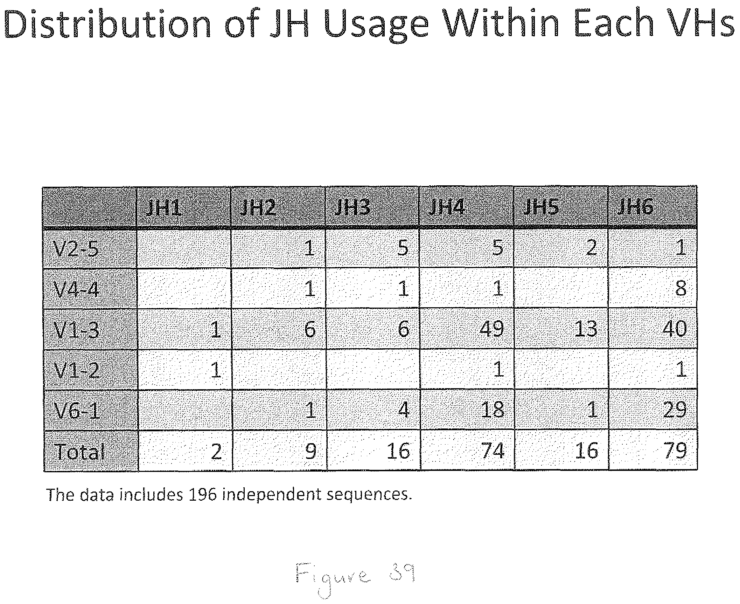

Fig 39 illustrates Distribution of JH Usage Within Each VHs

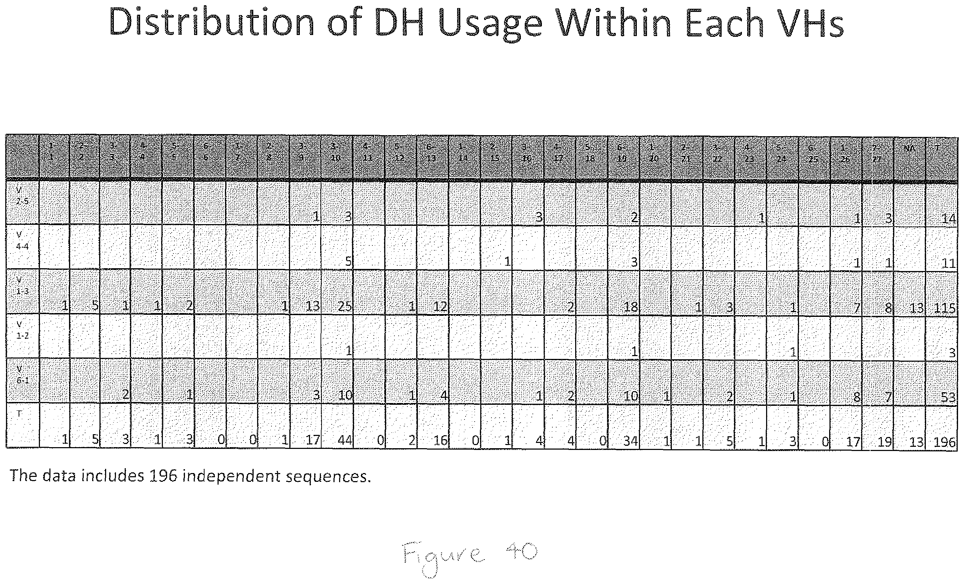

Fig 40 illustrates Distribution of DH Usage Within Each VHs
Fig 41 illustrates Nucleotide Gain or Loss at VJ Joints Generates IGK Variants
Fig 42 illustrates Hypermutaion in J Regions Generates IGK Variants
Fig 43 illustrates Joint Diversity Produces Functional CDS

Seq ID no 1 is that of a Rat switch sequence
Seq ID no 2 is that of a landing pad targeting vector (long version)
Seq ID no 3 is that of landing pad targeting vector (shorter version)
SEQ ID no 4 is the mouse strain 129 switch
SEQ ID 5 is the mouse strain C57 switch
SEQ ID No 6 is the 5' homology arm of the landing pad
SEQ ID No 7 is oligo HV2-5
SEQ ID No 8 is oligo HV4-4
SEQ ID No 9 is oligo HV1-3
SEQ ID No 10 is oligo HV1-2
SEQ ID No 11 is oligo HV6-1
SEQ ID No 12 is oligo Cµ
SEQ ID No 13 is oligo KV1-9
SEQ ID No 14 is oligo KV1-8
SEQ ID No 15 is oligo KV1-6
SEQ ID No 16 is oligo KV1-5
SEQ ID No 17 is oligo Cκ

### General Description

All nucleotide co-ordinates for the mouse are those corresponding to NCBI m37 for the mouse C57BL/6J strain, e.g. April 2007 ENSEMBL Release 55.37h, e.g. NCBI37 July 2007 (NCBI build 37) (e.g. UCSC version mm9 see www.genome.ucsc.edu and http://genome.ucsc.edu/FAQ/FAQreleases.html) unless otherwise specified. Human nucleotides coordinates are those corresponding to GRCh37 (e.g. UCSC version hg 19, http://genome.ucsc.edu/FAQ/FAQreleases.html), Feb 2009 ENSEMBL Release 55.37, or are those corresponding to NCBI36, Ensemble release 54 unless otherwise specified. Rat nucleotides are those corresponding to RGSC 3.4 Dec 2004 ENSEMBL release 55.34w, or Baylor College of Medicine HGSC v3.4 Nov 2004(e.g., UCSC rn4, see www.genome.ucsc.edu and http://genome.ucsc.edu/FAQ/FAQreleases.html) unless otherwise specified.

In the present invention we disclose methods for the construction of chimaeric human heavy and light chain loci in a non-human mammal, for example a mouse. Reference to work in mice herein is by way of example only, and reference to mice is taken to include reference to all non-human mammals unless otherwise apparent from the disclosure, with mice being preferred as the non-human mammal.

In one aspect the invention relates to a non-human mammal whose genome comprises:
(a) a plurality of human IgH V regions, one or more human D regions and one or more human J regions upstream of the host non-human mammal constant region; and
(b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region;
wherein the non-human mammal is able to produce a repertoire of chimaeric antibodies or antibody chains having a non-human mammal constant region and a human variable region.

In a further aspect the invention relates to a non-human mammal whose genome comprises:
(a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
(b) optionally one or more human IgH V regions, one or more human D regions and one or more human J regions upstream of the host non-human mammal constant;
wherein the non-human mammal is able to produce a repertoire of chimaeric antibodies having a non-human mammal constant region and a human variable region.

Optionally the non-human mammal genome is modified to prevent expression of fully host-species specific antibodies.

In one aspect the inserted human DNA comprises at least 50% of the human heavy chain variable (V) genes, such as at least 60%, at least 70%, at least 80%, at least 90%, and in one aspect all of the human V genes.

In one aspect the inserted human DNA comprises at least 50% of the human heavy chain diversity (D) genes, such as at least 60%, at least 70%, at least 80%, at least 90%, and in one aspect all of the human D genes.

In one aspect the inserted human DNA comprises at least 50% of the human heavy chain joining (J) genes, such as at least 60%, at least 70%, at least 80%, at least 90%, and in one aspect all of the human J genes.

In one aspect the inserted human DNA comprises at least 50% of the human light chain Variable (V) genes, such as at least 60%, at least 70%, at least 80%, at least 90%, and in one aspect all of the human light chain V genes.

In one aspect the inserted human DNA comprises at least 50% of the human light chain joining (J) genes, such as at least 60%, at least 70%, at least 80%, at least 90%, and in one aspect all of the human light chain J genes.

The inserted human genes may be derived from the same individual or different individuals, or be synthetic or represent human consensus sequences.

Although the number of V D and J regions is variable between human individuals, in one aspect there are considered to be 51 human V genes, 27 D and 6 J genes on the heavy chain, 40 human V genes and 5 J genes on the kappa light chain and 29 human V genes and 4 J genes on the lambda light chain (Janeway and Travers, Immunobiology, Third edition)

In one aspect the human heavy chain locus inserted into the non-human mammal contains the full repertoire of human V, D and J regions, which in the genome is in functional arrangement with the non-human mammal constant regions such that functional chimaeric antibodies can be produced between the human variable and non-human mammal constant regions. This total inserted human heavy chain genetic material is referred to herein as the human IgH VDJ region, and comprises DNA from a human genome that encodes all the exons encoding human V,D and J portions and suitably also the associated introns. Similarly, reference to the human Ig light chain kappa V and J regions herein refers to human DNA comprising all the exons encoding V and J regions and suitably also the associated introns of the human genome. Reference to the human Ig light chain lambda V and J regions herein refers to human DNA comprising all the exons encoding V and J regions and suitably also the associated introns of the human genome.

Human variable regions are suitably inserted upstream of a non-human mammal constant region, the latter comprising all of the DNA required to encode the full constant region or a sufficient portion of the constant region to allow the formation of an effective chimaeric antibody capable of specifically recognising an antigen.

In one aspect the chimaeric antibodies or antibody chains have a part of a host constant region sufficient to provide one or more effector functions seen in antibodies occurring naturally in a host mammal, for example that they are able interact with Fc receptors, and/or bind to complement.

Reference to a chimaeric antibody or antibody chain having a host non mammal constant region herein therefore is not limited to the complete constant region but also includes chimaeric antibodies or chains which have all of the host constant region, or a part thereof sufficient to provide one or more effector functions. This also applies to non-human mammals and cells and methods of the invention in which human variable region DNA may be inserted into the host genome such that it forms a chimaeric antibody chain with all or part of a host constant region. In one aspect the whole of a host constant region is operably linked to human variable region DNA.

The host non-human mammal constant region herein is preferably the endogenous host wild-type constant region located at the wild type locus, as appropriate for the heavy or light chain. For example, the human heavy chain DNA is suitably inserted on mouse chromosome 12, suitably adjacent the mouse heavy chain constant region.

In one aspect the insertion of the human DNA, such as the human VDJ region is targeted to the region between the J4 exon and the Cµ locus in the mouse genome IgH locus, and in one aspect is inserted between co-ordinates 114,667,090 and 114,665,190, or at co-ordinate 114,667,091, after 114,667,090. In one aspect the insertion of the human DNA, such as the human light chain kappa VJ is targeted into mouse chromosome 6 between co-ordinates 70,673,899 and 70,675,515, suitably at position 70,674,734, or an equivalent position in the lambda mouse locus on chromosome 16.

In one aspect the host non-human mammal constant region for forming the chimaeric antibody may be at a different (non endogenous) chromosomal locus. In this case the inserted human DNA, such as the human variable VDJ or VJ region(s) may then be inserted into the non-human genome at a site which is distinct from that of the naturally occurring heavy or light constant region. The native constant region may be inserted into the genome, or duplicated within the genome, at a different chromosomal locus to the native position, such that it is in a functional arrangement with the human variable region such that chimaeric antibodies of the invention can still be produced.

In one aspect the human DNA is inserted at the endogenous host wild-type constant region located at the wild type locus between the host constant region and the host VDJ region.

Reference to location of the variable region upstream of the non-human mammal constant region means that there is a suitable relative location of the two antibody portions, variable and constant, to allow the variable and constant regions to form a chimaeric antibody or antibody chain *in vivo* in the mammal. Thus, the inserted human DNA and host constant region are in functional arrangement with one another for antibody or antibody chain production.

In one aspect the inserted human DNA is capable of being expressed with different host constant regions through isotype switching. In one aspect isotype switching does not require or involve trans switching. Insertion of the human variable region DNA on the same chromosome as the relevant host constant region means that there is no need for trans-switching to produce isotype switching.

As explained above, the transgenic loci used for the prior art models were of human origin, thus even in those cases when the transgenes were able to complement the mouse locus so that the mice produced B-cells producing fully human antibodies, individual antibody affinities rarely reached those which could be obtained from intact (non-transgenic) animals. The principal reason for this (in addition to repertoire and expression levels described above) is the fact that the control elements of the locus are human. Thus, the signalling components, for instance to activate hyper-mutation and selection of high affinity antibodies are compromised.

In contrast, in the present invention, host non-human mammal constant regions are maintained and it is preferred that at least one non-human mammal enhancer or other control sequence, such as a switch region, is maintained in functional arrangement with the non-human mammal constant region, such that the effect of the enhancer or other control sequence, as seen in the host mammal, is exerted in whole or in part in the transgenic animal.

This approach above is designed to allow the full diversity of the human locus to be sampled, to allow the same high expression levels that would be achieved by non-human mammal control sequences such as enhancers, and is such that signalling in the B-cell, for example isotype switching using switch recombination sites, would still use non-human mammal sequences.

A mammal having such a genome would produce chimaeric antibodies with human variable and non-human mammal constant regions, but these could be readily humanized, for example in a cloning step. Moreover the *in vivo* efficacy of these chimaeric antibodies could be assessed in these same animals.

In one aspect the inserted human IgH VDJ region comprises, in germline configuration, all of the V, D and J regions and intervening sequences from a human.

In one aspect 800-1000kb of the human IgH VDJ region is inserted into the non-human mammal IgH locus, and in one aspect a 940, 950 or 960 kb fragment is inserted. Suitably this includes bases 105,400,051 to 106,368,585 from human chromosome 14. In one aspect the inserted IgH human fragment consists of bases 105,400,051 to 106,368,585 from chromosome 14. In one aspect the inserted human heavy chain DNA, such as DNA consisting of bases 105,400,051 to 106,368,585 from chromosome 14, is inserted into mouse chromosome 12 between the end of the mouse J4 region and the Eµ region, suitably between co-ordinates 114,667,090 and 114,665,190, or at co-ordinate 114,667,091, after 114,667,090 . In one aspect the insertion is between co-ordinates 114,667,089 and 114,667,090 (co-ordinates refer to NCBI m37, for the mouse C57BL/6J strain), or at equivalent position in another non-human mammal genome.

In one aspect the inserted human kappa VJ region comprises, in germline configuration, all of the V and J regions and intervening sequences from a human. Suitably this includes bases 88,940,356 to 89,857,000 from human chromosome 2, suitably approximately 917kb. In a further aspect the light chain VJ insert may comprise only the proximal clusters of V segments and J segments. Such an insert would be of approximately 473 kb. In one aspect the human light chain kappa DNA, such as the human IgK fragment of bases 88,940,356 to 89,857,000 from human chromosome 2, is suitably inserted into mouse chromosome 6 between co-ordinates 70,673,899 and 70,675,515, suitably at position 70,674,734. These co-ordinates refer to NCBI36 for the human genome, ENSEMBL Release 54 and NCBIM37 for the mouse genome, relating to mouse strain C57BL/6J..

In one aspect the human lambda VJ region comprises, in germline configuration, all of the V and J regions and intervening sequences from a human.

Suitably this includes analogous bases to those selected for the kappa fragment, from human chromosome 2.

A cell or non-human mammal of the invention, in one embodiment, comprises an insertion of human heavy chain variable region DNA between co-ordinates 114, 666, 183 and 114, 666, 725, such as between 114 666 283 and 114 666 625, optionally between co-ordinates 114,666,335 and 114,666,536, optionally between 114,666,385 and 114,666,486, or between 114,666,425 and 114,666,446, or between 114,666,435 and 114,666,436 of mouse chromosome 12 with reference to NCBIM37 for the mouse genome, relating to mouse strain C57BL/6J or an equivalent position of mouse chromosome 12 from a different mouse strain or an equivalent position in the genome of another non-human vertebrate, e.g., a rat. The insertion between co-ordinates 114,666,435 and 114,666,436 relating to mouse strain C57BL/6J is equivalent to an insertion between co-ordinates 1207826 and 1207827 on chromosome 12 with reference to the 129/SvJ genomic sequence of the geneBank access number NT114985.2. An insertion may be made at equivalent position in another genome, such as another mouse genome. In an example of this embodiment, the cell or mammal of the invention comprises a human IgH VDJ region which comprises or consists of nucleotides 106,328,851-107,268,544, such as nucleotides 106,328,901-107,268,494, such as nucleotides 106,328,941-107,268,454, such as nucleotides 106,328,951-107,268,444 of human Chromosome 14, with reference to the GRCH37/hg19 sequence database, or insertion of equivalent nucleotides relating to chromosome 14 from a different human sequence or database. The human insertion may be made between the regions indicated above.

A cell or mammal of the invention, in one embodiment, comprises an insertion of the human kappa VJ region, suitably comprising or consisting of, in germline configuration, all of the V and J regions and intervening sequences from a human, the insertion of the human DNA being made between co-ordinates 70,673,918 - 70,675,517, such as between co-ordinates 70, 674,418 and 70 675, 017, such as between co-ordinates 70,674, 655 - 70,674,856, such as between co-ordinates 70,674, 705 - 70,674,906, such as between co-ordinates 70,674, 745 - 70,674,766, such as between co-ordinates 70,674,755 and 70,674,756 of mouse chromosome 6, numbering with reference to NCBIM37 for the mouse genome, relating to mouse strain C57BL/6J, or an insertion at an equivalent position in another genome, such as another mouse genome. In an example of this embodiment, a cell or mammal of the invention comprises an insertion of nucleotides 89,159,079-89,630,437 and/or 89,941,714-90,266,976 of human chromosome 2 with reference to the GRCH37/hg19 sequence database (or equivalent nucleotides relating to chromosome 2 from a different human sequence or database), such as an insertion of these 2 discrete fragments without the intervening sequence, or an insertion of the complete 89,159,079-90,266,976 region.

The insertion may comprise, or consist, of:
(i) nucleotides 89,158,979 - 89,630,537, such as 89,159,029-89,630,487, such as 89,159,069-89,630,447, such as 89,159,079 - 89,630,437, optionally in addition to fragment (ii) below
(ii) nucleotides 89,941,614 - 90,267,076, such as 89,941,664 - 90,267,026, such as 89, 941,704-90,266,986, such as 89,941,714 - 90,266,976; optionally in addition to fragment (i)
(iii) nucleotides 89,158,979 - 90,267,076, such as nucleotides 89,159,079 - 90,266,976.

The human insertion may be made between the regions indicated above.

In an embodiment, a cell or mammal of the invention comprises an insertion of a human lambda region which comprises at least one human Jλ region (eg, a germline region) and at least one human Cλ region (eg, a germline region), optionally C_{λ}6 and/or C_{λ}7. For example, the cell or mammal comprises a plurality of human Jλ regions, optionally two or more of J_{λ}1, J_{λ}2, J_{λ}6 and J_{λ}7, optionally all of J_{λ}1, J_{λ}2, J_{λ}6 and J_{λ}7. In an example, the cell or mammal comprises at least one human J_{λ}-C_{λ} cluster, optionally at least J_{λ}7-C_{λ}7.

In one aspect the human JC cluster is inserted 3' of the last endogenous J lambda or is inserted 3' of the last endogenous J kappa region, suitably immediately 3' of these sequences, or substantially immediately 3' of these sequences.

In one aspect the insertion into the mouse lambda locus is made downstream of the endogenous C1 gene segment, for example where there is a 3' J1C1 cluster, suitably immediately 3' of the C1 segment, or substantially immediately 3' of the segment.

In one aspect (e.g. cell or non-human mammal) a human JC cluster is inserted into a kappa locus and any resulting cell or animal is heterozygous at that locus, such that the cell has one chromosome with human lambda DNA inserted into the kappa locus, and another chromosome with human kappa DNA at the endogenous kappa locus.

In an embodiment, a cell or mammal of the invention comprises a human Eλ enhancer.

A cell or mammal may of the invention comprise an inserted human lambda VJ region, suitably comprising or consisting of, in germline configuration, all of the V and J regions and intervening sequences from a human, the inserted region comprises or consisting of nucleotides 22,375,509 - 23,327,984, such as nucleotides 22,375,559 - 23,327,934, such as nucleotides 22,375,599 - 23,327,894, such as nucleotides 22,375,609 - 23,327,884 from human Chromosome 22, with reference to the GRCH37/hg19 sequence database, or equivalent DNA from another human sequence or database. The insertion into the mouse genome may be made between co-ordinates 19,027,763 and 19,061,845, such as between co-ordinates 19, 037, 763 and 19, 051, 845, such as between co-ordinates 19,047,451 and 19,047,652, such as between co-ordinates 19,047,491 and 19,047,602, such as between co-ordinates 19,047,541 and 19,047,562, such as between co-ordinates 19,047,551 and 19,047,552 of mouse Chromosome 16 (with reference to NCBIM37 for the mouse genome, relating to mouse strain C57BL/6J, equivalent to co-ordinates 1,293,646 - 1,293,647 of the 129 SvJ genomic sequence in the sequence file of NT_039630.4), or may be an insertion at an equivalent position in other genome, such as another mouse genome. The insertion of the human lambda nucleic acid into the mouse genome may alternatively be made between co-ordinates 70,673,918 and 70,675,517, such as between co-ordinates 70, 674,418 and 70 675, 017, such as between co-ordinates 70,674,655 and 70,674,856, such as between co-ordinates 70,674,705 and 70,674,806, such as between co-ordinates 70,674,745 and 70,674,766, such as between co-ordinates 70,674,755 and 70,674,756 of mouse Chromosome 6 (with reference to NCBIM37 for the mouse genome, relating to mouse strain C57BL/6J) or equivalent in another genome. The human insertion may be made between the regions indicated above.

All specific human fragments described above may vary in length, and may for example be longer or shorter than defined as above, such as 500 bases, 1KB, 2K, 3K, 4K, 5KB, 10 KB, 20KB, 30KB, 40KB or 50KB or more, which suitably comprise all or part of the human V(D)J region, whilst preferably retaining the requirement for the final insert to comprise human genetic material encoding the complete heavy chain region and light chain region, as appropriate, as described above.

In one aspect the 5' end of the human insert described above is increased in length. Where the insert is generated in a stepwise fashion then the increase in length is generally in respect of the upstream (5') clone.

In one aspect the 3' end of the last inserted human gene, generally the last human J gene to be inserted is less than 2kb, preferably less than 1KB from the human-mouse join region.

In one aspect the non-human mammal comprises some or all of the human light chain kappa VJ region as disclosed herein but not the human light chain lambda VJ region.

In one aspect the cell or non-human mammal comprises a fully human lambda locus (lambda VJC regions from a human), a chimaeric kappa locus (human kappa VJ regions operatively linked to a host kappa constant region) and a chimaeric heavy chain locus, having a human VDJ region operatively linked to a host heavy chain constant region.

In a further aspect the genome comprises an insertion of V, D (heavy chain only) and J genes as described herein at the heavy chain locus and one light chain locus, or at the heavy chain locus and both light chain loci. Preferably the genome is homozygous at one, or both, or all three loci.

In another aspect the genome may be heterozygous at one or more of the loci, such as heterozygous for DNA encoding a chimaeric antibody chain and native (host cell) antibody chain. In one aspect the genome may be heterozygous for DNA capable of encoding 2 different antibody chains of the invention, for example, comprising 2 different chimaeric heavy chains or 2 different chimaeric light chains.

In one aspect the invention relates to a non-human mammal or cell, and methods for producing said mammal or cell, as described herein, wherein the inserted human DNA, such as the human IgH VDJ region and/or light chain V, J regions are found on only one allele and not both alleles in the mammal or cell. In this aspect a mammal or cell has the potential to express both an endogenous host antibody heavy or light chain and a chimaeric heavy or light chain.

In a further aspect of the invention the human VDJ region, or light chain VJ region, is not used in its entirety, but parts of the equivalent human VDJ or VJ region, such as the exons, from other species may be used, such as one or more V, D, or J exons from other species, or regulatory sequences from other species. In one aspect the sequences used in place of the human sequences are not human or mouse. In one aspect the sequences used may be from rodent, or, primate such as chimp. For example, 1, 2, 3, 4, or more, or all of the J regions from a primate other than a human may be used to replace, one, 2, 3, 4, or more or all of the human J exons in the VDJ/VJ region of the cells and animals of the invention.

In a further aspect the inserted human DNA, such as the human IgH VDJ region, and/or light chain VJ regions, may be inserted such that they are operably linked in the genome with a mu constant region from a non-human, non-mouse species, such as a rodent or primate sequence, such as a rat sequence.

Other non-human, non-mouse species from which DNA elements may be used in the present invention include rabbits, lamas, dromedary, alpacas, camels and sharks.

In one aspect the inserted human DNA, such as the human VDJ or VJ region, is not operably linked to the endogenous host mu sequence but rather to a non-host mu sequence.

Operable linkage suitably allows production of an antibody heavy or light chain comprising the human variable region.

In one aspect the inserted human DNA, such as the human IgH VDJ region (and/or light chain VJ regions) may be inserted into the host chromosome together with mu constant region nucleic acid which is not host mu constant region nucleic acid, and preferably is a mu constant region from a non-mouse, non-human species. Suitably the inserted human DNA, such as the human VDJ region (and/or light chain VJ regions) is operably linked to a non-human, non-mouse mu, and is able to form a chimaeric antibody heavy or light chain. In another aspect a non-mouse, non-human mu may be inserted into the host chromosome on a separate genetic element to that of the human variable region, or at a different location in the genome, suitably operably linked to the variable region such that a chimaeric antibody heavy or light can be formed.

In an additional aspect the invention relates to a non-human mammal or a cell whose genome comprises a plurality of human IgH V regions, one or more human D regions and one or more human J regions upstream of a host non-human mammal light chain constant region, arranged such that the cell or mammal is able to express a chimaeric antibody chain. The invention also relates to a non-human mammal or a cell whose genome additionally or alternatively comprises a plurality of human Ig light chain V regions, and one or more human J regions upstream of a host non-human mammal heavy chain constant region, such that the cell or mammal is able to express a chimaeric antibody chain. The cell or mammal may be able to express an antibody having both heavy and light chains, including at least one chimaeric antibody chain, as disclosed above.

The inserted human heavy chain variable regions may be any of those described herein, and may be inserted at the positions described above for insertion 5' of the lambda and kappa constant regions. Likewise the inserted human light chain variable regions may be those described above, and may be inserted at the positions described above for insertion 5' of the heavy chain constant region.

For example, the genome or the cell or non-human mammal of the invention may encode an antibody comprising an antibody chain having a human heavy chain variable region upstream of a mouse light chain constant region, or an antibody chain having a human light chain variable region upstream of a mouse heavy chain constant region, in combination with one of:
a fully human antibody light chain;
a fully human antibody heavy chain;
a non-human vertebrate (e.g., mouse or rat) antibody light chain;
a non-human vertebrate (e.g., mouse or rat) antibody heavy chain;
a chimaeric non-human vertebrate (e.g., mouse or rat) - human antibody chain;
an antibody chain having a human heavy chain variable region upstream of a non-human vertebrate (e.g., mouse or rat) light chain constant region;
an antibody chain having a human light chain variable region upstream of a non-human vertebrate (e.g., mouse or rat) heavy chain constant region.

The invention also relates to a transgene encoding a plurality of human IgH V regions, one or more human D regions and one or more human J regions upstream of a host non-human mammal light chain constant region, optionally comprised within a vector.

The invention also relates to a transgene encoding a plurality of human Ig light chain V regions, and one or more human light chain J regions upstream of a host non-human mammal heavy chain constant region, optionally comprised within a vector.

In one aspect the invention relates to a cell, or non-human mammal, the genome of which comprises: one or more human Ig light chain kappa V regions and one or more human Ig light chain kappa J regions upstream of all or part of the human kappa constant region.

In another aspect the invention relates to a cell, or non-human mammal, the genome of which comprises: one or more human Ig light chain lambda V regions and one or more human Ig light chain lambda J regions upstream of all or part of the human lambda constant region.

Suitably the light chain VJ and C regions are able to form antibody chains in vivo capable of specifically reacting with an antigen.

In one aspect of the invention there is no non-human coding sequence in the inserted light chain region.

In such aspects a human kappa and/or lambda region is inserted into the genome, in combination with insertion of the heavy chain VDJ region or part thereof, upstream of the host heavy chain constant region as disclosed herein.

The cell or non-human mammal of the invention may comprise:
(a) a plurality of human IgH V regions, one or more human D regions and one or more human J regions upstream of the host non-human mammal constant region; and
(b) one or more human Ig light chain kappa V regions and one or more human Ig light chain kappa J regions upstream of all or part of the non-human kappa constant region,
wherein the non-human mammal is able to produce a repertoire of antibodies having an antibody chain comprising non-human mammal constant region and a human variable region.

The cell or non-human mammal of the invention may comprise
(a) a plurality of human IgH V regions, one or more human D regions and one or more human J regions upstream of the host non-human mammal constant region; and
one or more human Ig light chain lambda V regions and one or more human Ig light chain lambda J regions upstream of the host non-human mammal lambda constant region;
wherein the non-human mammal is able to produce a repertoire of antibodies having an antibody chain comprising a non-human mammal constant region and a human variable region.

Suitably the insertion of the human VJC light chain DNA, or part thereof as disclosed above, is made at the equivalent mouse locus. In one aspect the human light chain kappa VJC DNA, or part thereof, is inserted immediately upstream or downstream of the mouse kappa VJC region. In one aspect, the human light chain lambda VJC region or part thereof is inserted immediately upstream or downstream of the mouse lambda VJC region. In one aspect only the human kappa VJC locus is inserted and not the human lambda VJC locus. In one aspect only the human lambda VJC locus is inserted and not the human kappa VJC locus. Insertions may be made using the techniques disclosed herein, and suitably do not remove the host sequences from the genome. In one aspect the non-human mammal host VJC sequences may be inactivated in some way, by mutation, or inversion, or by insertion of the human variable region DNA, or by any other means. In one aspect the cell or non-human mammal of the invention may comprise an insertion of the complete VJC human region.

The human kappa variable region DNA might be inserted into the genome in functional arrangement with a lambda constant region, for example inserted upstream of a lambda constant region. Alternatively human lambda region variable DNA might be inserted in functional arrangement with a kappa constant region, for example inserted upstream of a kappa constant region.

In one aspect one or more non-human mammal control sequences such as the enhancer sequence(s) is maintained upstream of the nonhuman mammal Mu constant region, suitably in its native position with respect to the distance from the constant region.

In one aspect one or more non-human mammal control sequences such as an enhancer sequence(s) are maintained downstream of the nonhuman mammal Mu constant region, suitably in its native position with respect to the distance from the constant region.

In one aspect a non-human mammal switch sequence, suitably the endogenous switch sequence, is maintained upstream of the non-human mammal Mu constant region, suitably in its native position with respect to distance from the constant region.

In such location the host enhancer or switch sequences are operative in vivo with the host constant region sequence(s).

In one aspect a switch sequence is neither human, nor native in the non-human mammal, for example in one aspect a non-human mammal switch sequence is not a mouse or human switch sequence. The switch sequence may be, for example, a rodent or primate sequence, or a synthetic sequence. In particular the switch sequence may be a rat sequence where the non-human mammal is a mouse. By way of example, a mouse or human constant mu sequence may be placed under the control of a switch sequence from a rat, or chimp, or other switch sequence, suitably capable of allowing isotype switching to occur in vivo.

In one aspect the switch sequence of the invention is a switch sequence comprising 3, 4, 5, 6 or more contiguous repeats of the repeat sequence GGGCT, such as a rat switch sequence. By "rat switch" herein it is meant that the switch is a wild-type switch corresponding to a switch from a rat genome or derived from such a switch.

In one aspect the switch sequence of the invention is a rat switch sequence comprising the following repeats: GAGCT (296 repeats), GGGGT (50 repeats), and GGGCT (83 repeats).

In one example the rat switch sequence comprises or consists of the sequence of SEQ ID no 1.

In these embodiments, and where the non-human mammal is a mouse or the cell is a mouse cell, the switch is optionally a rat switch as described herein.

Alternatively, the switch sequence present in cells or mammal of the invention is a mouse switch, eg, is from a mouse such as a mouse 129 strain or mouse C57 strain, or from a strain derived therefrom, optionally comprising or consisting of the sequence of SEQ ID no 4 or 5. By "mouse switch" herein it is meant that the switch is a wild-type switch corresponding to a switch from a mouse genome or derived from such a switch. In this embodiment, and where the non-human mammal is a mouse or the cell is a mouse cell, the mouse switch sequence is optionally the endogenous switch or is a mouse switch from another mouse strain.

The cell or mammal of the invention may therefore comprise a human or non-human mammal switch sequence and a human or non-human mammal enhancer region or regions. They may be upstream of a human or non-human mammal constant region. Preferably the control sequences are able to direct expression or otherwise control the production of antibodies comprising a constant region with which they are associated. One combination envisaged is a rat switch with mouse enhancer sequences and mouse constant regions in a mouse cell.

In one aspect the invention relates to a cell, preferably a non-human cell, or non-human mammal comprising an immunoglobulin heavy chain or light chain locus having DNA from 3 or more species. For example, the cell or animal may comprise host cell constant region DNA, one or more human V, D or J coding sequences and one or more non-human, non-host DNA regions that are able to control a region of the immunoglobulin locus, such as a switch sequence, promoter or enhancer which are able to control expression or isotype switching in vivo of the Ig DNA. In one aspect the cell or animal is a mouse and comprises additionally human DNA from the human Ig locus and additionally a non-mouse DNA sequence, such as a rat DNA sequence, capable of regulation of the mouse or human DNA.

In another aspect the invention relates to a cell, preferably non-human cell, or non-human mammal comprising an immunoglobulin heavy chain or light chain locus having DNA from 2 or more different human genomes. For example, it could comprise heavy chain V(D)J sequences from more than one human genome within a heavy or light chain, or heavy chain VDJ DNA from one genome and light chain VJ sequences from a different genome.

In one aspect the invention relates to a DNA fragment or cell or non-human mammal comprising an immunoglobulin heavy chain or light chain locus, or part thereof, having DNA from 2 or more species, where one species contributes a non-coding region such as a regulatory region, and the other species coding regions such as V, D, J or constant regions.

In one aspect the human promoter and/or other control elements that are associated with the different human V, D or J regions are maintained after insertion of the human VDJ into the mouse genome.

In a further aspect one or more of the promoter elements, or other control elements, of the human regions, such as the human V regions, are optimised to interact with the transcriptional machinery of a non-human mammal.

Suitably a human coding sequence may be placed under the control of an appropriate non-human mammal promoter, which allows the human DNA to be transcribed efficiently in the appropriate non-human animal cell. In one aspect the human region is a human V region coding sequence, and a human V region is placed under the control of a non-human mammal promoter.

The functional replacement of human promoter or other control regions by non-human mammal promoter or control regions may be carried out by use of recombineering, or other recombinant DNA technologies, to insert a part of the human Ig region (such as a human V region) into a vector (such as a BAC) containing a non-human Ig region. The recombineering/recombinant technique suitably replaces a portion of the non-human (e.g. mouse) DNA with the human Ig region, and thus places the human Ig region under control of the non-human mammal promoter or other control region. Suitably the human coding region for a human V region replaces a mouse V region coding sequence. Suitably the human coding region for a human D region replaces a mouse D region coding sequence. Suitably the human coding region for a human J region replaces a mouse J region coding sequence. In this way human V, D or J regions may be placed under the control of a non-human mammal promoter, such as a mouse promoter.

In one aspect the only human DNA inserted into the non-human mammalian cell or animal are V, D or J coding regions, and these are placed under control of the host regulatory sequences or other (non-human, non-host) sequences, In one aspect reference to human coding regions includes both human introns and exons, or in another aspect simply exons and no introns, which may be in the form of cDNA.

It is also possible to use recombineering, or other recombinant DNA technologies, to insert a non-human-mammal (e.g. mouse) promoter or other control region, such as a promoter for a V region, into a BAC containing a human Ig region. A recombineering step then places a portion of human DNA under control of the mouse promoter or other control region.

The approaches described herein may also be used to insert some or all of the V, D and J regions from the human heavy chain upstream of a light chain constant region, rather than upstream of the heavy chain constant region. Likewise some or all of the human light chain V and J regions may be inserted upstream of the heavy chain constant region. Insertion may be at the endogenous constant region locus, for example between the endogenous constant and J region, and may be of some, or all, of the V, D or J genes alone, excluding promoter or enhancer sequences, or may be of some, or all, of the V, D or J genes with one or more or all respective promoter or enhancer sequences. In one aspect the full repertoire of V, D or J fragments in germline orientation may be inserted upstream and in functional arrangement with a host constant region.

Thus the present invention allows V and/or D and/or J regions from a human, or any species, to be inserted into a chromosome of a cell from a different species that comprises a constant region, allowing a chimaeric antibody chain to be expressed.

In one aspect the invention requires only that some human variable region DNA is inserted into the genome of a non-human mammal in operable arrangement with some, or all, of the human heavy chain constant region at the region of the endogenous heavy chain constant region locus such that an antibody chain can be produced. In this aspect of the invention and where human light chain DNA is additionally inserted, the light chain DNA insertion can be in the form of a completely human construct, having both human variable DNA and human constant region DNA, or have human variable region DNA and constant region DNA from a non-human, non-host species. Other variations are also possible, such as insertion of both of the light chain human variable region and host genome constant region. In addition the insertion of said light chain transgenes need not be at the equivalent endogenous locus, but may be anywhere in the genome. In such a scenario the cell or mammal may produce chimaeric heavy chains (comprising human variable region DNA and mouse constant region DNA) and light chains comprising human variable and human constant region DNA. Thus in one aspect of the invention the lambda and or kappa human variable region DNA can be inserted upstream of the endogenous locus, or downstream, or indeed on a different chromosome to the endogenous locus, and inserted with or without constant region DNA.

As well insertion of human light chain DNA upstream of the host non-human mammal constant region, a further aspect of the invention relates to insertion of one or both light chain human variable regions downstream of the equivalent endogenous locus constant region, or elsewhere in the genome.

Generally, insertion of human variable region DNA at or close to the equivalent endogenous locus in the recipient genome is preferred, for example within 1, 2, 3, 4, 5, 6, 7, 8, 9, or 10kb of the boundary (upstream or downstream) of a host immunoglobulin locus.

Thus in one aspect the invention can relate to a cell or non-human mammal whose genome comprises:
(a) a plurality of human IgH V regions, one or more human D regions and one or more human J regions upstream of the host non-human mammal constant region; and
(b) one or more human Ig light chain kappa V regions and one or more human Ig light chain kappa J regions, and/or, one or more human Ig light chain lambda V regions and one or more human Ig light chain lambda J regions;
wherein the non-human mammal is able to produce a repertoire of chimaeric antibodies, or chimaeric light or heavy chains, having a non-human mammal constant region and a human variable region.

In one particular aspect the genome of the cell or non-human mammal comprises:
a plurality of human IgH V regions, one or more human D regions and one or more human J regions upstream of the host non-human mammal constant region;
one or more human Ig light chain kappa V regions and one or more human Ig light chain kappa J regions upstream of the host non-human mammal kappa constant region, and
one or more human Ig light chain lambda V regions and one or more human Ig light chain lambda J regions downstream of the host non-human mammal lambda constant region,
optionally in which the human lambda variable region may be inserted upstream or downstream of the endogenous host lambda locus in operable linkage with a human lambda constant region, such that the non-human mammal or cell can produce fully human antibody light chains and chimaeric heavy chains.

In a further, different, aspect of the invention, the use of the methods of the invention allows a locus to be built up in a stepwise manner by sequential insertions, and thus allows for the insertion of human variable DNA together with human or non-human constant region DNA at any suitable location in the genome of a non-human host cell. For example, methods of the invention can be used to insert human immunoglobulin variable region DNA together with constant region DNA from the host genome anywhere in the genome of a non-human host cell, allowing a chimaeric antibody chain to be produced from a site other than the endogenous heavy region. Any human heavy chain or light chain DNA construct contemplated above can be inserted into any desired position into the genome of a non-human host cell using the techniques described herein. The present invention thus also relates to cells and mammals having genomes comprising such insertions.

The invention also relates to a vector, such as a BAC, comprising a human V, D or J region in a functional arrangement with a non-human mammal promoter, or other control sequence, such that the expression of the human V, D or J region is under the control of the non-human mammal promoter in a cell of the non-human mammal, such as an ES cell, in particular once inserted into the genome of that cell.

The invention also relates to cells and non-human mammals containing said cells, which cells or mammals have a human V, D or J region in a functional arrangement with a non-human mammal promoter, or other control sequence, such that the expression of the human V, D or J region is under the control of the non-human mammal promoter in the cells or mammal.

Generally, one aspect of the invention thus relates to a non-human mammal host cell capable of expression of a human V, D or J coding sequence under the control of a host promoter or control region, the expression capable of producing a humanised antibody having a human variable domain and non-human mammal constant region.

In one aspect the invention relates to a cell, such as a non mammalian cell, such as an ES cell, the genome of which comprises
a) a plurality of human IgH V regions, one or more human D regions and one or more human J regions upstream of the host non-human mammal constant region; and
(b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region;

In another aspect the invention relates to a cell, such as a non-human mammal cells, such as ES cells whose genome comprises
(a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
(b) optionally one or more human IgH V regions, one or more human D regions and one or more human J regions upstream of the host non-human mammal constant region

In one aspect the cell is an ES cell is capable of developing into a non-human mammal able to produce a repertoire of antibodies which are chimaeric, said chimaeric antibodies having a non-human mammal constant region and a human variable region. Optionally the genome of the cell is modified to prevent expression of fully host-species specific antibodies.

In one aspect the cell is an induced pluripotent stem cell (iPS cell).

In one aspect cells are isolated non-human mammalian cells.

In one aspect a cell as disclosed herein is preferably a non-human mammalian cell.

In one aspect the cell is a cell from a mouse strain selected from C57BL/6, M129 such as 129/SV, BALB/c, and any hybrid of C57BL/6, M129 such as 129/SV, or BALB/c.

The invention also relates to a cell line which is grown from or otherwise derived from cells as described herein, including an immortalised cell line. The cell line may comprise inserted human V, D or J genes as described herein, either in germline configuration or after rearrangement following in vivo maturation. The cell may be immortalised by fusion to a tumour cell to provide an antibody producing cell and cell line, or be made by direct cellular immortalisation.

The present invention also relates to vectors for use in the invention. In one aspect such vectors are BACs (bacterial artificial chromosomes). It will be appreciated that other cloning vectors may be used in the invention, and therefore reference to BACs herein may be taken to refer generally to any suitable vector.

In one aspect BACs used for generation of human DNA to be inserted, such as the VDJ or VJ regions are trimmed so that, in the final human VDJ or VJ region or part thereof in the non-human mammal, no sequence is duplicated or lost when compared to the original human genomic sequence.

In one aspect the invention relates to a vector comprising an insert, preferably comprising a region of human DNA from some of the human VDJ or VJ locus, flanked by DNA which is not from that locus. The flanking DNA may comprise one or more selectable markers or one or more site specific recombination sites. In one aspect the vector comprises 2 or more, such as 3, heterospecific and incompatible site specific recombination sites. In one aspect the site specific recombination sites may be loxP sites, or variants thereof, or FRT sites or variants thereof. In one aspect the vector comprises one or more transposon ITR (inverted terminal repeat) sequences.

In one aspect the non-human animals of the invention suitably do not produce any fully humanised antibodies. In one aspect this is because there is no DNA inserted from the human constant region. Alternatively there is no human constant region DNA in the genome capable of forming an antibody in conjunction with the inserted human variable region DNA component, for example due to mutation within any human constant region DNA or distance from any constant region human DNA and human variable region DNA.

In one aspect human light chain constant region DNA may be included in the cell genome, such that a fully human lambda or kappa human antibody chain might be generated, but this would only be able to form an antibody with a chimaeric heavy chain, and not produce a fully human antibody having human variable and constant regions.

In one aspect the non-human mammal genome is modified to prevent expression of fully host-species specific antibodies. Fully host species specific antibodies are antibodies that have both variable and constant regions from the host organism. In this context the term 'specific' is not intended to relate to the binding of the antibodies produced by the cells or animals of the invention but rather to the origin of the DNA which encodes those antibodies.

In one aspect the non-human mammal genome is modified to prevent expression of the native (fully host species specific) antibodies in the mammal by inactivation of all or a part of the host non-human mammal Ig loci. In one aspect this is achieved by inversion of all or part of the non-human mammal VDJ region, or VJ region, optionally by insertion of one or more site specific recombinase sites into the genome and then use of these sites in recombinase-mediated excision or inversion of all or a part of the non-human mammal Ig locus. In one aspect a double inversion, may be employed, the first to move the V(D)Js away from the endogenous locus and then a more local inversion which puts them in the correct orientation. In one aspect a single /*ox*P site is used to invert the non-human mammal VDJ region to a centromeric locus or telomeric locus.

In one aspect the non-human mammal genome into which human DNA is inserted comprises endogenous V, (D) and J regions, and the endogenous sequences have not been deleted.

The invention comprises a method for insertion of multiple DNA fragments into a DNA target, suitably to form a contiguous insertion in which the inserted fragments are joined together directly without intervening sequences. The method is especially applicable to the insertion of a large DNA fragment into a host chromosome which can be carried out in a stepwise fashion.

In one aspect the method comprises insertion of a first DNA sequence into a target, the sequence having a DNA vector portion and a first sequence of interest (X1); insertion of a second DNA sequence into the vector portion of the first sequence, the second DNA sequence having a second sequence of interest (X2) and a second vector portion; and then excising any vector sequence DNA separating X1 and X2 to provide a contiguous X1X2, or X2X1 sequence within the target. There is optionally insertion of a further one or more DNA sequences, each DNA sequence having a further sequence of interest (X3,...) and a further vector portion, into the vector portion of the preceding DNA sequence, to build up a contiguous DNA fragment in the target.

The DNA target for insertion of the first DNA sequence may be a specific site or any point in the genome of a particular cell.

The general method is described herein in relation to the insertion of elements of the human VDJ region, but is applicable to insertion of any DNA region, from any organism, and in particular insertion of large DNA fragments of > 100kB, such as 100 - 250kb, or even larger, such as that of the TCR or HLA. Features and approaches described herein in respect of the VDJ insertion may be equally applied to the any of the methods disclosed

In one aspect the inserted DNA is human DNA, such as the human VDJ or VJ region, is built up in the genome of a cell, such as an ES cell, in a stepwise manner using 2, 3, 4, 5, 6, 7, 8, 9, 10, 11, 12, 13, 14, 15, 20 or more separate insertions for each heavy chain or light chain region. Fragments are suitably inserted at the same or substantially the same cell locus, e.g. ES cell locus, one after another, to form the complete VDJ or VJ region, or part thereof. The present invention also relates to cells and non-human animals comprising intermediates in the process whose genomes may comprise only a partial VDJ region, such as only human variable region DNA.

In a further aspect the method for producing a transgenic non-human mammal comprises the insertion of human VDJ or VJ regions upstream of the host non-human mammal constant region by step-wise insertion of multiple fragments by homologous recombination, preferably using an iterative process. Suitably fragments of approximately 100KB from the human VDJ and VJ locus are inserted, suitably to form part of, or a complete, VDJ or VJ region after the final iteration of the insertion process, as disclosed herein.

In one aspect the insertion process commences at a site where an initiation cassette has been inserted into the genome of a cell, such as an ES cell, providing a unique targeting region. In one aspect the initiation cassette is inserted in the non-human mammal heavy chain locus, for use in insertion of human heavy chain DNA. Similarly an initiation cassette may be inserted in the non-human mammal light chain locus, for use in insertion of human light chain VJ DNA The initiation cassette suitably comprises a vector backbone sequence with which a vector having a human DNA fragment in the same backbone sequence can recombine to insert the human DNA into the cell (e.g. ES) cell genome, and suitably a selection marker, such as a negative selection marker. Suitably the vector backbone sequence is that of a BAC library, to allow BACs to be used in the construction of the ES cells and mammals. The vector backbone sequence may however be any sequence which serves as a target site into which a homologous sequence can insert, for example by homologous recombination, for example RMCE, and is preferably not DNA encoding any of the VDJ or constant region.

In one aspect the insertion of the first DNA fragment into an initiation cassette is followed by insertion of a second DNA fragment into a portion of the first DNA fragment, suitably a part of the vector backbone of the second DNA fragment. In one aspect an inserted DNA fragment comprises a part of the human VDJ region flanked by 5' and/or 3' sequences that are not from the human VDJ region. In one aspect the 5' and/or 3' flanking sequences may each contain one or more selectable markers, or be capable of creating a selectable system once inserted into the genome. In one aspect one or both flanking sequences may be removed from the genome in vitro, or in vivo, following insertion. In one aspect the method comprises insertion of a DNA fragment followed by selection of both 5' and 3' ends of the inserted fragment flanking the human VDJ DNA. In one aspect the iterative insertion is made by insertion of DNA fragments at the 5' end of the previous inserted fragment, and in this aspect there may be deletion in vivo of the vector DNA which separates the inserted human DNA sequences, to provide a contiguous human DNA sequence.

In one aspect insertion of human VDJ DNA into a genome may be achieved without leaving any flanking DNA in the genome, for example by transposase mediate DNA excision. One suitable transposase is the Piggybac transposase.

In one aspect the first human variable region fragment is inserted by homologous recombination at the initiation cassette backbone sequence and then the DNA of any negative selection marker and initiation cassette are subsequently removed by recombination between recombinase target sequences, such as FRT using in this example, FLPase expression. Generally repeated targeted insertions at the (e.g. BAC) backbone initiation sequence and subsequent removal by rearrangement between recombinase target sequences are repeated to build up the entire human VDJ region upstream of the host non-mammal constant region.

In one aspect a selectable marker or system may be used in the method. The marker may be generated upon insertion of a DNA fragment into a genome, for example forming a selectable marker in conjunction with a DNA element already present in the genome.

In one aspect the cell (e.g. ES) cell genome does not contain 2 identical selectable markers at the same time during the process. It can be seen that the iterative process of insertion and selection can be carried out using only 2 different selection markers, as disclosed in the examples herein, and for example the third selectable marker may be identical to the first marker, as by the time of insertion of the third vector fragment the first vector fragment and the first marker has been removed.

In one aspect a correct insertion event, is confirmed before moving to the next step of any multistep cloning process, for example by confirmation of BAC structure using high density genomic arrays to screen ES cells to identify those with intact BAC insertions, sequencing and PCR verification.

### Initiation cassette (also called a 'landing pad')

The invention also relates to a polynucleotide 'landing pad' sequence, the polynucleotide comprising nucleic acid regions homologous to regions of a target chromosome to allow for insertion by homologous recombination into the target chromosome, and comprising a nucleic acid site which permits recombinase-driven insertion of nucleic acid into the landing pad. The invention also relates to vectors, cells and mammals of the invention comprising a landing pad as disclosed herein inserted into the genome of the cell.

The landing pad optionally comprises a non-endogenous S-mu, e.g. a rat S-mu switch

The landing pad optionally comprises (in 5' to 3' orientation) a mouse Eµ sequence, a non-human, non-mouse (e.g. rat) Switch µ and at least a portion of a mouse Cµ or the entire mouse Cµ.

The rat switch sequence optionally comprises or consists of SEQ ID NO 1.

The landing pad optionally comprises the 5' homology arm of SEQ ID NO 6.

The landing pad optionally has the sequence of SEQ ID 2 or SEQ ID NO 3.

The methods of the invention include methods wherein the landing pad sequence comprises any of the configurations or sequences as disclosed herein.

Another method of the invention comprises the step of insertion of the landing pad into a mouse chromosome by homologous recombination between mouse J1-4 and mouse C mu sequences.

Another method of the invention comprises the step of insertion of the landing pad into the mouse chromosome 12 by homologous recombination between mouse J1-4 and E mu.

In one aspect the method uses site specific recombination for insertion of one or more vectors into the genome of a cell, such as an ES cell. Site specific recombinase systems are well known in the art and may include Cre-lox, and FLP/FRT or combinations thereof, in which recombination occurs between 2 sites having sequence homology.

Additionally or alternatively to any particular Cre/Lox or FLP/FRT system described herein, other recombinases and sites that may be used in the present invention include Dre recombinase, rox sites, and PhiC31 recombinase.

Suitable BACs are available form the Sanger centre, see "A genome-wide, end-sequenced 129Sv BAC library resource for targeting vector construction". Adams DJ, Quail MA, Cox T, van der Weyden L, Gorick BD, Su Q, Chan WI, Davies R, Bonfield JK, Law F, Humphray S, Plumb B, Liu P, Rogers J, Bradley A. Genomics. 2005 Dec;86(6):753-8. Epub 2005 Oct 27. The Wellcome Trust Sanger Institute, Hinxton, Cambridgeshire CB10 1SA, UK. BACs containing human DNA are also available from, for example, Invitrogen. A suitable library is described in Osoegawa K et al, Genome Research 2001. 11: 483-496.

In one aspect a method of the invention specifically comprises:
(1) insertion of a first DNA fragment into a non-human ES cell, the fragment containing a first portion of human VDJ or VJ region DNA and a first vector portion containing a first selectable marker;
(2) optionally deletion of the a part of the first vector portion;
(3) insertion of a second DNA fragment into a non-human ES cell containing the first DNA fragment, the insertion occurring within the first vector portion, the second DNA fragment containing a second portion of the human VDJ or VJ region and a second vector portion containing a second selectable marker,
(4) deletion of the first selectable marker and first vector portion, preferably by a recombinase enzyme action;
(5) insertion of a third DNA fragment into a non-human ES cell containing the second DNA fragment, the insertion occurring within the second vector portion, the third DNA fragment containing a third portion of the human VDJ or VJ region and a third vector portion containing third selectable marker,
(6) deletion of the second selectable marker and second vector portion; and
(7) iteration of the steps of insertion and deletion, as necessary, for fourth and further fragments of the human VDJ or VJ human regions, as necessary, to produce an ES cell with a part or all of the human VDJ or VJ region inserted as disclosed herein, and suitably to remove all the vector portions within the ES cell genome.

In another aspect the invention comprises
1 insertion of DNA forming an initiation cassette into the genome of a cell;
2 insertion of a first DNA fragment into the initiation cassette, the first DNA fragment comprising a first portion of a human DNA and a first vector portion containing a first selectable marker or generating a selectable marker upon insertion;
3 optionally removal of part of the vector DNA
4 insertion of a second DNA fragment into the vector portion of the first DNA fragment, the second DNA fragment containing a second portion of human DNA and a second vector portion, the second vector portion containing a second selectable marker, or generating a second selectable marker upon insertion;
5 optionally, removal of any vector DNA to allow the first and second human DNA fragments to form a contiguous sequence; and
6 iteration of the steps of insertion of human VDJ DNA and vector DNA removal, as necessary, to produce a cell with all or part of the human VDJ or VJ region sufficient to be capable of generating a chimaeric antibody in conjunction with a host constant region,
wherein the insertion of one, or more, or all of the DNA fragments uses site specific recombination.

In one aspect the non-human mammal is able to generate a diversity of at least 1 X 10⁶ different functional chimaeric immunoglobulin sequence combinations.

In one aspect the targeting is carried out in ES cells derived from the mouse C57BL/6N, C57BL/6J, 129S5 or 129Sv strain.

In one aspect non-human animals, such as mice, are generated in a RAG-1 or RAG-2 deficient background, or other suitable genetic background which prevents the production of mature host B and T lymphocytes.

In one aspect the non-human mammal is a rodent, suitably a mouse, and cells of the invention, are rodent cells or ES cells, suitably mouse ES cells.

The ES cells of the present invention can be used to generate animals using techniques well known in the art, which comprise injection of the ES cell into a blastocyst followed by implantation of chimaeric blastocystys into females to produce offspring which can be bred and selected for homozygous recombinants having the required insertion. In one aspect the invention relates to a chimeric animal comprised of ES cell-derived tissue and host embryo derived tissue. In one aspect the invention relates to genetically-altered subsequent generation animals, which include animals having a homozygous recombinants for the VDJ and/or VJ regions.

In a further aspect the invention relates to a method for producing an antibody specific to a desired antigen the method comprising immunizing a transgenic non-human mammal as above with the desired antigen and recovering the antibody (see e.g. Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259). Suitably an immunogenic amount of the antigen is delivered. The invention also relates to a method for detecting a target antigen comprising detecting an antibody produced as above with a secondary detection agent which recognises a portion of that antibody.

In a further aspect the invention relates to a method for producing a fully humanised antibody comprising immunizing a transgenic non-human mammal as above with the desired antigen, recovering the antibody or cells expressing the antibody, and then replacing the non-human mammal constant region with a human constant region. This can be done by standard cloning techniques at the DNA level to replace the non-human mammal constant region with an appropriate human constant region DNA sequence - see e.g. Sambrook, J and Russell, D. (2001, 3'd edition) Molecular Cloning: A Laboratory Manual (Cold Spring Harbor Lab. Press, Plainview, NY).

In a further aspect the invention relates to humanised antibodies and antibody chains produced according to the present invention, both in chimaeric and fully humanised form, and use of said antibodies in medicine. The invention also relates to a pharmaceutical composition comprising such an antibodies and a pharmaceutically acceptable carrier or other excipient.

Antibody chains containing human sequences, such as chimaeric human-non-human antibody chains, are considered humanised herein by virtue of the presence of the human protein coding regions region. Fully humanised antibodies may be produced starting from DNA encoding a chimaeric antibody chain of the invention using standard techniques.

Methods for the generation of both monoclonal and polyclonal antibodies are well known in the art, and the present invention relates to both polyclonal and monoclonal antibodies of chimaeric or fully humanised antibodies produced in response to antigen challenge in non-human mammals of the present invention.

In a yet further aspect, chimaeric antibodies or antibody chains generated in the present invention may be manipulated, suitably at the DNA level, to generate molecules with antibody-like properties or structure, such as a human variable region from a heavy or light chain absent a constant region, for example a domain antibody; or a human variable region with any constant region from either heavy or light chain from the same or different species; or a human variable region with a non-naturally occurring constant region; or human variable region together with any other fusion partner. The invention relates to all such chimaeric antibody derivatives derived from chimaeric antibodies identified according to the present invention.

In a further aspect, the invention relates to use of animals of the present invention in the analysis of the likely effects of drugs and vaccines in the context of a quasi-human antibody repertoire.

The invention also relates to a method for identification or validation of a drug or vaccine, the method comprising delivering the vaccine or drug to a mammal of the invention and monitoring one or more of: the immune response, the safety profile; the effect on disease.

The invention also relates to a kit comprising an antibody or antibody derivative as disclosed herein and either instructions for use of such antibody or a suitable laboratory reagent, such as a buffer, antibody detection reagent.

The invention also relates to a method for making an antibody, or part thereof, the method comprising providing:
(i) a nucleic acid encoding an antibody, or a part thereof, obtained according to the present invention; or
(ii) sequence information from which a nucleic acid encoding an antibody obtained according to the present invention, or part thereof, can be expressed to allow an antibody to be produced.

The present invention also relates to a chimaeric antibody comprising a human variable region and a non-human vertebrate or mammal (optionally a rat or mouse) constant region (optionally a C gamma or C mu), wherein the antibody is encoded by a nucleotide sequence corresponding to the nucleotide sequence of a chimaeric heavy chain locus of a cell (optionally a B-cell, ES cell or hybridoma), the locus comprising a non-human vertebrate constant region nucleotide sequence and a rearranged VDJ nucleotide sequence produced by the *in vivo* rearrangement of a human V region, a human D region and a human J region, the V region being selected from one of a V1-3 region, V2-5 region, V4-4 region, V1-2 region or V6-1 region, and optionally a V1-3 or V6-1 segment. Optionally, the J region is any of JH1, JH2, JH3, JH4, JH5 or JH6, and in one aspect is JH4 or JH6. The D region is, in one aspect, any D3-9, D3-10, D6-13 or D6-19. In one example , rearranged VDJ nucleotide sequence is produced by the *in vivo* rearrangement of human V1-3 and JH4 (optionally with D3-9, D3-10, D6-13 or D-19); or V1-3 and JH6 (optionally with D3-9, D3-10, D6-13 or D-19); or V6-1 and JH4 (optionally with D3-9, D3-10, D6-13 or D-19); or V6-1 and JH6 (optionally with D3-9, D3-10, D6-13 or D-19). In one example the rearranged VDJ nucleotide sequence is produced by the *in vivo* rearrangement of human V6-1 DH3-10, V1-3 DH3-10, V1-3 DH6-19, V1-3 Dh3-9 or V6-1 DH6- 19. In one aspect the antibody comprises any combination exemplified in the Examples and Figures herein. Optionally, the *in vivo* rearrangement is in a cell (eg, B cell or ES cell) derived from the same non-human vertebrate species as the constant region sequence (eg, a mouse B cell or ES cell). The invention also relates to a non-human vertebrate or mammal cell (eg, a B-cell or ES cell or hybridoma) whose genome comprises a chimaeric heavy chain locus as described above in this paragraph. The invention also relates to a non-human vertebrate or mammal (eg, a mouse or rat) whose genome comprises a chimaeric heavy chain locus as described above in this paragraph.

The present invention also relates to a non-human vertebrate or mammal having a genome encoding a chimaeric antibody, the chimaeric antibody comprising a human variable region and a non-human vertebrate or mammal (optionally a rat or mouse) constant region (optionally a C gamma or C mu), the mammal:
expressing more V1-3 antibodies than V2-5, V4-4, V1-2 or V6-1 antibodies; and/or
expressing more V1-3 JH4 or V1-3 JH6 antibodies than any of, individually, V1-3 JH1, V1-3 JH2, V1-3 JH3 or V1-3 JH5 antibodies, and/or
expressing more V6-1 JH4 or V6-1 JH6 antibodies than any of, individually, V6-1 JH1, V6-1 JH2, V6-1 JH3 or V6-1 JH5 antibodies and/or
expressing a greater number of V1-3 DH3-10 antibodies than antibodies V1-3 with any other D region. Expression of antibodies can be assessed by methods readily available to the skilled person and as conventional in the art. For example, expression can be assessed at the mRNA level as shown in the examples below.

The invention also relates to a chimaeric antibody comprising a human variable region and a non-human vertebrate or mammal (optionally a rat or mouse) constant region (optionally a light chain constant region), wherein the antibody is obtainable from a mammal (optionally a rat or mouse) whose genome comprises an antibody chain locus comprising a germline human kappa V1-8 and germline human kappa J1 sequence, and wherein the antibody is obtainable by *in vivo* recombination in said mammal of the V1-8 and J1 sequences and wherein the antibody has a variable region sequence which is different from that which is encoded by germline human kappa V1-8 and germline human kappa J1sequences. Thus, in this aspect of the invention the human germline sequences are able to undergo productive rearrangement to form a coding sequence which, in conjunction with the non-human constant region sequence, can be expressed as a chimaeric antibody chain having at least a complete human variable region and a non-human constant region. This is in contrast (as the examples show below) to the combination of the germline human kappa V1-8 and germline human kappa J1sequendces *per se*, which do not provide for an antibody coding sequence (due to the inclusion of stop codons). In one aspect the rearranged sequence of the chimaeric antibody is a result of somatic hypermutation. In one aspect the antibody is a kappa antibody; in another aspect the antibody comprises a non-human heavy chain constant region (eg, a rat or mouse C gamma or C mu). The antibody sequence optionally comprises a X1X2 T F G Q, where X1X2= PR, RT, or PW; optionally a X1X2 T F G Q G T K V E I K R A D A motif. Such motifs are not found in the equivalent position in the germline sequence as shown in the examples. The invention also relates to a non-human vertebrate or mammal cell (eg, a B-cell or ES cell or hybridoma) whose genome comprises a chimaeric antibody chain locus as described above in this paragraph. The invention also relates to a non-human vertebrate or mammal (eg, a mouse or rat) whose genome comprises a chimaeric antibody chain locus as described above in this paragraph.

The invention also relates to a chimaeric antibody comprising a human variable region and a non-human vertebrate or mammal (optionally a rat or mouse) constant region (optionally a light chain constant region), wherein the antibody is obtainable from a mammal (optionally a rat or mouse) whose genome comprises an antibody chain locus comprising a germline human kappa V1-6 and germline human kappa J1 sequence, and wherein the antibody is obtainable by *in vivo* recombination in said mammal of the V1-6 and J1 sequences and wherein the antibody has a variable region sequence which is different from that which is encoded by germline human kappa V1-6 and germline human kappa J1sequences. Thus, in this aspect of the invention the human germline sequences are able to undergo productive rearrangement to form a coding sequence which, in conjunction with the non-human constant region sequence, can be expressed as a chimaeric antibody chain having at least a complete human variable region and a non-human constant region. This is in contrast (as the examples show below) to the combination of the germline human kappa V1-6 and germline human kappa J1sequendces *per se*, which do not provide for an antibody coding sequence (due to the inclusion of stop codons). In one aspect the rearranged sequence of the chimaeric antibody is a result of somatic hypermutation. In one aspect the antibody is a kappa antibody; in another aspect the antibody comprises a non-human heavy chain constant region (eg, a rat or mouse C gamma or C mu). The antibody sequence optionally comprises a X3X4 T F G Q, where X3X4= PR or PW; optionally a X3X4 T F G Q G T K V E I K R A D A motif. Such motifs are not found in the equivalent position in the germline sequence as shown in the examples. The invention also relates to a non-human vertebrate or mammal cell (eg, a B-cell or ES cell or hybridoma) whose genome comprises a chimaeric antibody chain locus as described above in this paragraph. The invention also relates to a non-human vertebrate or mammal (eg, a mouse or rat) whose genome comprises a chimaeric antibody chain locus as described above in this paragraph.

The invention also relates to a chimaeric antibody comprising a human variable region and a non-human (optionally a rat or mouse) constant region (optionally a C gamma or C mu or a C kappa), wherein the antibody is obtainable from a mammal (optionally a rat or mouse) whose genome comprises an antibody chain locus comprising a germline human kappa V1-5 and germline human kappa J1 sequence, and wherein the antibody is obtainable by *in vivo* recombination in said mammal of the V1-5 and J1 sequences. The invention also relates to a non-human vertebrate or mammal cell (eg, a B-cell or ES cell or hybridoma) whose genome comprises a chimaeric antibody chain locus as described above in this paragraph. The invention also relates to a non-human vertebrate or mammal (eg, a mouse or rat) whose genome comprises a chimaeric antibody chain locus as described above in this paragraph.

The invention also relates to a chimaeric antibody comprising a human variable region and a non-human (optionally a rat or mouse) constant region (optionally a C gamma or C mu or a C kappa), wherein the antibody is obtainable from a mammal (optionally a rat or mouse) whose genome comprises an antibody chain locus comprising a germline human kappa V1-5 and germline human kappa J4 sequence, and wherein the antibody is obtainable by *in vivo* recombination in said mammal of the V1-5 and J4 sequences. The invention also relates to a non-human vertebrate or mammal cell (eg, a B-cell or ES cell or hybridoma) whose genome comprises a chimaeric antibody chain locus as described above in this paragraph. The invention also relates to a non-human vertebrate or mammal (eg, a mouse or rat) whose genome comprises a chimaeric antibody chain locus as described above in this paragraph.

Antibodies of the invention may be isolated, in one aspect being isolated from the cell or organism in which they are expressed.

In certain aspects the invention relates to:
A non-human mammal whose genome comprises:
(a) the human IgH VDJ region upstream of the host non-human mammal constant region; and
(b) the human Ig light chain kappa V and J regions upstream of the host non-human mammal kappa constant region and/or the human Ig light chain lambda V and J regions upstream of the host non-human mammal lambda constant region;
wherein the non-human mammal is able to produce a repertoire of chimaeric antibodies having a non-human mammal constant region and a human variable region,
and optionally wherein the non-human mammal genome is modified to prevent expression of fully host-species specific antibodies.

A non-human mammal ES cell whose genome comprises:
(a) the human IgH V, D and J region upstream of a non-human mammal constant region; and
(b) the human Ig locus light chain kappa V and J regions upstream of the host non-human mammal kappa constant region, and /or the human Ig locus light chain lambda V and J regions upstream of the host non-human mammal lambda constant region
wherein the ES cell is capable of developing into a non-human mammal, being able to produce a repertoire of antibodies which are chimaeric, having a non-human mammal constant region and a human variable region.

A method for producing a transgenic non-human mammal able to produce a repertoire of chimaeric antibodies, the antibodies having a non-human mammal constant region and a human variable region, the method comprising inserting by homologous recombination into a non-human mammal ES cell genome
(a) the human IgH VDJ region upstream of the host non-human mammal heavy chain constant region, and
(b) the human IgL VJ region for lambda or kappa chains upstream of the host non-human mammal lambda or kappa chain constant region, respectively
such that the non-human mammal is able to produce a repertoire of chimaeric antibodies having a non-human mammal constant region and a human variable region, wherein steps (a) and (b) can be carried out in either order and each of steps (a) and (b) can be carried out in a stepwise manner or as a single step.

In one aspect the insertion of human VDJ or VJ regions upstream of the host non-human mammal constant region is accomplished by step-wise insertion of multiple fragments by homologous recombination.

In one aspect the step-wise insertions commence at a site where an initiation cassette has been inserted into the genome of an ES cell providing a unique targeting region consisting of a BAC backbone sequence and a negative selection marker.

In one aspect the first human variable region fragment is inserted by homologous recombination at the initiation cassette BAC backbone sequence and said negative selection marker and initiation cassette are subsequently removed by recombination between recombinase target sequences.

In one aspect repeated targeted insertions at the BAC backbone initiation sequence and subsequent removal of the backbone by rearrangement between recombinase target sequences is repeated to build up the entire human VDJ region upstream of the host non-mammal constant region.

Other aspects include:
A method for producing an antibody specific to a desired antigen the method comprising immunizing a non-human mammal as disclosed herein with the desired antigen and recovering the antibody or a cell producing the antibody.

A method for producing a fully humanised antibody comprising immunizing a non-human mammal as disclosed herein and then replacing the non-human mammal constant region of an antibody specifically reactive with the antigen with a human constant region, suitably by engineering of the nucleic acid encoding the antibody.

A method, cell or mammal as disclosed herein wherein a human coding region DNA sequence is in a functional arrangement with a non-human mammal control sequence, such that transcription of the DNA is controlled by the non-human mammal control sequence. In one aspect the human coding region V, D or J region is in a functional arrangement with a mouse promoter sequence.

The invention also relates to a humanised antibody produced according to any methods disclosed herein and use of a humanised antibody so produced in medicine.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps

The term "or combinations thereof as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

The present invention is described in more detail in the following non limiting Examples. Example 3 discloses experimental data that has been obtained which supports the proof of concept of certain aspects of the invention, while Example 1 and 2 provide detailed guidance for the person skilled in the art in carrying out the claimed invention.

### Example 1

### Overall strategy

A mouse model of the invention can be achieved by inserting -960kb of the human heavy chain locus containing all the V, D and J-regions upstream of the mouse constant region and 473kb of the human kappa region upstream of the mouse constant region. Alternatively, or in tandem, the human lambda region is inserted upstream of the mouse constant region. This insertion is achieved by gene targeting in ES cells using techniques well known in the art.

High fidelity insertion of intact V-D-J regions into each locus in their native (wild-type) configuration is suitably achieved by insertion of human bacterial artificial chromosomes (BACs) into the locus. Suitably the BACs are trimmed so that in the final locus no sequence is duplicated or lost compared to the original. Such trimming can be carried out by recombineering.

The relevant human BACs, suitably trimmed covering these loci are on average 90kb in size.

In one approach the full complement of human D and J-elements as well as seven or eight human V-regions are covered by the first BACs to be inserted in the experimental insertion scheme described below. The first BACs to be inserted in the IgH and IgK loci may contain the following V-regions. IgH :V6-1, VII-1-1, V1-2, VIII-2-1, V1-3, V4-4, V2-5 and IgK: V4-1, V5-2, V7-3, V2-4, V1-5, V1-6, V3-7, V1-8.

Suitably the performance of each locus is assessed after the first BAC insertion using chimaeric mice and also after each subsequent BAC addition. See below for detailed description of this performance test.

Nine additional BAC insertions will be required for the *IgH* locus and five for *IgK* to provide the full complement of human V-regions covering all 0.96Mb and 0.473Mb of the *IgH* and *IgK* loci, respectively.

Not all BACs retain their wild-type configuration when inserted into the ES cell genome. Thus we deploy high density genomic arrays to screen ES cells to identify those with intact BAC insertions (Barrett, M.T., Scheffer, A., Ben-Dor, A., Sampas, N., Lipson, D., Kincaid, R., Tsang, P., Curry, B., Baird, K., Meltzer, P.S., et al. (2004). Comparative genomic hybridization using oligonucleotide microarrays and total genomic DNA. Proceedings of the National Academy of Sciences of the United States of America 101, 17765-17770.).This screen also enables one to identify and select against ES clones in which the ES cell genome is compromised and thus not able to populate the germ line of chimeric animals. Other suitable genomic tools to facilitate this assessment include sequencing and PCR verification.

Thus in one aspect the correct BAC structure is confirmed before moving to the next step.

It is implicit from the description above that in order to completely engineer the loci with 90kb BACs, it is necessary to perform a minimum of 10 targeting steps for *IgH* and 5 steps for the *IgK.* Mice with an IgL locus can be generated in a similar manner to the IgK locus. Additional steps are required to remove the selection markers required to support gene targeting. Since these manipulations are being performed in ES cells in a step-wise manner, in one aspect germ line transmission capacity is retained throughout this process.

Maintaining the performance of the ES cell clones through multiple rounds of manipulation without the need to test the germ line potential of the ES cell line at every step may be important in the present invention. The cell lines currently in use for the KOMP and EUCOMM global knockout projects have been modified twice prior to their use for this project and their germ line transmission rates are unchanged from the parental cells (these lines are publicly available, see www.komp.org and www.eucomm.org). This cell line, called JM8, can generate 100% ES cell-derived mice under published culture conditions (Pettitt, S.J., Liang, Q., Rairdan, X.Y., Moran, J.L., Prosser, H.M., Beier, D.R., Lloyd, K.C., Bradley, A., and Skarnes, W.C. (2009). Agouti C57BL/6N embryonic stem cells for mouse genetic resources. Nature Methods.). These cells have demonstrated ability to reproducibly contribute to somatic and germ line tissue of chimaeric animals using standard mouse ES cell culture conditions. This capability can be found with cells cultured on a standard feeder cell line (SNL) and even feeder-free, grown only on gelatine-coated tissue culture plates. One particular sub-line, JM8A3, maintained the ability to populate the germ line of chimeras after several serial rounds of sub-cloning. Extensive genetic manipulation via, for example, homologous recombination - as would be the case in the present invention - cannot compromise the pluripotency of the cells. The ability to generate chimeras with such high percentage of ES cell-derived tissue has other advantages. First, high levels of chimerism correlates with germ line transmission potential and provide a surrogate assay for germ line transmission while only taking 5 to 6 weeks. Second, since these mice are 100% ES cell derived the engineered loci can be directly tested, removing the delay caused by breeding. Testing the integrity of the new Ig loci is possible in the chimera since the host embryo will be derived from animals that are mutant for the RAG-1 gene as described in the next section.

Another cell line that may be used is an HPRT-ve cell line, such as AB2.1, as disclosed in "Chromosome engineering in mice, Ramirez-Solis R, Liu P and Bradley A, Nature 1995;378;6558;720-4.

### RAG-1 complementation

While many clones will generate 100% ES derived mice some will not. Thus, at every step mice are generated in a RAG-1-deficient background. This provides mice with 100% ES-derived Band T-cells which can be used directly for immunization and antibody production. Cells having a RAG-2 deficient background, or a combined RAG-1/RAG-2 deficient background may be used, or equivalent mutations in which mice produce only ES cell-derived B cells and/or T cells.

In order that only the human-mouse *IgH* or *IgK* loci are active in these mice, the human-mouse *IgH* and *IgK* loci can be engineered in a cell line in which one allele of the *IgH* or *IgK* locus has already been inactivated. Alternatively the inactivation of the host Ig locus, such as the IgH or IgK locus, can be carried out after insertion.

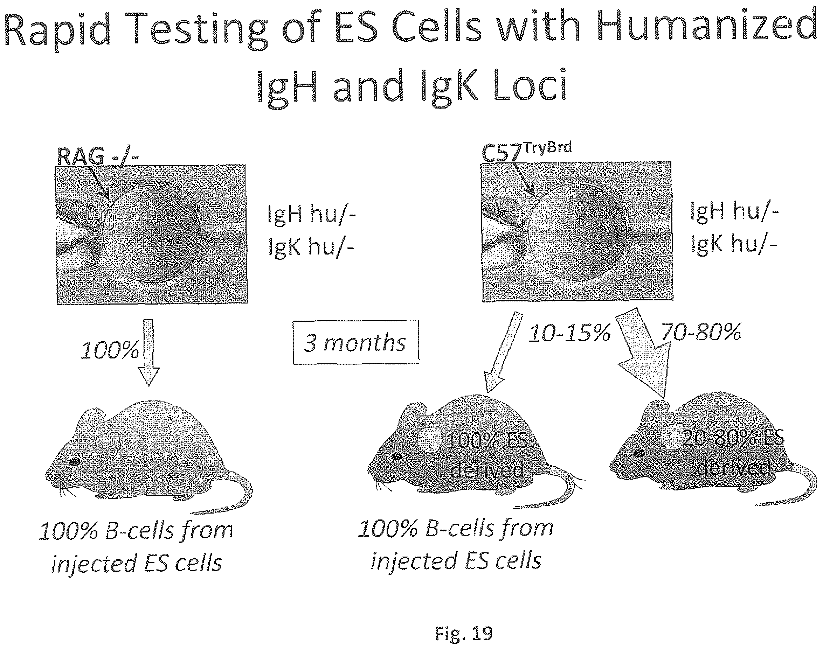

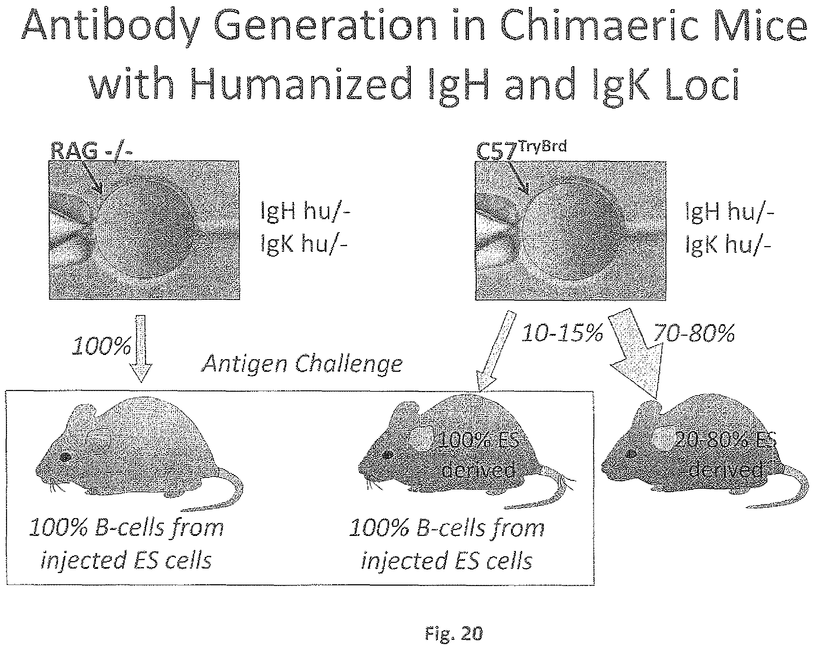

Mouse strains that have the RAG-1 gene mutated are immunodeficient as they have no mature B- or T-lymphocytes (US 5,859,307). T- and B-lymphocytes only differentiate if proper V(D)J recombination occurs. Since RAG-1 is an enzyme that is crucial for this recombination, mice lacking RAG-1 are immunodeficient. If host embryos are genetically RAG-1 homozygous mutant, a chimera produced by injecting such an embryo will not be able to produce antibodies if the animal's lymphoid tissues are derived from the host embryo. However, JM8 cells and AB2.1 cells, for example, generally contribute in excess of 80% of the somatic tissues of the chimeric animal and would therefore usually populate the lymphoid tissue. JM8 cells have wild-type RAG-1 activity and therefore antibodies produced in the chimeric animal would be encoded by the engineered JM8 ES cell genome only. Therefore, the chimeric animal can be challenged with an antigen by immunization and subsequently produce antibodies to that antigen. This allows one skilled in the art to test the performance of the engineered human/mouse IgH and IgK loci as described in the present invention. See figures 19 and 20.

One skilled in the art would use the chimeric animal as described to determine the extent of antibody diversity (see e.g. Harlow, E. & Lane, D. 1998, 5th edition, Antibodies: A Laboratory Manual, Cold Spring Harbor Lab. Press, Plainview, NY). For example, the existence in the chimeric animal's serum of certain antibody epitopes could be ascertained by binding to specific anti-idiotype antiserum, for example, in an ELISA assay. One skilled in the art could also sequence the genomes of B-cell clones derived from the chimeric animal and compare said sequence to wild-type sequence to ascertain the level of hypermutation, such hypermutation indicative of normal antibody maturation.

One skilled in the art would also use said chimeric animal to examine antibody function wherein said antibodies are encoded from the engineered Ig loci (see e.g. Harlow, E. & Lane, D. 1998, 5th edition, Antibodies: A Laboratory Manual, Cold Spring Harbor Lab. Press, Plainview, NY). For example, antisera could be tested for binding an antigen, said antigen used to immunize the chimeric animal. Such a measurement could be made by an ELISA assay. Alternatively, one skilled in the art could test for neutralization of the antigen by addition of the antisera collected from the appropriately immunized chimeric animal.

It is well known to those skilled in the art that positive outcomes for any of these tests demonstrate the ability of the engineered Ig loci, the subject of the instant invention, to encode antibodies with human variable regions and mouse constant regions, said antibodies capable of functioning in the manner of wild-type antibodies.

### Experimental Techniques

Recombineering for the production of vectors for use in homologous recombination in ES cells is disclosed in, for example, WO9929837 and WO0104288, and the techniques are well known in the art. In one aspect the recombineering of the human DNA takes place using BACs as a source of said human DNA. Human BAC DNA will be isolated using Qiagen BAC purification kit. The backbone of each human BAC will be modified using recombineering to the exact same or similar configuration as the BAC already inserted into the mouse IgH region. The genomic insert of each human BAC will be trimmed using recombineering so that once the BACs are inserted, a seamless contiguous part of the human V(D)J genomic region will form at the mouse IgH or IgK locus. BAC DNA transfection by electroporation and genotyping will be performed accordingly to standard protocols (Prosser, H.M., Rzadzinska, A.K., Steel, K.P., and Bradley, A. (2008). Mosaic complementation demonstrates a regulatory role for myosin VIIa in actin dynamics of stereocilia. Molecular and Cellular Biology 28, 1702-1712; Ramirez-Solis, R., Davis, A.C., and Bradley, A. (1993). Gene targeting in embryonic stem cells. Methods in Enzymology 225, 855-878.). Recombineering will be performed using the procedures and reagents developed by Pentao Liu and Don Court's laboratories (Chan, W., Costantino, N., Li, R., Lee, S.C., Su, Q., Melvin, D., Court, D.L., and Liu, P. (2007). A recombineering based approach for high-throughput conditional knockout targeting vector construction. Nucleic Acids Research 35, e64).

These and other techniques for gene targeting and recombination of BAC-derived chromosomal fragments into a non-human mammal genome, such as a mouse are disclosed in, for example, in http://www.eucomm.org/information/targeting/ and http://www.eucomm.org/information/publications.

Cell culture of C57BL/6N-derived cell lines, such as the JM8 male ES cells will follow standard techniques. The JM8 ES cells have been shown to be competent in extensively contributing to somatic tissues and to the germline, and are being used for large mouse mutagenesis programs at the Sanger Institute such as EUCOMM and KOMP (Pettitt, S.J., Liang, Q., Rairdan, X.Y., Moran, J.L., Prosser, H.M., Beier, D.R., Lloyd, K.C., Bradley, A., and Skarnes, W.C. (2009). Agouti C57BL/6N embryonic stem cells for mouse genetic resources. Nature Methods.). JM8 ES cells (1.0X10⁷) will be electroporated (500µF, 230V; BioRad) with 10µg I-Scel linearized human BAC DNA. The transfectants will be selected with either Puromycin (3µg/ml) or G418 (150µg/ml). The selection will begin either 24 hours (with G418) or 48 hours (with Puromycin) post electroporation and proceed for 5 days. 10µg linearized human BAC DNA can yield up to 500 Puromycin or G418 resistant ES cell colonies. The antibiotic resistant ES cell colonies will be picked into 96-well cell culture plates for genotyping to identify the targeted clones.

Once targeted mouse ES cell clones are identified, they will be analyzed by array Comparative Genomic Hybridization (CGH) for total genome integrity (Chung, Y.J., Jonkers, J., Kitson, H., Fiegler, H., Humphray, S., Scott, C., Hunt, S., Yu, Y., Nishijima, I., Velds, A., et al. (2004). A whole-genome mouse BAC microarray with 1-Mb resolution for analysis of DNA copy number changes by array comparative genomic hybridization. Genome research 14, 188-196.and Liang, Q., Conte, N., Skarnes, W.C., and Bradley, A. (2008). Extensive genomic copy number variation in embryonic stem cells. Proceedings of the National Academy of Sciences of the United States of America 105, 17453-17456.). ES cells that have abnormal genomes do not contribute to the germline of the chimeric mice efficiently. BAC integrity will be examined by PCR-amplifying each known functional V gene in the BAC. For example, in one approach the first human BAC chosen for the IgH locus has 6 functional V genes. To confirm the integrity of this BAC for the presence of these 6 IGH V genes, at least 14 pairs of PCR primers will be designed and used to PCR-amplify genomic DNA from the targeted ES cells. The human wild-type size and sequence of these fragments will ensure that the inserted BAC has not been rearranged.

More detailed CGH will also confirm the integrity of the inserted BACs. For example, one skilled in the art could use an oligo aCGH platform, which is developed by Agilent Technologies, Inc. This platform not only enables one to study genome-wide DNA copy number variation at high resolution (Barrett, M.T., Scheffer, A., Ben-Dor, A., Sampas, N., Lipson, D., Kincaid, R., Tsang, P., Curry, B., Baird, K., Meltzer, P.S., et al. (2004). Comparative genomic hybridization using oligonucleotide microarrays and total genomic DNA. Proceedings of the National Academy of Sciences of the United States of America 101, 17765-17770.), but permit examination of a specific genome region using custom designed arrays. Comparing the traditional aCGH techniques which rely on cDNA probes or whole BAC probes, the 60-mer oligonucleotides probes can ensure specific hybridization and high sensitivity and precision that is needed in order to detect the engineered chromosome alterations that we have made. For example, oligos designed to hybridize at regular intervals along the entire length of the inserted BAC would detect even quite short deletions, insertions or other rearrangements. Also, this platform provides the greatest flexibility for customized microarray designs. The targeted ES cell genomic DNA and normal human individual genomic DNA will be labelled separately with dyes and hybridized to the array. Arrays slides will be scanned using an Aglient Technologies DNA microarray scanner. Reciprocal fluorescence intensities of dye Cy5 and dye Cy3 on each array image and the log2 ratio values will be extracted by using Bluefuse software (Bluegnome). Spots with inconsistent fluorescence patterns ("confidence" < 0.29 or "quality" = 0) will be excluded before normalizing all log2 ratio values. Within an experiment, Log2 ratio between -0.29 and +0.29 for the signal from any oligo probe are regarded as no copy number change. The log2 ratio threshold for "Duplication" is usually >0.29999, and for deletion is <0.29999.

Once the first human BAC is inserted into the mouse IgH locus and confirmed to be in its intact, native configuration, the FRT-flanked BAC backbone will be excised by using Flp site-specific recombinase. If regular Flp-catalyzed FRT recombination is not high enough, one can use Flo, an improved version of Flpo recombinase which in certain tests is 3-4 times more efficient than the original Flp in ES cells. After the BAC backbone is excised, ES cells will become sensitive to Puromycin (or G418) and resistant to FIAU (for loss of the TK cassette). The excision events will be further characterized by PCR amplification of the junction fragment using human genomic DNA primers. These FRT-flanked BAC backbone-free ES cells will be used for the next round of human BAC insertion and for blastocyst injection.

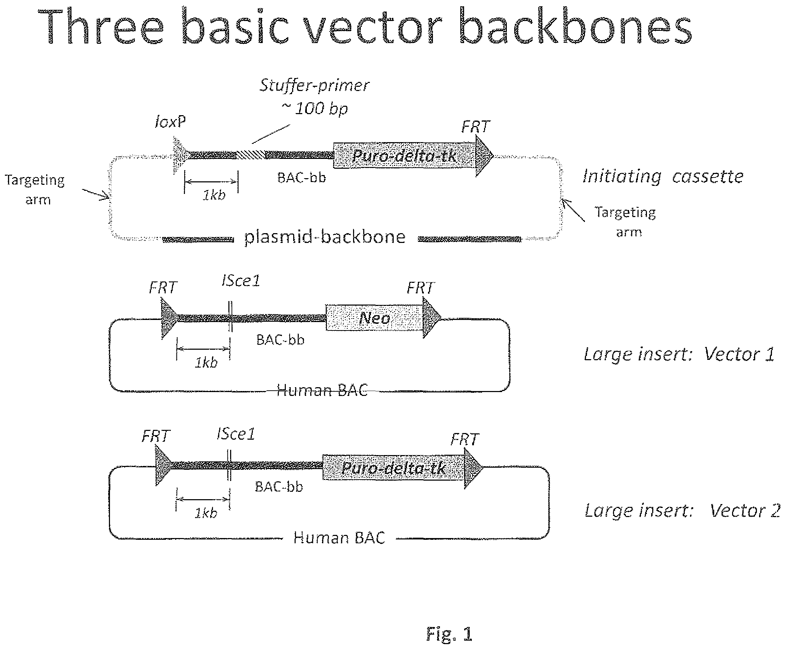

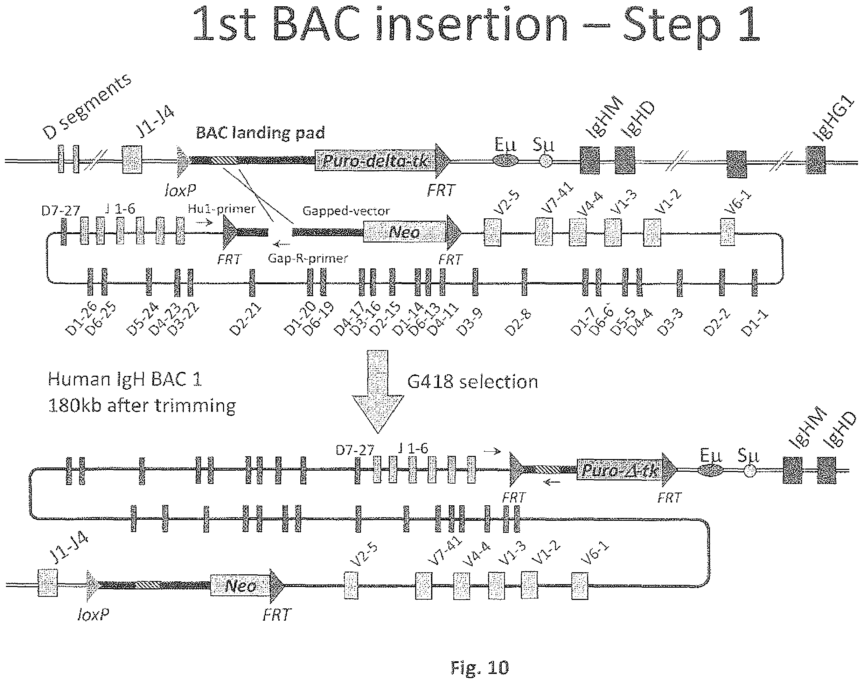

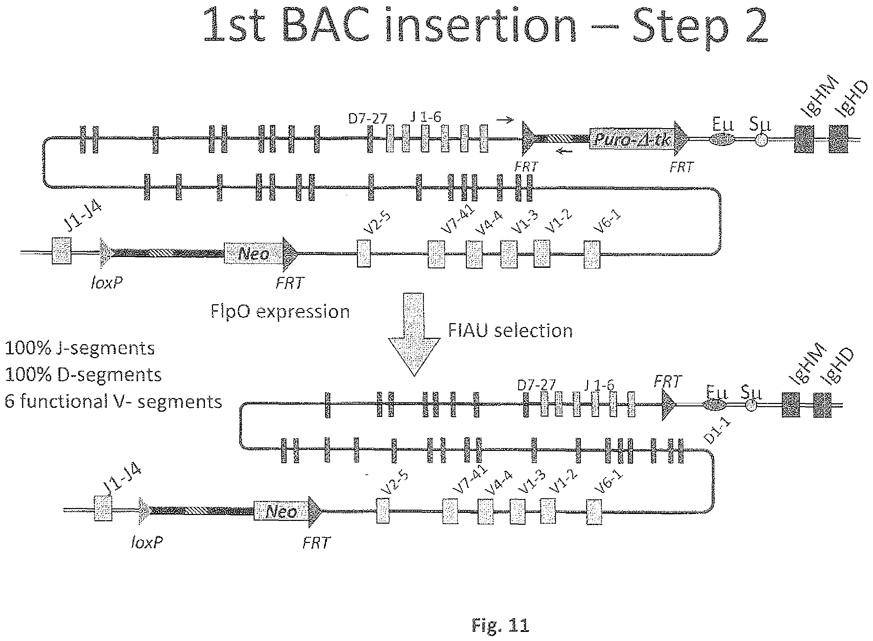

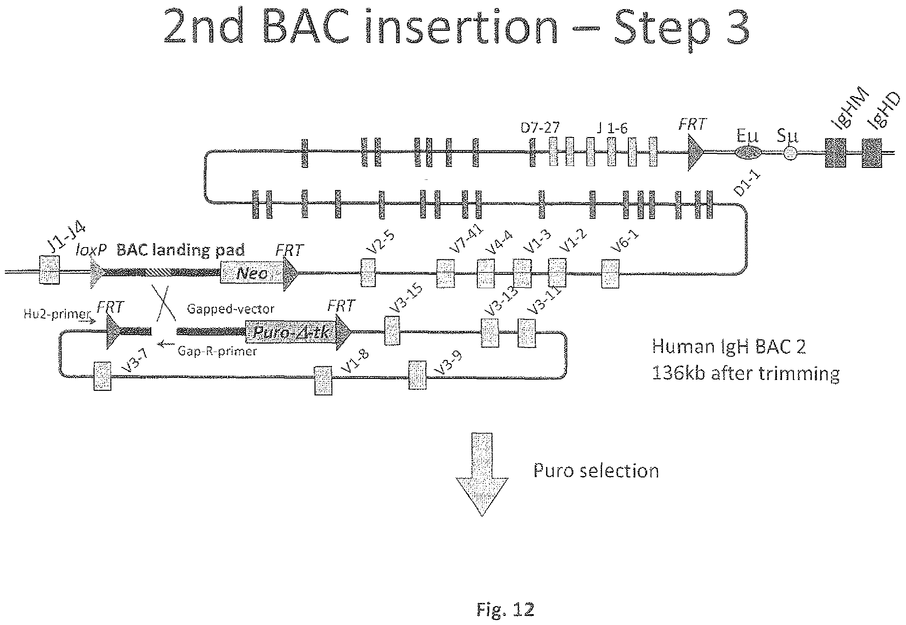

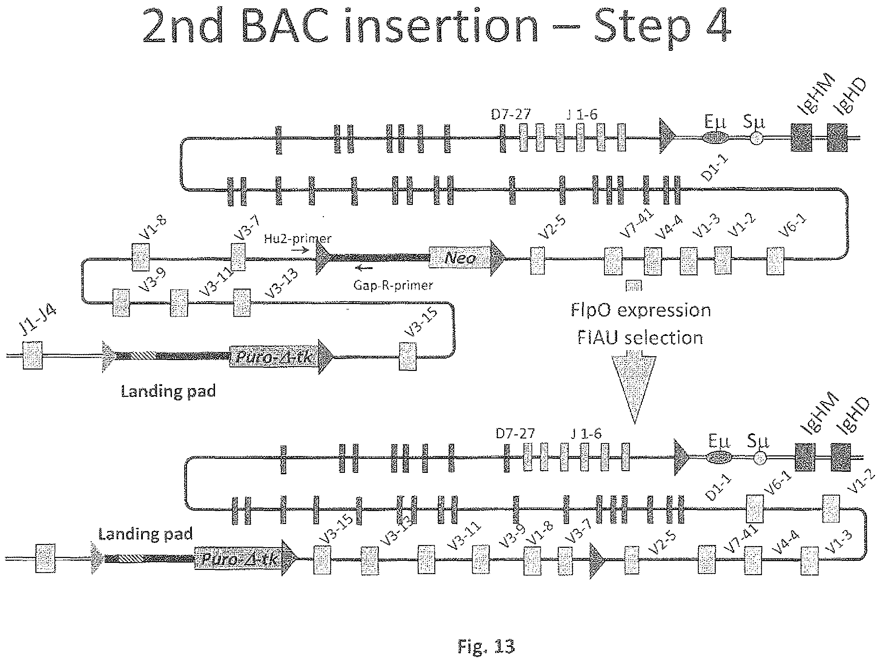

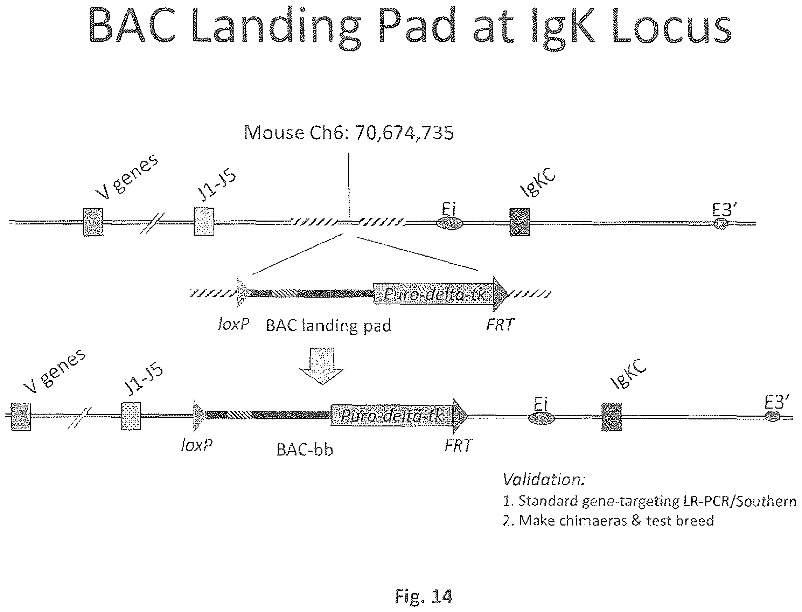

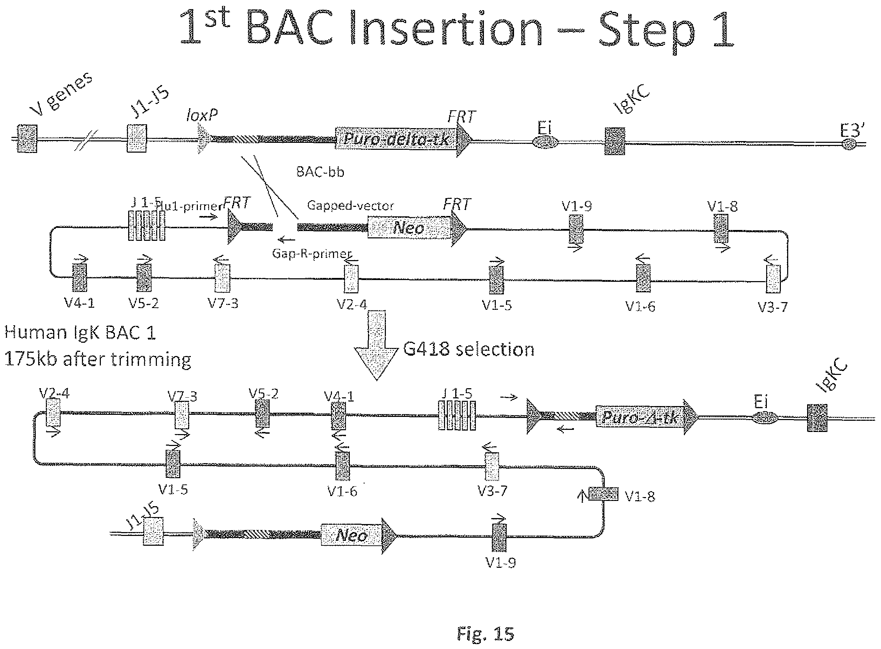

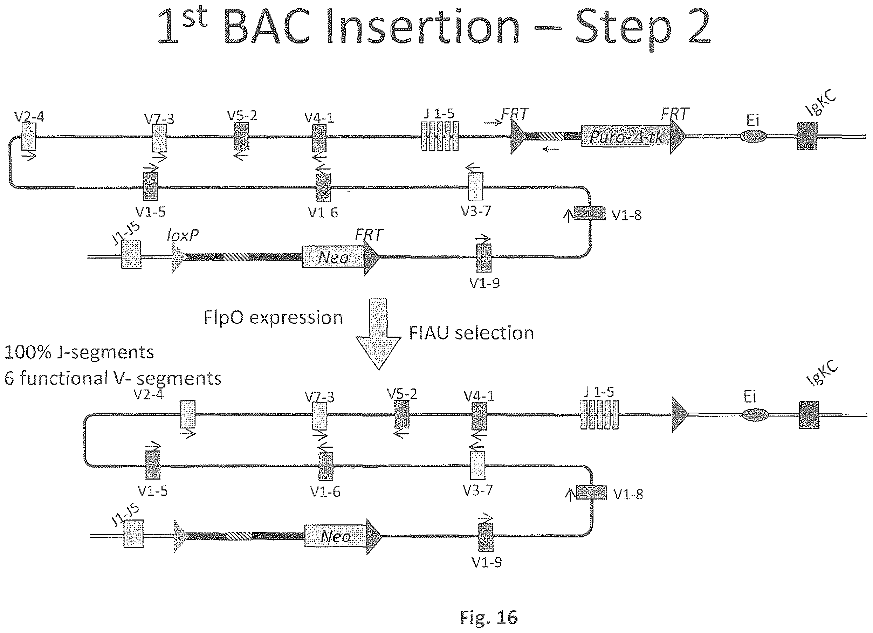

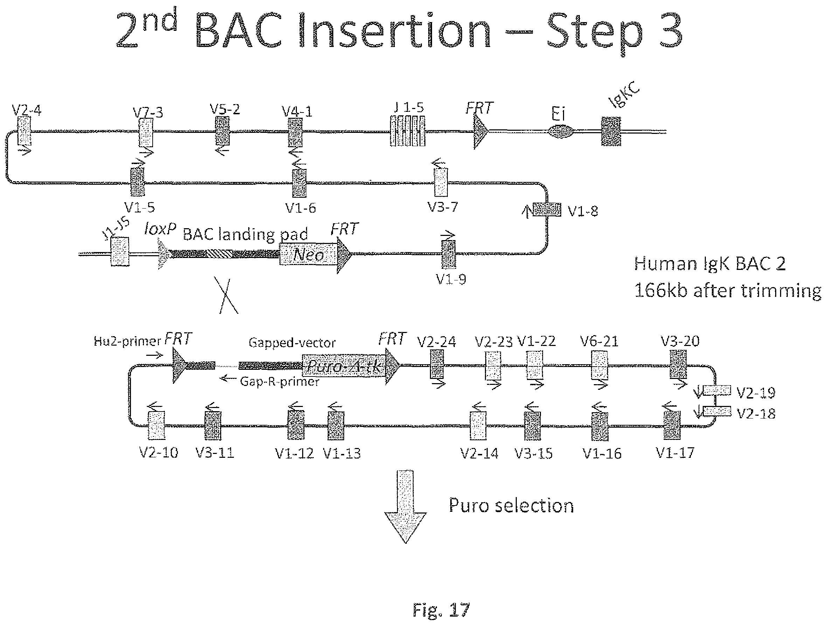

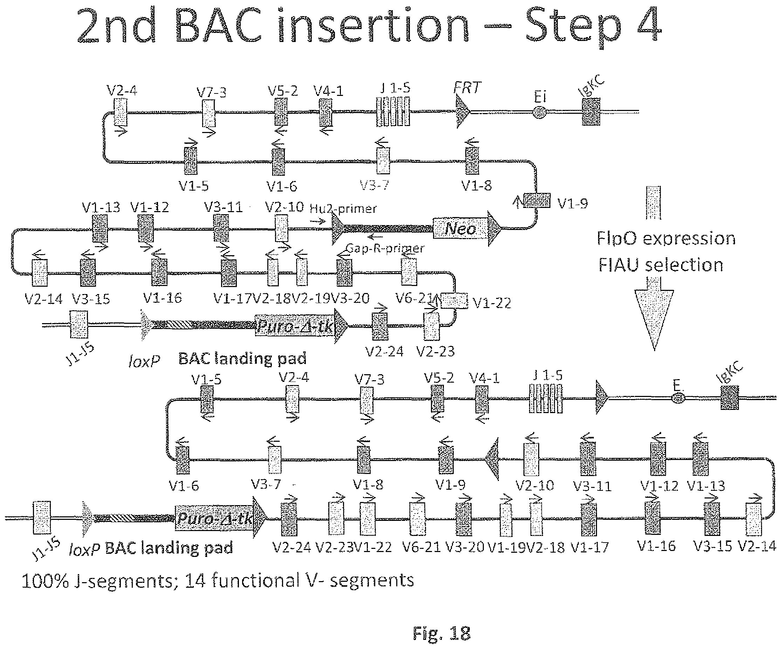

Targeting of the genome of an ES cell to produce a transgenic mouse may be carried out using a protocol as explained by reference to the attached figures 1- 18.

Figure 1 illustrates three basic backbone vectors; an initiating cassette and 2 large insert vectors 1 and 2 respectively. The initiating cassette comprises sequences homologous to the desired site of insertion into the mouse genome, those sites flanking a selectable marker and stuffer primer sequence for PCR based genotyping to confirm correct insertion of BACs. The Stuffer-primer sequence provides the basis for genotyping each BAC addition step. This sequence is considered to provide a robust well validated sequence template for PCR primer and may be located at the IScel site, ideally ∼1kb from the BAC insert.

The large insert vectors comprise human DNA on plasmids with selectable markers and a unique restriction site for linearisation of the plasmid to aid in homologous recombination into the genome of the ES cell.

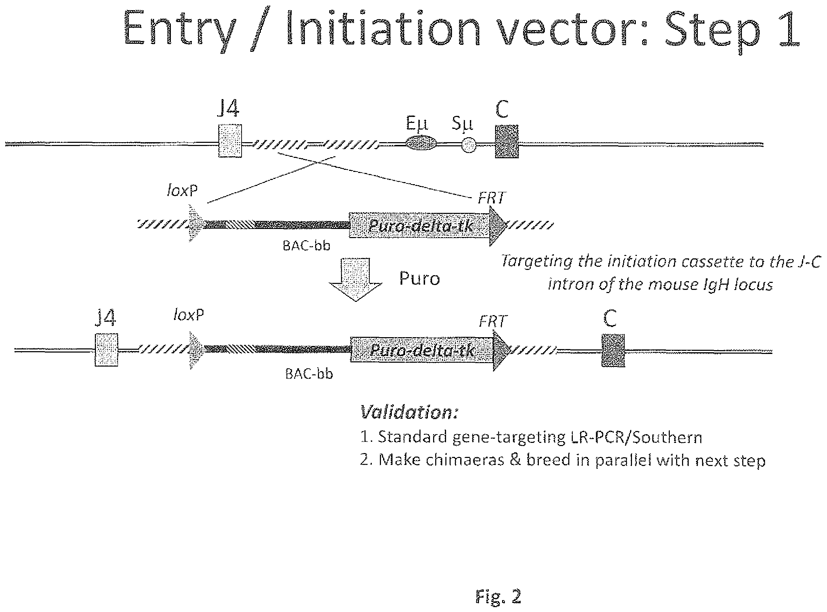

Figure 2 illustrates insertion of an initiating cassette into the mouse genome by Homologous recombination between the mouse J4 and C alpha exons. Puromycin selection allows identification of ES cells with insertion of the cassette. pu(Delta)tk is a bifunctional fusion protein between puromycin N-acetyltransferase (Puro) and a truncated version of herpes simplex virus type 1 thymidine kinase (DeltaTk). Murine embryonic stem (ES) cells transfected with pu(Delta)tk become resistant to puromycin and sensitive to 1-(-2-deoxy-2-fluoro-1-beta-D-arabino-furanosyl)-5-iodouracil (FIAU). Unlike other HSV1 tk transgenes, puDeltatk is readily transmitted through the male germ line. Thus pu(Delta)tk is a convenient positive/negative selectable marker that can be widely used in many ES cell applications.

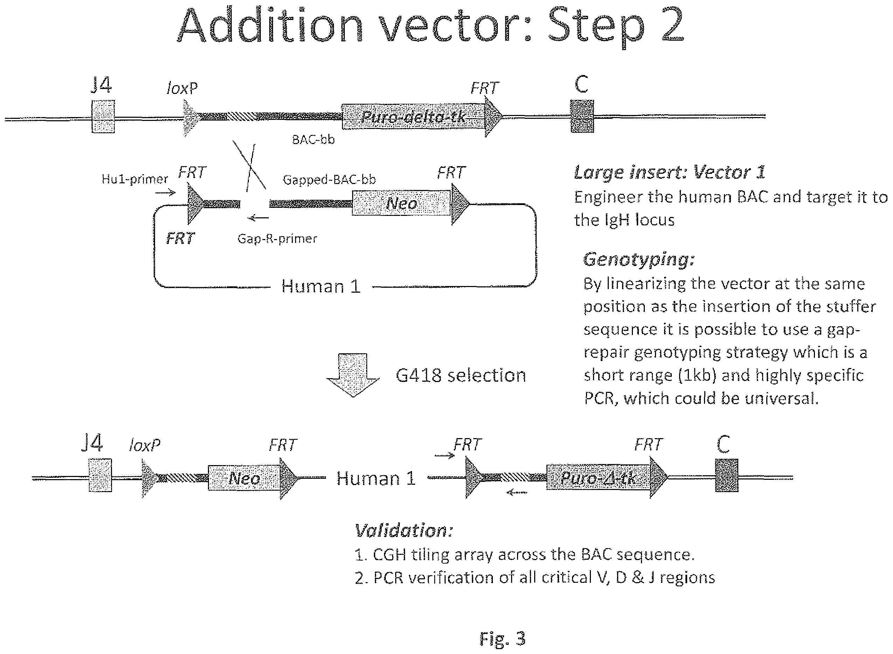

Figure 3 illustrates targeting of the large insert vector 1 to the mouse ES cell genome. Linearisation of the vector is made at the same position as the stuffer primer sequence which allows for a gap repair genotyping strategy, well known in the art - see Zheng et al NAR 1999, Vol 27, 11, 2354 - 2360. In essence, random insertion of the targeting vector into the genome will not 'repair' the gap whereas a homologous recombination event will repair the gap. Juxtaposition of appropriate PCR primer sequences allows colonies to be screened individually for a positive PCR fragment indicating proper insertion. Positive selection using G418 allows for identification of mouse ES cells containing the neo selection marker. PCR verification can be made of all critical V, D and J regions. Array comparative genomic hybridization can be used to validate the BAC structure.

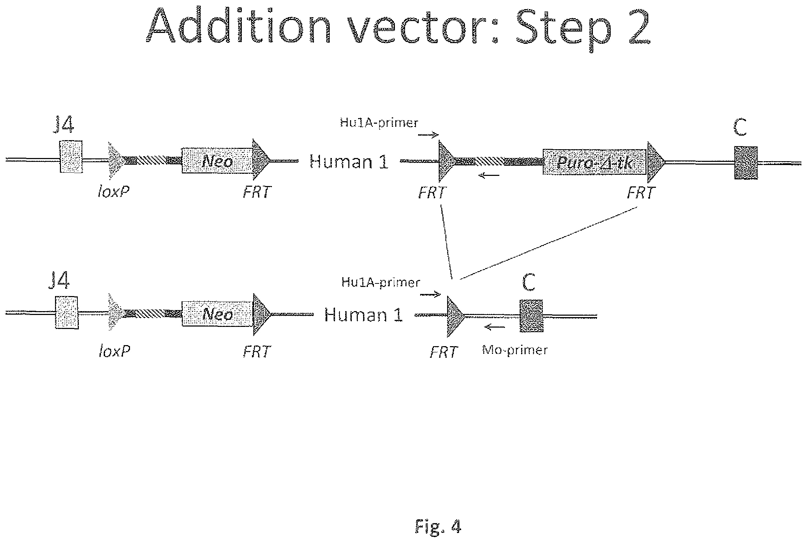

Figure 4 illustrates the *puro-delta-tk* cassette and the BAC plasmid backbone is deleted using Flpe and select in FIAU. Since Flpe works inefficiently in mouse ES cells (5% deletion with transient Flpe expression), it is expected that in most cases, the recombination occurs between the two FRT sites flanking the BAC backbone. Flpo can also be tested to find out the recombination efficiency between two FRT sites that are 10kb away.

Given that the FRT deletion step is selectable it is possible to pool FIAU resistant clones and proceed immediately to the next step in parallel with clonal analysis. Alternatively it may be desirable to show by short range PCR that the human sequences are now adjacent to those of the mouse as shown (Hu-primer 1 and Mo-primer)

At this stage a 200kb human locus will have been inserted.

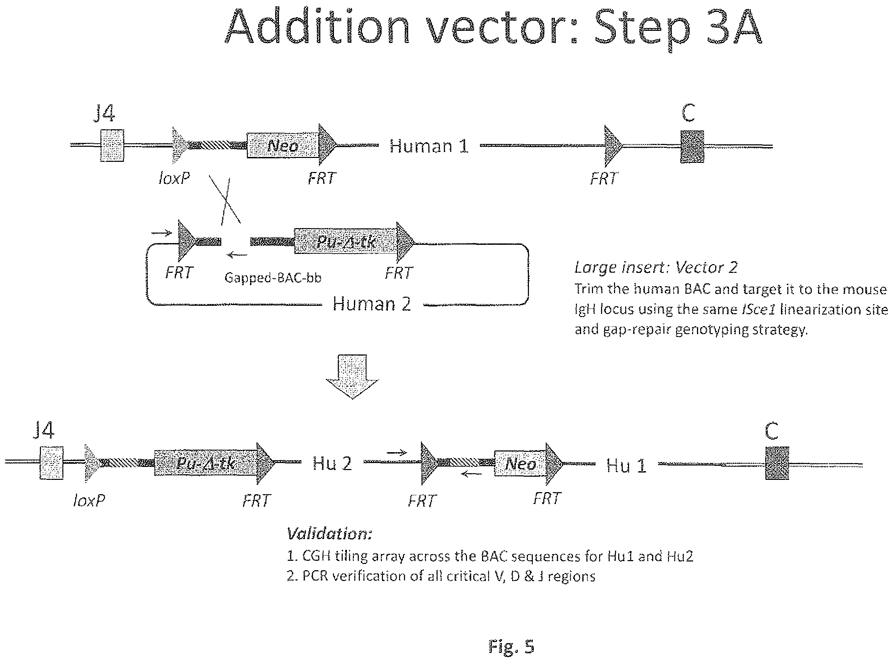

Figure 5 illustrates a second large insert vector is targeted into the ES cell chromosome. The human BAC is targeted to the mouse IgH locus using the same initiation cassette insertion followed by *ISce1* BAC linearization, BAC targeting to the initiation cassette and gap-repair genotyping strategy. Verification of the BAC insertion is carried out as before.

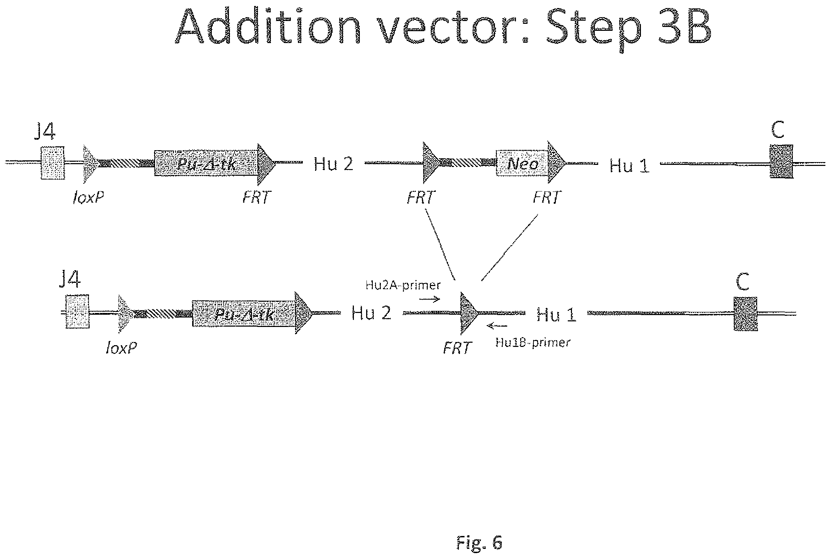

Figure 6 illustrates the FRTY flanked BAC backbone of large insert vector 2 and the neo marker are deleted via *Flpo.* Note that this is not selectable, thus it will be necessary for clonal analysis at this point. This will enable confirmation of the juxtaposition of the human 2 insert with human 1 and other validation efforts.

At this stage a ∼ 200kb human locus will have been inserted.

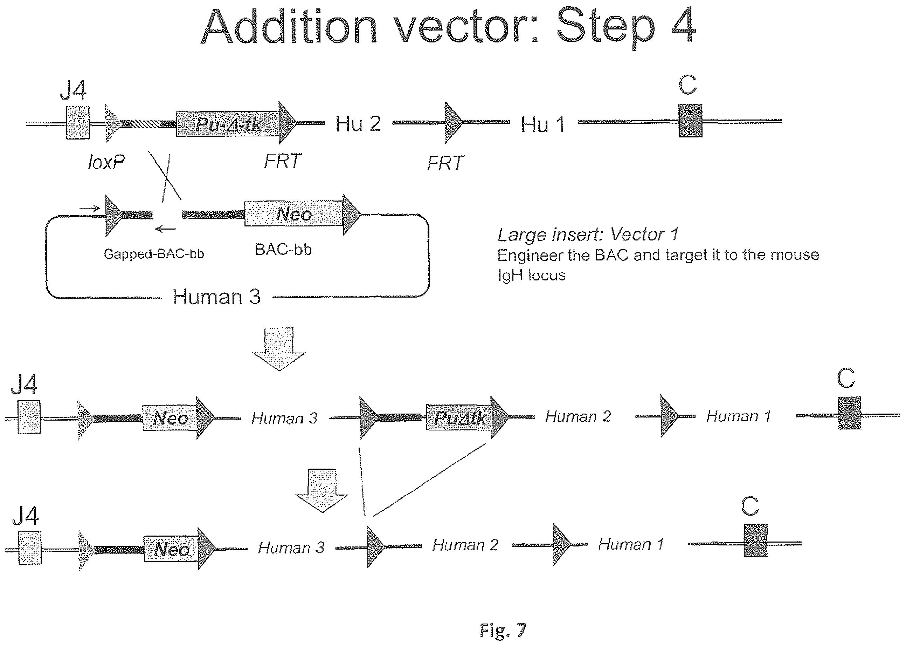

Figure 7 illustrates the next large insert vector targeted to the mouse IgH locus. The pu-delta TK cassette is then removed, as for figure 4. The process can be repeated to incorporate other BACs.

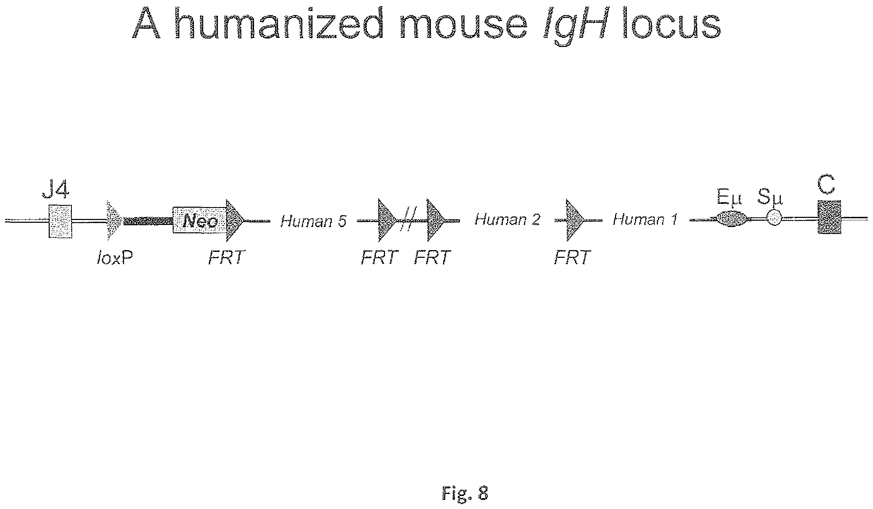

Figure 8 illustrates the final predicted ES cell construct.

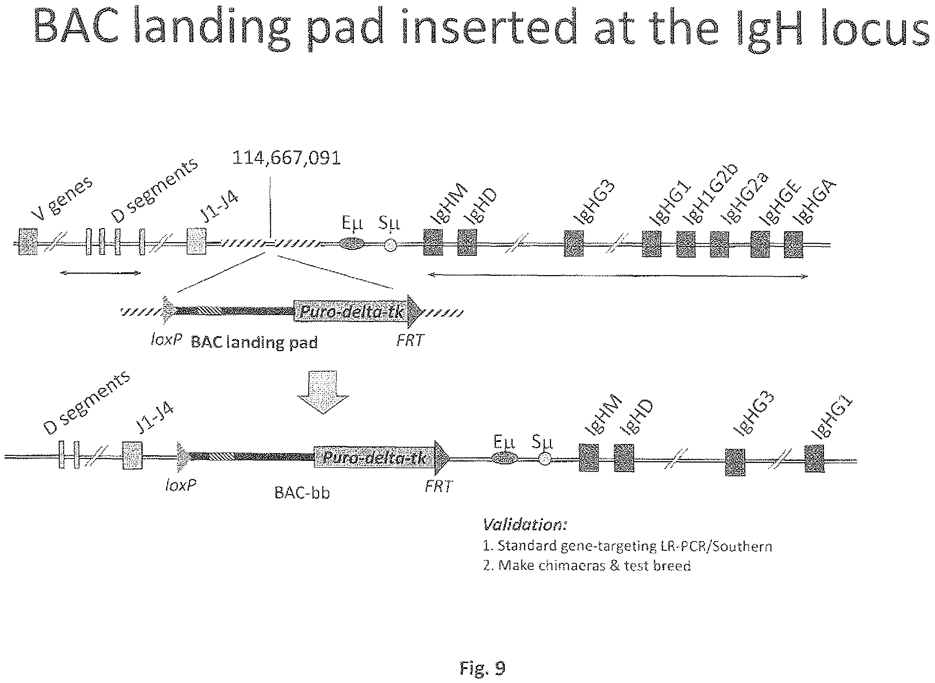

Figures 9 - 18 provide a further level of detail of this process.

### Example 2

In a further method of the invention site specific recombination can also be employed. Site-specific recombination (SSR) has been widely used in the last 20-years for the integration of transgenes into defined chromosomal loci. SSR involves recombination between homologous DNA sequences.

The first generation of SSR-based chromosomal targeting involved recombination between (i) a single recombination target site (RT) such as loxP or FRT in a transfected plasmid with (ii) a chromosomal RT site provided by a previous integration. A major problem with this approach is that insertion events are rare since excision is always more efficient than insertion. A second generation of SSR called RMCE (recombinase-mediated cassette exchange) was introduced by Schlake and Bode in 1994 (Schlake, T.; J. Bode (1994). "Use of mutated FLP-recognition-target-(FRT-)sites for the exchange of expression cassettes at defined chromosomal loci". Biochemistry 33: 12746-12751). Their method is based on using two heterospecific and incompatible RTs in the transfected plasmid which can recombine with compatible RT sites on the chromosome resulting in the swap of one piece of DNA for another - or a cassette exchange. This approach has been successfully exploited in a variety of efficient chromosomal targeting, including integration of BAC inserts of greater than 50 kb (Wallace, H.A.C. et al. (2007). "Manipulating the mouse genome to engineering precise functional syntenic replacements with human sequence". Cell 128: 197-209; Prosser, H.M. et al. (2008). "Mosaic complementation demonstrates a regulatory role for myosin VIIa in actin dynamics of Stereocilia". Mol. Cell. Biol. 28: 1702-12).

The largest insert size of a BAC is about 300-kb and therefore this places an upper limit on cassette size for RMCE.

In the present invention we utilise a new SSR-based technique called sequential RMCE (SRMCE), which allows continuous insertion of BAC inserts into the same locus.

The method comprises the steps of
1 insertion of DNA forming an initiation cassette (also called a landing pad herein) into the genome of a cell;
2 insertion of a first DNA fragment into the insertion site, the first DNA fragment comprising a first portion of a human DNA and a first vector portion containing a first selectable marker or generating a selectable marker upon insertion;
3 removal of part of the vector DNA;
4 insertion of a second DNA fragment into the vector portion of the first DNA fragment, the second DNA fragment containing a second portion of human DNA and a second vector portion, the second vector portion containing a second selectable marker, or generating a second selectable marker upon insertion;
5 removal of any vector DNA to allow the first and second human DNA fragments to form a contiguous sequence; and
6 iteration of the steps of insertion of a part of the human V(D)J DNA and vector DNA removal, as necessary, to produce a cell with all or part of the human VDJ or VJ region sufficient to be capable of generating a chimaeric antibody in conjunction with a host constant region,
wherein the insertion of at least one DNA fragment uses site specific recombination.

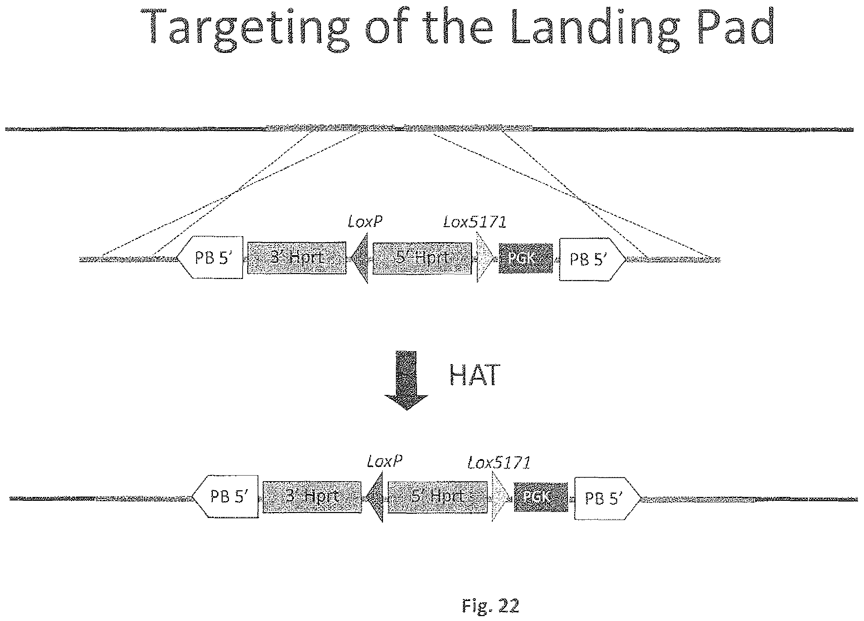

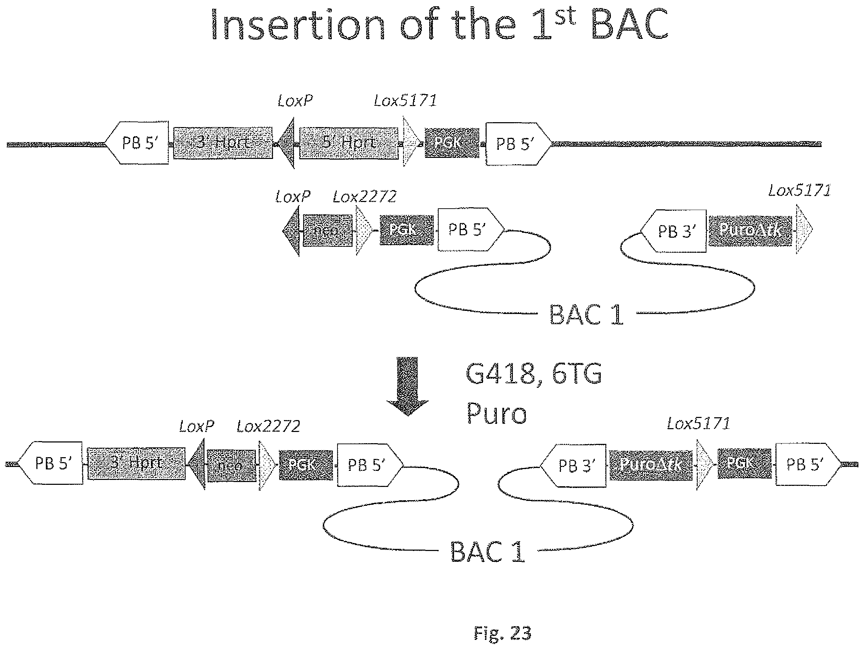

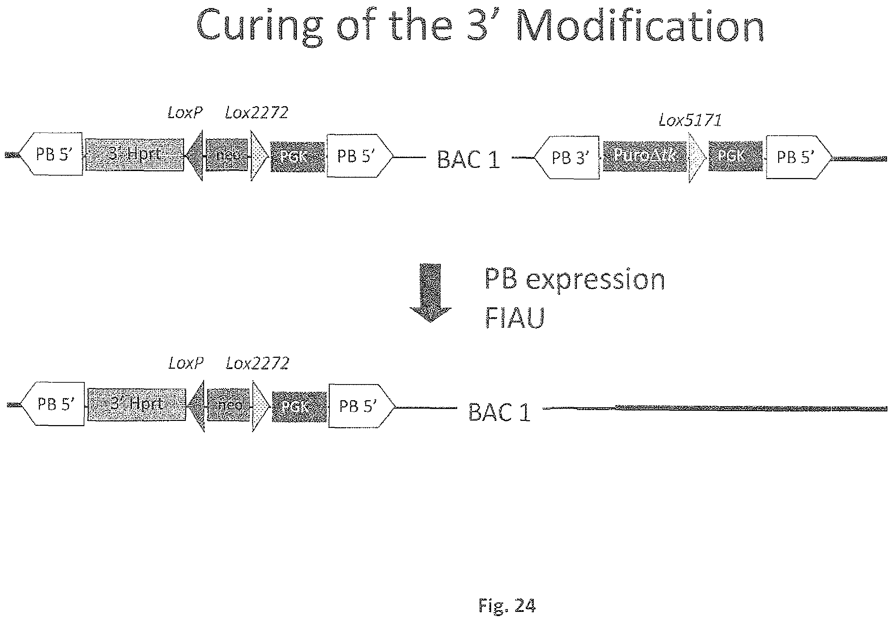

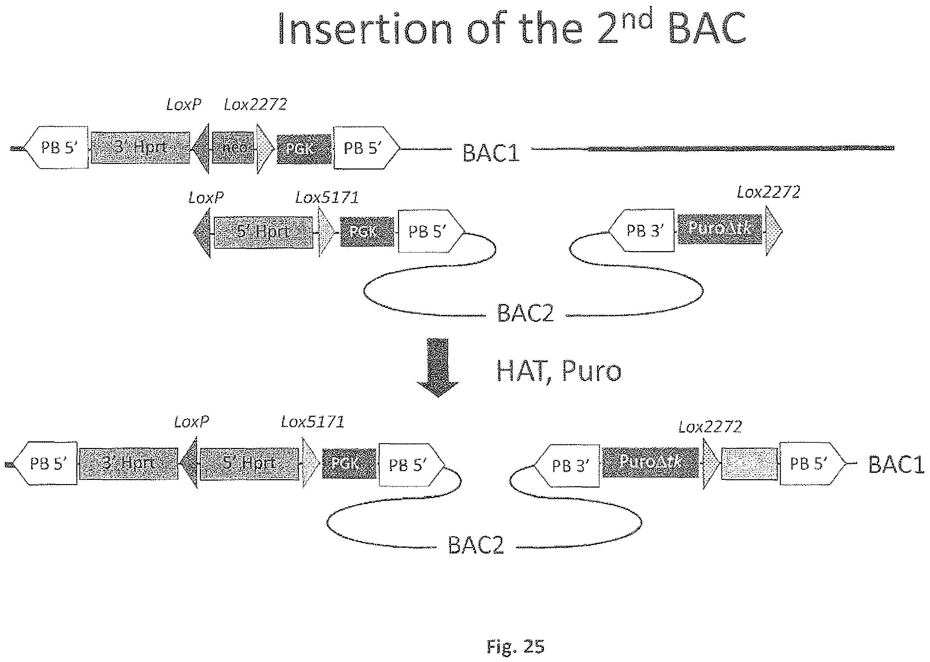

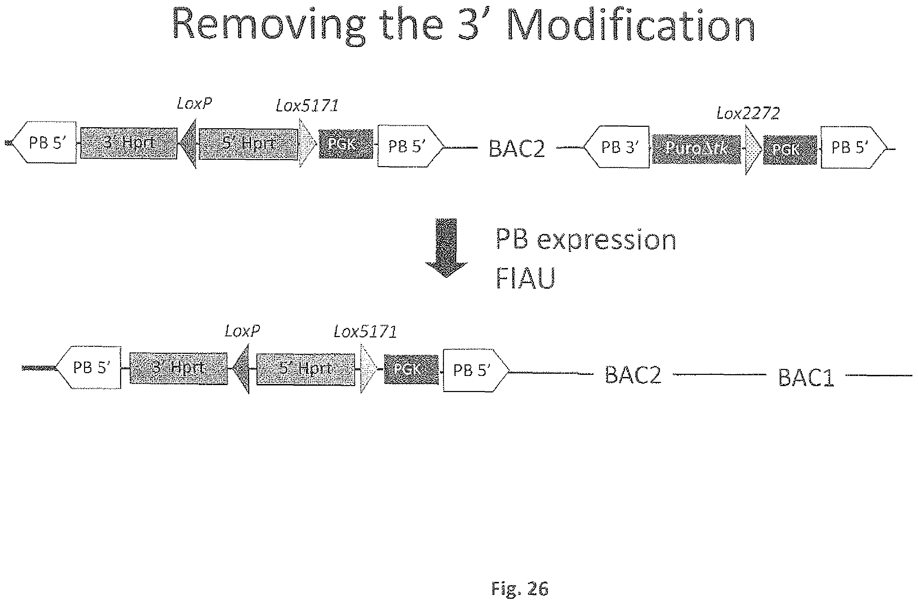

In one specific aspect the approach utilizes three heterospecific and incompatible loxP sites. The method is comprised of the steps as follows, and illustrated in Figures 22 - 26:
1. Targeting a landing pad into the defined locus. An entry vector containing an HPRT mini-gene flanked by inverted piggyBac (PB) ITRs is targeted into defined region (for example: a region between IGHJ and Eµ or IGKJ and Eκ or IGLC1 and Eλ3-1) to serve as a landing pad for BAC targeting. The HPRT mini-gene is comprised of two synthetic exons and associated intron. The 5' HPRT exon is flanked by two heterospecific and incompatible loxP sites (one wild-type and the other a mutated site, lox5171) in inverted orientation to each other (Fig. 22). These two loxP sites provide recombination sites for the BAC insertion through RMCE.
2. Insertion of the 1^{st} modified BAC into the targeted landing pad. The 1^{st} BAC has a length of DNA to be inserted into the genome flanked by engineered modifications. The 5' modification (loxP - neo gene - lox2272 - PGK promoter - PB 5'LTR) and 3' modification (PB3'LTR - puroΔTK gene - lox5171) is depicted in Fig 23 along with the relative orientations of the lox sites and PB LTRs. With transient CRE expression from a co-electroporated vector, the DNA sequence would be inserted into the defined locus through RMCE. The cells in which a correct insertion has occurred can be selected as follows: (i) Puromycin-resistance (the puroΔTK gene has acquired a promoter - "PGK" - from the landing pad), (ii) 6TG-resistance (the HPRT mini-gene has been disrupted), and (iii) G418-resistance (selects for any insertion via the 5' region PGK-neo arrangement). Any combination of these selection regimes can be used. G418- and 6TG-resistance select for correct events on the 5' end while puro-resistance selects for correct events on the 3' end.
3. Curing (removing) the 3' modification of the 1^{st} insertion. A properly inserted 1^{st} BAC results the 3' end having a puroΔTK gene flanked by inverted PB LTRs (Fig. 24) - essentially a proper transposon structure. This transposon can then be removed by the transient expression of the piggyBac transposase (from an electroporated vector). Cells with the correct excision event can be selected by FIAU resistance - ie, no thymidine kinase activity from the puroΔTK gene. This completely removes the 3' modification leaving no trace nucleotides.
4. Insertion of a 2^{nd} modified BAC into the 5' end of 1^{st} insertion. The 2^{nd} BAC has a length of DNA to be inserted into the genome (usually intended to be contiguous with the DNA inserted with the 1^{st} BAC) flanked by engineered modifications. The 5' modification (loxP - HPRT mini gene 5' portion - lox5171 - PGK promoter - PB5'LTR) and 3' modification (PB3'LTR - puroΔTK - lox2272) is depicted in Fig 25 along with the relative orientations of the lox sites and PB LTRs. With transient CRE expression from a co-electroporated vector, the DNA sequence would be inserted into the defined locus through RMCE. The cells in which a correct insertion has occurred can be selected as follows: (i) HAT-resistance (the HPRT mini-gene is reconstituted by a correct insertion event, ie: the 5' and 3' exon structures are brought together), and (ii) puromycin-resistance (puroΔTK gene has acquired a promoter - "PGK" - from the landing pad).
5. Curing (removing) the 3' modification of the 2^{nd} insertion. A properly inserted 2^{nd} BAC results the 3' end having a puroΔTK gene flanked by inverted PB LTRs (Fig. 26) - essentially a proper transposon structure, exactly analogous to the consequence of a successful 1^{st} BAC insertion. And therefore this transposon can likewise be removed by the transient expression of the piggyBac transposase (from an electroporated vector). Cells with the correct excision event can be selected by FIAU resistance - ie, no thymidine kinase activity from the puroΔTK gene. This completely removes the 3' modification leaving no trace nucleotides.
6. After curing of the 3' modification of the 2^{nd} BAC insertion, the landing pad becomes identical to the original. This entire process, steps 2 through 5, can be repeated multiple times to build up a large insertion into the genome. When complete, there are no residual nucleotides remaining other than the desired insertion.

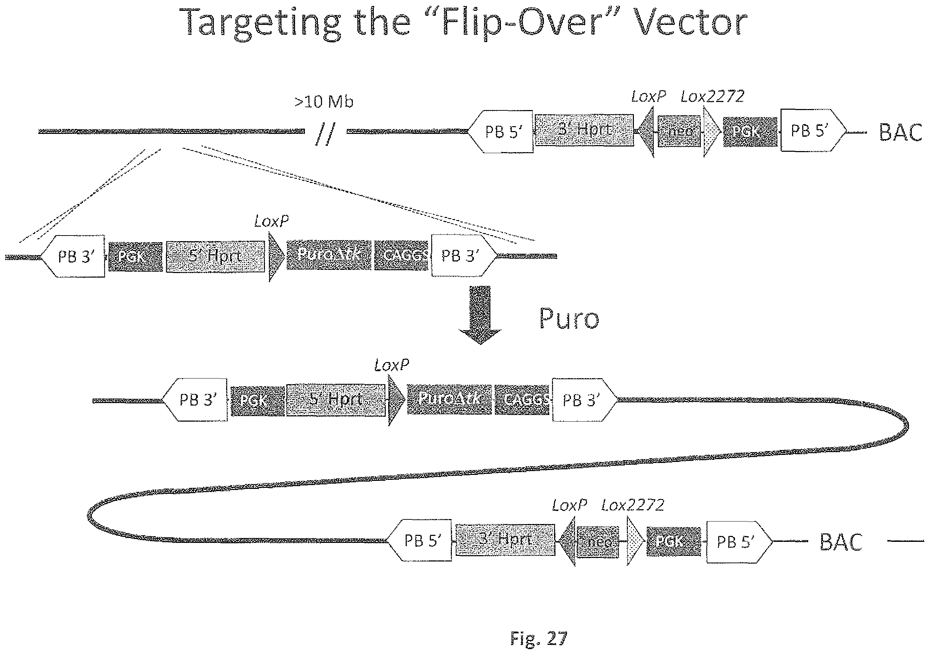

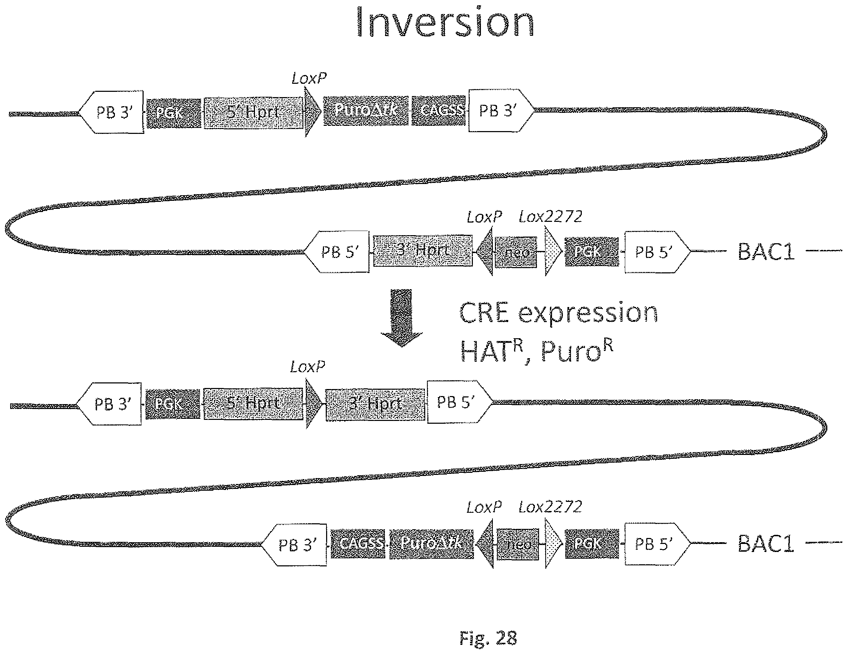

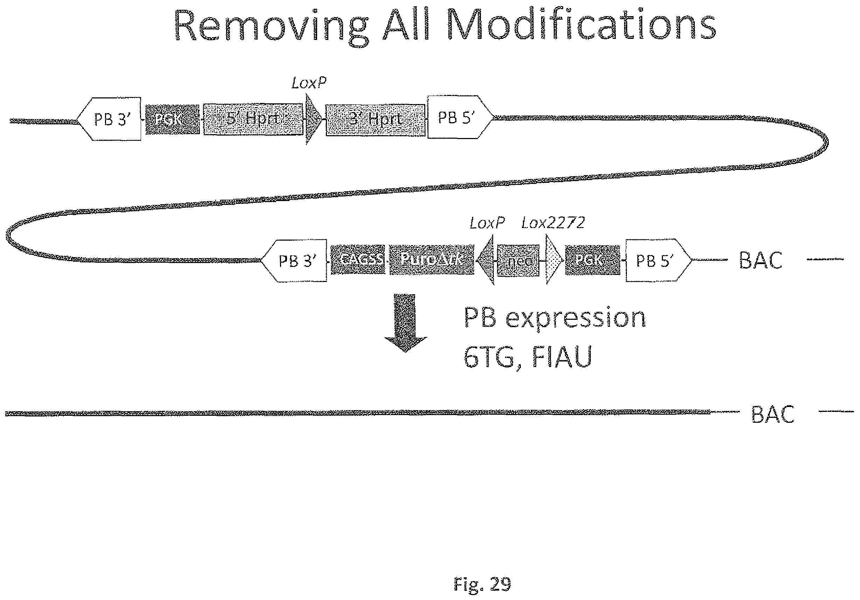

With the insertion of an odd number of BACs into the Ig loci, the endogenous VDJ or VJ sequences can be inactivated through an inversion via chromosomal engineering as follows (see figures 27 - 29):
1. Targeting a "flip-over" cassette into a 5' region 10 to 40 megabases away from the endogenous VDJ or VJ. The flip-over vector (PB3'LTR - PGK promoter - HPRT mini gene 5' portion - loxP - puroΔTK - CAGGS promoter - PB3'LTR) is depicted in Fig 27 along with the relative orientations of the lox sites and PB LTRs.
2. Transient CRE expression will result in recombination between the loxP site in the "flip-over" cassette and the loxP site in the 5' modification. This 5' modification is as described in Steps 2 and 3 above - essentially the modification resulting from insertion of an odd number of BACs, after the 3' modification has been cured. The loxP sites are inverted relative to one another and therefore the described recombination event results in an inversion as depicted in Fig 28. Cells with the correct inversion will be HAT-resistance since the HPRT mini-gene is reconstituted by a correct inversion.
3. A correct inversion also leaves two transposon structures flanking the "flip-over" cassette and the 5' modification. Both can be excised with transient piggyBAC transposase expression, leaving no remnant of either modification (Fig 29). Cells with the correct excisions can be selected as follows: (i) 6TG-resistance (the HPRT mini-gene is deleted) and (ii) FIAU-resistance (the puroΔTK gene is deleted). An inversion as described in the Ig loci would move the endogenous IGH-VDJ or IGK-VJ region away from the Eµ or Eκ enhancer region, respectively, and lead to inactivation of the endogenous IGH-VDJ or IGK-VJ regions.

The methods of insertion of the invention suitably provide one or more of:
Selection at both 5' and 3' ends of the inserted DNA fragment;
Efficient curing of the 3' modification, preferably by transposase mediated DNA excision;
Inactivation of endogenous IGH or IGK activity through an inversion; and
Excision of modifications, leaving no nucleotide traces remaining in the chromosome.

### Example 3

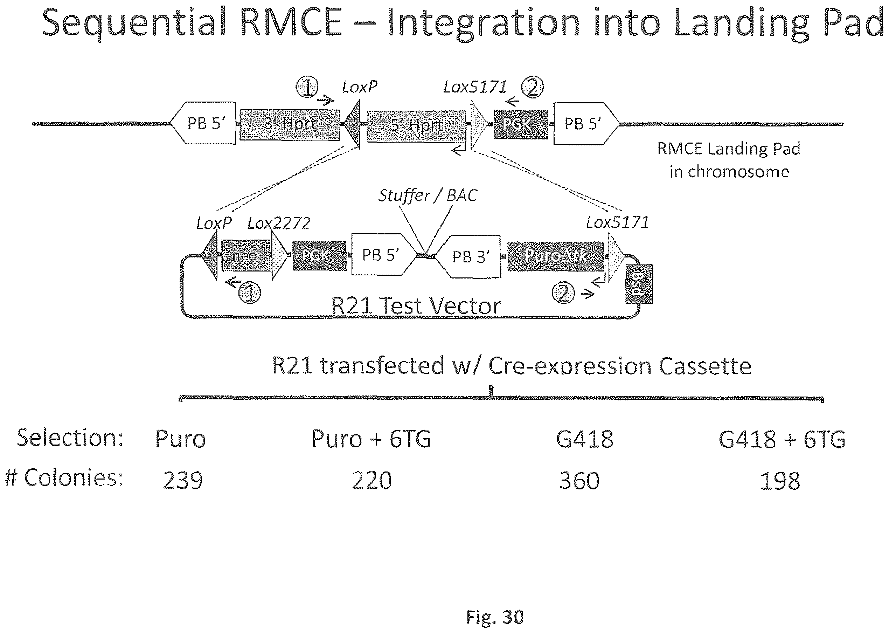

Proof of concept of the approach is disclosed in Figure 30. In Figure 30 a landing pad as shown in figure 22 was inserted into the genome of a mouse by homologous recombination, followed by insertion of the R21 plasmid into that landing pad via cre-mediated site specific recombination. The insertion event generated a number of general insertion events, 360 G418 resistant colonies, of which -220 were inserted into the desired locus, as demonstrated by disruption of the HRPT minilocus.

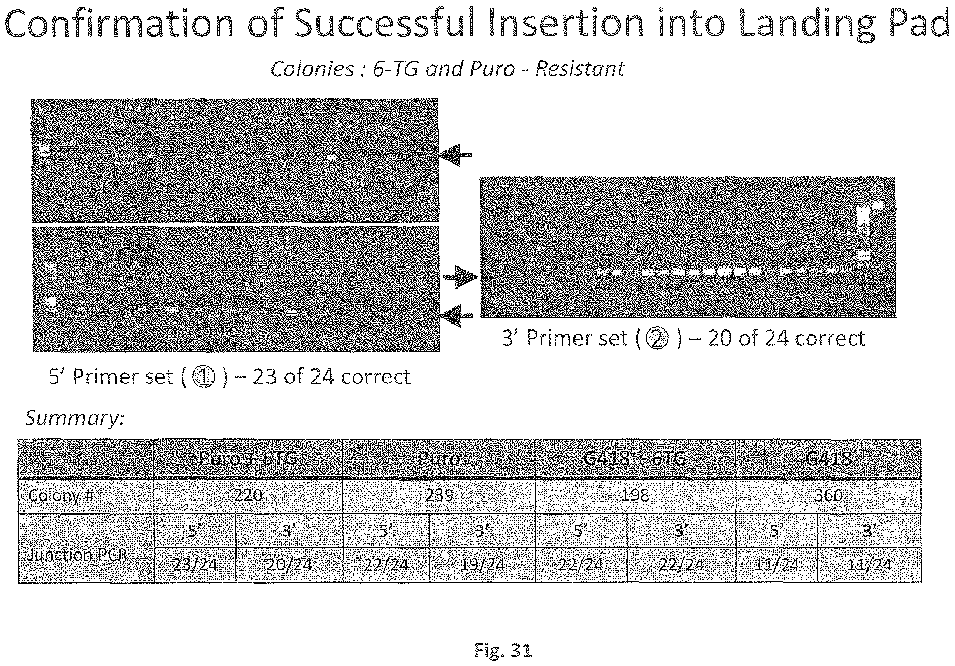

The R21 vector mimicks the 1^{st} BAC insertion vector at the 5' and 3' ends, including all selection elements and recombinase target sites. In place of BAC sequences, there is a small 'stuffer' sequence. This vector will both test all the principals designed in the invention and allow easy testing of the results in that PCR across the stuffer is feasible and therefore allows both ends of the insertion to be easily tested. R21 was co-electroporated with a cre-expressing vector into the ES cells harbouring the landing pad in the IGH locus. Four sets of transformed cells were transfected in parallel and then placed under different selection regimes as indicated in Figure 30. G418 selection (neo gene expression) resulted in the largest number of colonies due to there being no requirement for specific landing-pad integration. Any integration of R21 into the genome will provide neo expression leading to G418-resistance. Puro selection resulted in a similar colony number to Puro + 6TG or G418 + 6TG, suggesting that the stringency of Puro selection is due to the PuroΔTK lacking a promoter in the vector. Puro expression is only acquired when an integration occurs near a promoter element - in this design most likely specifically in the landing pad. These conclusions are supported by the results from junction PCR which is shown in Figure 31.

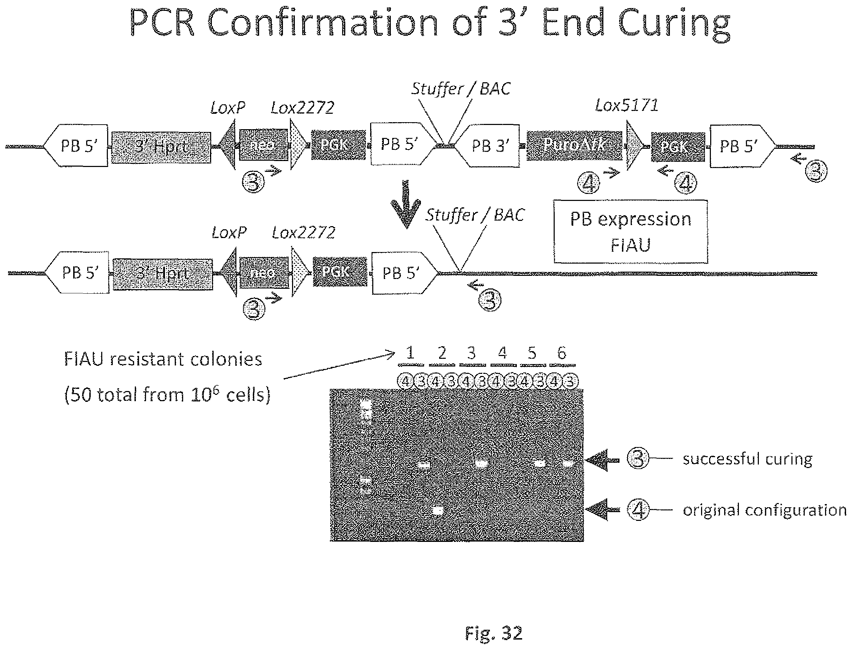

The next step in the invention is to 'cure' the 3' end of the integrated BAC vector, leaving a seamless transition between the insertion and the flanking genome. We demonstrated this curing by expanding an individual clone from above (R21 inserted into the landing pad) and expressing piggyBac recombinase in this clone via transfection of an expressing plasmid. FIAU was used to select colonies in which the 3' modification was excised - ie, through loss of the 'PGK-puroΔTK' element between the piggyBac terminal repeats. Fifty such clones resulted from a transfection of 10⁶ cells; of these we tested 6 for the expected genomic structure. Successful curing resulted in positive PCR between the primer set labelled "3" in Figure 32. Of the 6 clones, 4 had correct excisions, 1 clone remained in the original configuration and 1 other had a deletion.

These data demonstrate iterative insertion of DNA into a landing pad at a defined genomic locus using the approaches outlined above.

### Example 4

Example 3 demonstrated that the design of the claimed invention was capable of providing for the insertion of a test vector into the genome at a defined location, in this case the R21 vector into the mouse IGH locus. The use of the appropriate selection media and the expression of cre-recombinase resulted in a genomic alteration with the predicted structure.

The same design elements described in this invention were built into the 5' and 3' ends of a BAC insert. Said insert comprised human sequences from the IGH locus and was approximately 166-kb. This engineered BAC was electroporated along with a cre-expressing plasmid DNA into mouse ES cells harbouring the landing pad at the mouse IGH locus. The transfected cell population was grown in puro-containing media to select for appropriate insertion events.

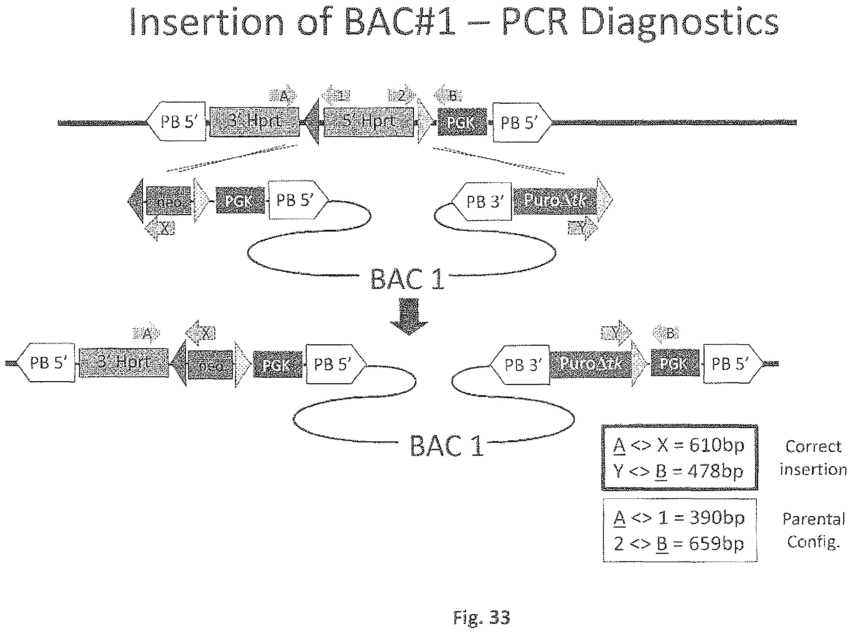

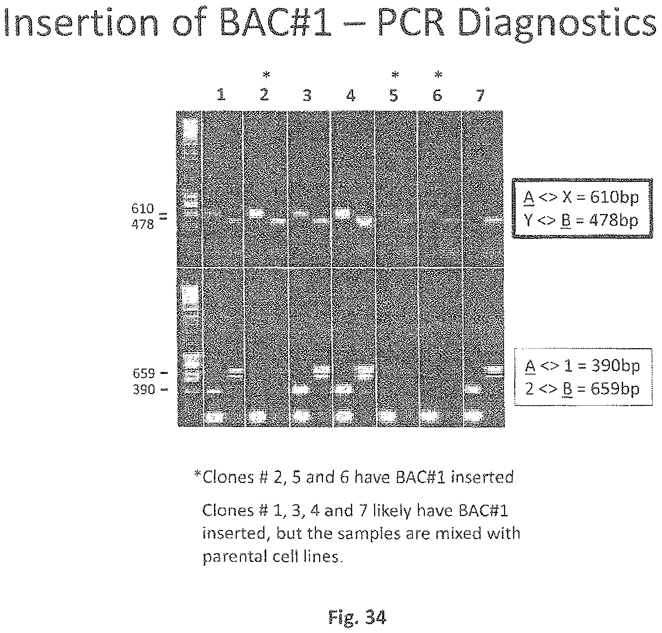

Seven resulting clones were isolated and further analysed. The expected recombination event and resulting structure are depicted in Figure 33. Based upon data from the R21 experiment outlined in Example 3, a stringent selection for correct clones was expected when the transfected population was selected in puro-containing media. This is because the puro-coding region requires a promoter element and this is preferentially supplied by the landing pad after recombination. Accordingly, the majority of the 7 isolated clones had inserted correctly into the genome at the landing pad as determined by the diagnostic PCR. The primers for diagnosing a correct insertion are depicted in Figure 33. Correct junctions are present in the genome if a 610-bp fragment is amplified between primers 'A' and 'X' and a 478-bp fragment is amplified between primers 'Y' and 'B' (Figures 33 and 34). Note that there are amplified fragments between 'A' and '1' primers and '2' and 'B' primers indicating the presence of parental genome (that is, the landing pad alone). These result from parental cells present internally in the cell colonies under puro-selection that escape the selection due to the geometry of a colony. After passaging the colony through puro-containing media, these parental junction fragments disappear indicating that the parental cells are removed from the population. In addition, all the clones were shown to be resistant to 6-TG as expected if the HPRT gene is inactivated by the correct insertion event.

These data indicate that the disclosed strategy for inserting large parts of the human IG loci into defined positions in the mouse genome will enable the construction of a mouse with a plurality of the variable regions of human IG regions upstream of the mouse constant regions as described.

### Example 5

Bacterial artificial chromosomes (BACs) were created, wherein the BACs had inserts of human Ig gene segments (human V, D and/or J gene segments). Using methods described herein, landing pads were used in a method to construct chimaeric Ig loci in mouse embryonic stem cells (ES cells), such that chimaeric IgH and IgK loci were provided in which human gene segments are functionally inserted upstream of endogenous constant regions. To test if the human IgH-VDJ or IgK-VJ gene segments in the chimaera mice derived from human BAC-inserted ES cell clones appropriately rearrange and express, we performed RT-PCR for the RNA samples of white blood cells from those mice with the primer pairs of human variable(V) region and mouse constant(C) region. The sequences of oligos are shown as follows. Each V oligo is paired with C oligo (HV with Cµ; KV with Cκ) for PCR reaction.

| Oligo | Sequence |
|---|---|
| HV2-5 | AGATCACCTTGAAGGAGTCTGGTCC (SEQ ID NO 7) |
| HV4-4 | TGGTGAAGCCTTCGGAGACCCTGTC (SEQ ID NO 8) |
| HV1-3 | CACTAGCTATGCTATGCATTGGGTG (SEQ ID NO 9) |
| HV1-2 | ATGGATCAACCCTAACAGTGGTGGC (SEQ ID NO 10) |
| HV6-1 | GGAAGGACATACTACAGGTCCAAGT (SEQ ID NO 11) |
| Cµ | TAGGTACTTGCCCCCTGTCCTCAGT (SEQ ID NO 12) |
| KV1-9 | AGCCCAGTGTGTTCCGTACAGCCTG (SEQ ID NO 13) |
| KV1-8 | ATCCTCATTCTCTGCATCTACAGGA (SEQ ID NO 14) |
| KV1-6 | GGTAAGGATGGAGAACACTGGCAGT (SEQ ID NO 15) |
| KV1-5 | TTAGTAGCTGGTTGGCCTGGTATCA (SEQ ID NO 16) |
| CK | CTTTGCTGTCCTGATCAGTCCAACT (SEQ ID NO 17) |

Using the one-step formulation of SuperScript™ III One-Step RT-PCR System with Platinum@ Taq High Fidelity, (http://www.invitrogen.com/site/us/en/home/References/protocols/nucleic-acid-amplification-and-expression-profiling/pcr-protocol/superscript-3-one-step-rt-pcr-system-with-platinum-taq-high-fidelity.html#prot3) (Invitrogen), both cDNA synthesis and PCR amplification were achieved in a single tube using gene-specific primers and target RNAs.

The RT-PCR results showed most of the human IGH-VDJ or IGK-VJ gene segments appropriately rearrange and express in the chimaera mice. To investigate the details about the diversity generated from VDJ/VJ rearrangement, those specific RT-PCR fragments were cloned into a common vector for sequencing.

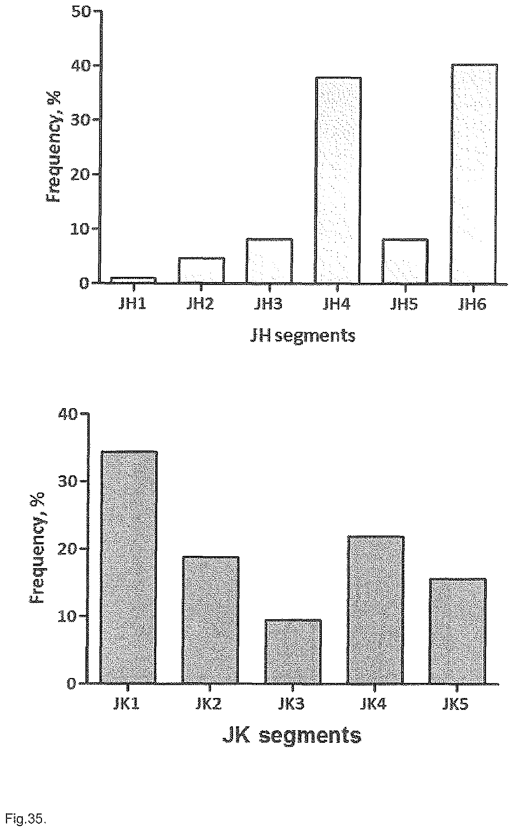

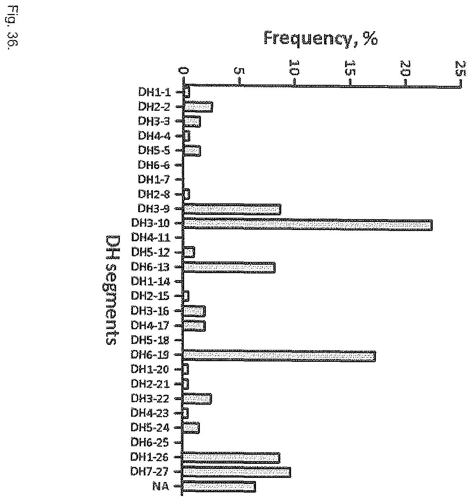

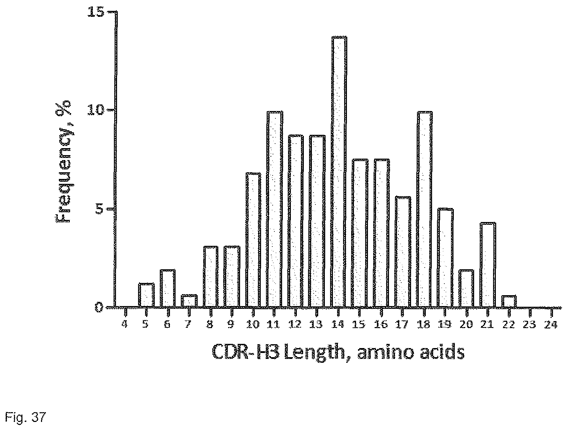

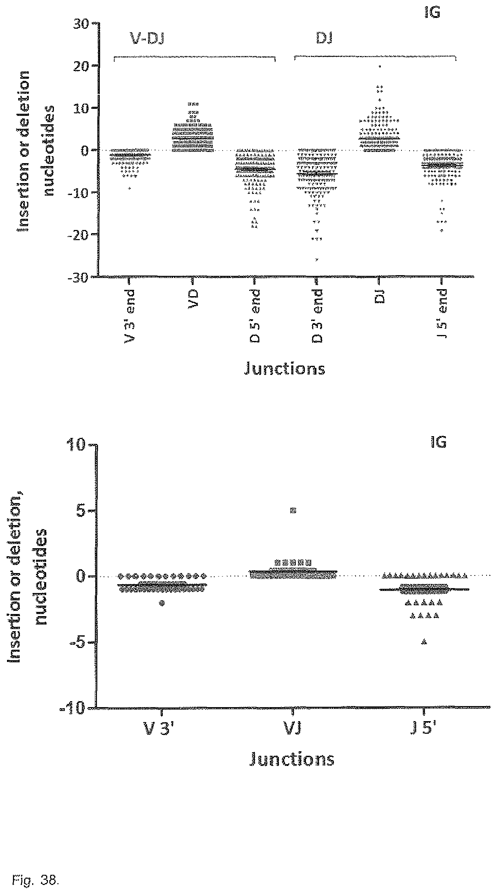

Sequencing results indicate that JH, DH, and JK usages (Fig. 35 and Fig. 36) are similar to human results. In addition, the results from the IGH-VDJCµ transcripts show that the range and mean of CDR-H3 length (Fig. 37) are similar to that observed in human. The junctional diversity generated from exonuclease and nucleotide addition activities (Fig. 38) was also observed. The IGH rearrangement possessed a higher frequency of these activities compared to the IGK one. These data suggest that the inserted loci are functional in terms of gene rearrangement, junctional diversity as well as expression.

### Example 6

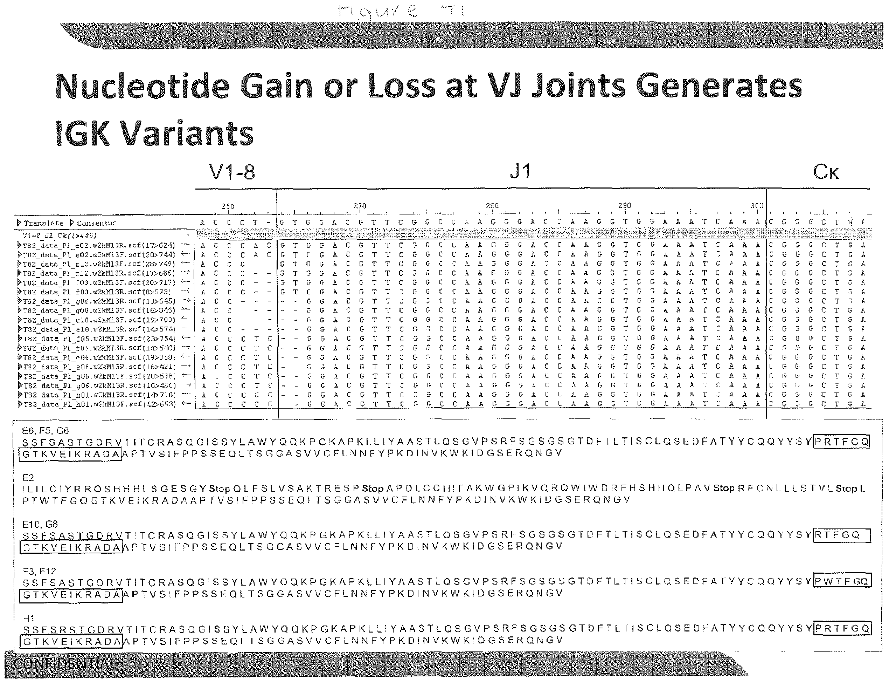

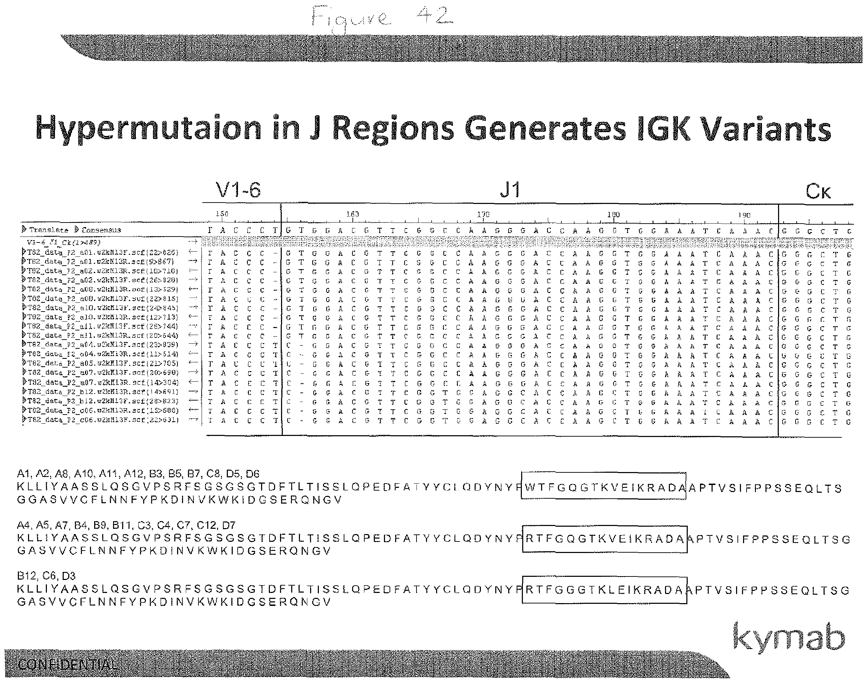

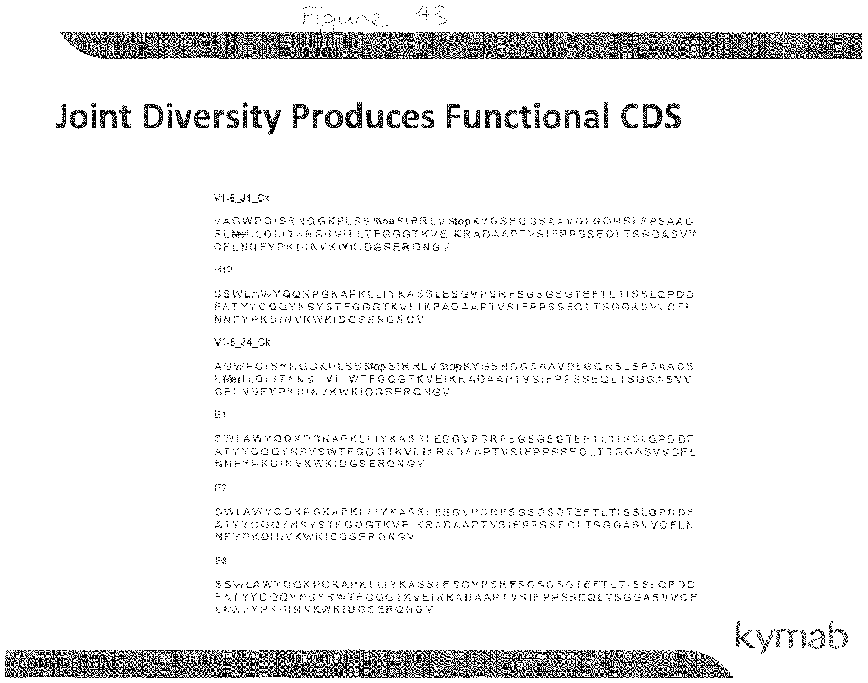

Figures 41 shows an analysis of kappa mRNA from mice B-cells bearing rearranged VJ, the VJ having been rearranged from human germline kappa V1-8 and J1, and demonstrates that both that productive VJ rearrangement and somatic hypermutation can be obtained, the latter as seen from the changes in antibodies encoded by mRNA with respect to the germline sequences. The same is displayed for V1-6 and J1 in Figure 42. Importantly, the recombination eliminates stop codons that are encoded by the combination of (unmutated) human germline gene segments, thereby allowing for antibody-encoding mRNA sequences. Figure 43 demonstrates that inserted human kappa V1-5 J1 and V1-5 J4 can produce functional coding sequences in vivo and junctional diversity.

### Statements of invention

1. A non-human mammal whose genome comprises:
   (a) a plurality of human IgH V regions, one or more human D regions and one or more human J regions upstream of the host non-human mammal constant region; and
   wherein the non-human mammal is able to produce a repertoire of chimaeric antibodies, or chimaeric heavy chains, having a non-human mammal constant region and a human variable region,
   wherein the insertion of the human DNA is made between the non-human mammal constant region and the last, 3', non-human mammal J region, and
   wherein the mammal comprises an insertion of the complete VJC human light chain region.
2. A non-human mammal cell whose genome comprises
   (a) a plurality of human IgH V regions, one or more human D regions and one or more human J regions upstream of the host non-human mammal constant region and
   wherein the insertion of the human DNA is made between the non-human mammal constant region and the last, 3', non-human mammal J region, and
   wherein the mammal comprises an insertion of the complete VJC human light chain region.
3. A non-human mammal whose genome comprises:
   (a) a plurality of human IgH V regions, one or more human D regions and one or more human J regions upstream of the host non-human mammal constant region; and
   (b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region;
   wherein the non-human mammal is able to produce a repertoire of chimaeric antibodies, or chimaeric light or heavy chains, having a non-human mammal constant region and a human variable region
   wherein the inserted human IgH VDJ region comprises, in germline configuration, all of the V, D and J regions and intervening sequences from a human.
4. A non-human mammal cell whose genome comprises.
   (a) a plurality of human IgH V regions, one or more human D regions and one or more human J regions upstream of the host non-human mammal constant region and
   (b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region
   wherein the inserted human IgH VDJ region comprises, in germline configuration, all of the V, D and J regions and intervening sequences from a human.
5. A non-human mammal whose genome comprises:
   (a) a plurality of human IgH V regions, one or more human D regions and one or more human J regions upstream of the host non-human mammal constant region; and
   (b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region
   wherein the non-human mammal is able to produce a repertoire of chimaeric antibodies, or chimaeric light or heavy chains, having a non-human mammal constant region and a human variable region;
   or a non-human mammal whose genome comprises
   (a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
   (b) optionally one or more human IgH V regions, one or more human D regions and one or more human J regions upstream of the host non-human mammal constant;
   wherein the non-human mammal is able to produce a repertoire of chimaeric antibodies, or chimaeric light or heavy chains, having a non-human mammal constant region and a human variable region
   wherein the non-human mammal genome into which DNA is inserted comprises endogenous V(D)J regions which have not been deleted
   and wherein the insertion of the human heavy chain DNA is made between the non-human mammal constant region and the last, 3', non-human mammal J region.
6. A non-human mammal cell whose genome comprises
   (a) a plurality of human IgH V regions, one or more human D regions and one or more human J regions upstream of the host non-human mammal constant region and
   (b) optionally one or more human ig iighi chain kappa V regions and one or mere human Ig light chain kappa J regions upstream of the host non-human mammal kappa constant region and/or one or more human Ig light chain lambda V regions and one or more human Ig light chain lambda J regions upstream of the host non-human mammal lambda constant region.
   or a non-human mammal cell whose genome comprises
   (a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
   (b) optionally one or more human IgH V regions, one or more human D regions and one or more human J regions upstream of the host non-human mammal constant region
   wherein the non-human mammal genome into which DNA is inserted comprises endogenous V(D)J regions which have not been deleted,
   and wherein the insertion of the human heavy chain DNA is made between the non-human mammal constant region and the last, 3', non-human mammal J region.
7. A non-human mammal whose genome comprises:
   (a) a plurality of human IgH V regions, one or more human D regions and one or more human J regions upstream of the host non-human mammal constant region; and
   (b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region;
   wherein the non-human mammal is able to produce a repertoire of chimaeric antibodies, or chimaeric light or heavy chains, having a non-human mammal constant region and a human variable region
   or a non-human mammal whose genome comprises
   (a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
   (b) optionally one or more human IgH V regions, one or more human D regions and one or more human J regions upstream of the host non-human mammal constant;
   wherein the non-human mammal is able to produce a repertoire of chimaeric antibodies, or chimaeric light or heavy chains, having a non-human mammal constant region and a human variable region
   wherein the non-human mammal genome is modified to prevent expression of the native (fully host species specific) antibodies by inversion of all or part of the non-human mammal VDJ region, or VJ region.
8. A non-human mammal cell whose genome comprises
   (a) a plurality of human IgH V regions, one or more human D regions and one or more human J regions upstream of the host non-human mammal constant region and
   (b) optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region.
   or a non-human mammal cell whose genome comprises
   (a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
   (b) optionally one or more human IgH V regions, one or more human D regions and one or more human J regions upstream of the host non-human mammal constant region
   wherein the non-human mammal genome is modified to prevent expression of the native (fully host species specific) antibodies by inversion of all or part of the non-human mammal VDJ region, or VJ region.
9. A non-human mammal whose genome comprises:
   (a) a plurality of human IgH V regions, one or more human D regions and one or more human J regions upstream of the host non-human mammal constant region; and
   wherein the non-human mammal is able to produce a repertoire of chimaeric antibodies, or chimaeric heavy chains, having a non-human mammal constant region and a human variable region,
   wherein the mammal is a mouse and the insertion of the human heavy chain DNA is made in a mouse genome between co-ordinates 114,667,090 and 114,665,190 of mouse chromosome 12 (co-ordinates refer to NCBIM37 for the mouse genome, relating to mouse strain C57BL/6J) or at equivalent position in another non-human mammal genome.
10. A non-human mammal cell whose genome comprises
   (a) a plurality of human IgH V regions, one or more human D regions and one or more human J regions upstream of the host non-human mammal constant region and
   wherein the mammal is a mouse and the insertion of the human heavy chain DNA is made in a mouse genome between co-ordinates 114,667,090 and 114,665,190 of mouse chromosome 12. (coordinates refer to NCBIM37 for the mouse genome, relating to mouse strain C57BL/6J) or at equivalent position in another non-human mammal genome.
11. A non-human mammal whose genome comprises:
   a plurality of human IgH V regions, one or more human D regions and one or more human J regions upstream of the host non-human mammal constant region; and
   wherein the non-human mammal is able to produce a repertoire of chimaeric antibodies, or chimaeric heavy chains, having a non-human mammal constant region and a human variable region,
   wherein the human IgH VDJ region comprises nucleotides 105,400,051 to 106,368,585 from human chromosome 14 (co-ordinates refer to NCBI36 for the human genome, ENSEMBL Release 54), or an equivalent human region from another human.
12. A non-human mammal cell whose genome comprises
   (a) a plurality of human IgH V regions, one or more human D regions and one or more human J regions upstream of the host non-human mammal constant region and
   wherein the human IgH VDJ region comprises nucleotides 105,400,051 to 106,368,585 from human chromosome 14 (co-ordinates refer to NCBI36 for the human genome,ENSEMBL Release 54), or an equivalent human region from another human.
13. A cell or mammal according to statements 3-4, 7- 8 or 11-12 wherein the insertion of the human DNA is made between the non-human mammal constant region and the last, 3' non- human mammal J region.
14. A cell or non-human mammal according to any of statements 1-8, or 11-13 wherein the mammal is a mouse or the cell is a mouse cell and wherein the insertion of the human heavy chain DNA is made in a mouse genome between co-ordinates 114,667,090 and 114,665,190 of mouse chromosome 12, such as between co-ordinates 114,667,089 and 114,667,090 (co-ordinates refer to NCBI m37, for the mouse C57BL/6J strain), or at equivalent position in another non-human mammal genome.
15. A cell or non-human mammal according to statements 1-8, 11-13 wherein the insertion of the human heavy chain DNA is made between positions 114,667,090 and 114,667,091.
16. A cell or non-human mammal according to statements 1-14 wherein the insertion of the human heavy chain DNA is made between co-ordinates 114, 666, 183 and 114, 666, 725, such as between co-ordinates 114,666,335 and 114,666,536, optionally between co-ordinates 114,666,385 and 114,666,486, optionally between co-ordinates 114,666,425 and 114,666,446, such as between co-ordinates 114,666,435 and 114,666,436 of mouse chromosome 12, with reference to NCBIM37 for the mouse genome relating to mouse strain C57BL/6J or an equivalent position of mouse chromosome 12 from a different mouse strain or an equivalent position in the genome of another non-human vertebrate.
17. A cell or mammal according to any preceding statement wherein the human IgH VDJ region comprises nucleotides 105,400,051 to 106,368,585 from human chromosome 14, co-ordinates refer to NCBI36 for the human genome, or equivalent nucleotides relating to chromosome 14 from a different human sequence or database.
18. A cell or mammal according to statement 1-16 wherein the human IgH VDJ region comprises or consists of nucleotides 106,328,851-107,268,544, such as nucleotides 106,328,901107,268,494, such as nucleotides 106,328,941-107,268,454, such as nucleotides 106,328,951107,268,444 of human Chromosome 14, with reference to the GRCH37/hgl9 sequence database, or equivalent nucleotides relating to chromosome 14 from a different human sequence or database.
19. A cell or mammal according to any preceding statement comprising an inserted human kappa VJ region comprising, in germline configuration, all of the V and J regions and intervening sequences from a human.
20. A cell or mammal according to statement 19 wherein the insertion of human kappa DNA is made between co-ordinates 70,673,918 - 70,675,517, such as between co-ordinates 70,674, 655 - 70,674,856, such as between co-ordinates 70,674, 705 - 70,674,906, such as between coordinates 70,674, 745 - 70,674,766, such as between co-ordinates 70,674,755 and 70,674,756 of mouse chromosome 6 (with reference to NCBIM37 for the mouse genome, relating to mouse strain C57BL/6J), or at an equivalent position in another genome.
21. A cell or mammal according to statement 19 or 20, wherein the inserted kappa DNA comprises or consists of a fragment from human chromosome 2, numbered with reference to the GRCH37/hgl9 sequence database, or equivalent nucleotides relating to chromosome 2 from a different human sequence or database, the fragment selected from 1 or more of:
   (i) nucleotides 89,158,979 - 89,630,537, such as 89,159,029-89,630,487, such as 89,159,069-89,630,447, such as 89,159,079 - 89,630,437, optionally in addition to fragment (ii) nucleotides 89,941,614 - 90,267,076, such as 89,941,664 - 90,267,026, such as 89, 941,704-90,266,986, such as 89,941,714 - 90,266,976; optionally in addition to fragment (i)
   (iii) nucleotides 89,158,979 - 90,267, 076, such as nucleotides 89,159,079 - 90,266,976.
22. A ceil or mamma! according to any of statements 3-21 comprising an inserted human lambda region which comprises at least one human JA region and at least one human CA region, optionally C*6 and/or C_{A}7.
23. A cell or mammal according to any of statements 3-22 which comprises a plurality of human JA regions , optionally two or more of J*1, J_{A}2, J*6 and J*7, optionally all of J*1, J*2, J*6 and J*7.
24. A cell or mammal according to any of statements 3-23 which comprises at least one human JᵣC_{A} cluster, optionally at least J_{A}7-C>,7.
25. A cell or mammal according to any preceding statement which comprises a human EA enhancer.
26. A cell or mammal according to any of statements 3-25 comprising an inserted human lambda VJ region which comprises, in germline configuration, all of the V and J regions and intervening sequences from a human.
27. A cell or mammal according to statement 26 wherein the region comprises or consists of nucleotides 22,375,509 - 23,327,984, such as nucleotides 22,375,559-23,327,934, such as nucleotides 22,375,599 - 23,327,894, such as nucleotides 22,375,609 - 23,327,884 from human chromosome 22, with reference to the GRCH37/hgl9 sequence database, or equivalent nucleotides relating to human chromosome 22 from a different human sequence or database:
28. A cell or mammal according to statement 26 or 27 wherein insertion into the mouse genome may be made between co-ordinates 19,027,763 and 19,061,845, such as between co-ordinates 19,047,451 and 19,047,652, such as between co-ordinates 19,047,491 and 19,047,602, such as between co-ordinates 19,047,541 and 19,047,562, such as between co-ordinates 19,047,551 and 19,047,552 of mouse Chromosome 16, with reference to NCBIM37 for the mouse genome, or at an equivalent position in other genome.
29. A cell or mammal according to statement 26 or 27 wherein insertion into the mouse genome may be made between co-ordinates 70,673,918 and 70,675,517 such as between co-ordinates 70,674,655 and 70,674,856, such as between co-ordinates 70,674,705 and 70,674,806, such as between co-ordinates 70,674,745 and 70,674,766, such as between co-ordinates 70,674,755 and 70,674,756 of mouse Chromosome 6, with reference to NCBIM37 for the mouse genome, relating to mouse strain C57BL/6J) or at an equivalent position in another genome.
30. A cell or mammal according to any preceding statement wherein the human light chain kappa VJC DNA, or part thereof, is inserted immediately upstream of the mouse kappa VJC region.
31. A cell or mammal according to statements 1-6 and 9-30 wherein the cell or mammal genome is modified to prevent or reduce expression of fully host-species specific antibodies.
32. A cell or mammal according to statement 31 wherein the non-human mammal genome is modified by inversion of all or part of the non-human mammal VDJ region, or VJ region.
33. A cell or mammal according to statements 1-4 and 7-32 wherein the non-human mammal genome into which DNA is inserted comprises endogenous V(D)J regions which have not been deleted.
34. A non-human cell or mammal according to any preceding statement generated in a genetic background which prevents the production of mature host B and T lymphocytes.
35. A non-human mammal according to statement 34 generated in a Rag-1 or Rag-2 deficient background.
36. A cell according to any of statements 2, 4, 6, 8, 10, 12 - 35 which is an ES cell, hematopoietic stem cell or other cell capable of developing into a non-human mammal able to produce a repertoire of antibodies or antibody chains which are chimaeric, said chimaeric antibodies or chains having a non-human mammal constant region and a human variable region.
37. A cell according to any of statements 2, 4, 6, 8, 10, 12- 36 which is an ES cell, hematopoietic stem cell or other cell capable of contributing to tissues and organs of a non-human mammal which is able to produce a repertoire of antibodies or antibody chains which are chimaeric, said chimaeric antibodies or chains having a non-human mammal constant region and a human variable region.
38. A cell or mammal according to any preceding statement claims comprising inserted human variable region DNA from at least a human heavy and human light chain.
39. A cell or mammal according to statements 1-38 wherein the cell or mammal is homozygous at one, two or all three immunoglobulin loci for DNA encoding a chimaeric antibody chain.
40. A cell or mammal according to statements 1-39 wherein the cell or mammal is heterozygous at one, two or all three immunoglobulin loci for DNA encoding a chimaeric heavy or light chain.
41. A cell or mammal according to statements 3-40 wherein the genome of the cell does not comprise constant region DNA from another cell or organism.
42. A cell according to statements 2, 4, 6, 10, 12 - 41 which is immortalised.
43. A cell or mammal according to any preceding statement the genome of which comprises a non-human, non-host switch sequence, such as S-mu.
44. A cell or mammal according to statement 43 wherein the switch sequence is a rat switch
45. A cell or mammal according to statement 44 wherein the rat switch:
   comprises or consists of GAGCT (296 repeats), GGGGT (50 repeats), and GGGCT (83repeats), and/or
   comprises 3,4, 5, 6 or more contiguous repeats of the sequence GGGCT.
46. A cell or mammai according to ciaim 45 wherein the rat switch has the sequence of SEGof
   ID NO 1.
47. A cell or mammal according to any preceding statement wherein the switch is from mouse 129 strain, optionally having SEQ ID no 4.
48. A cell or mammal according to statements 1-46 wherein the switch is from mouse C57 strain, optionally having SEQ ID no 5.
49. A cell according to any preceding statement which is ES cell line AB2.1, or a cell from a mouse strain selected from C57BL/6, M129 such as 129/SV, BALB/c, and any hybrid C57BL/6, M129 such as 129/SV, or BALB/c.
50. A method for producing a non-human mammal or cell, the method comprising inserting into a non-human mammal cell genome;
   (a) a plurality of human IgH V regions, one or more human D regions and one or more human J regions upstream of the host non-human mammal constant region; and
      optionally one or more human Ig light chain kappa V regions and one or more human Ig light chain kappa J regions upstream of the host non-human mammal kappa constant region and/or one or more human Ig light chain lambda V regions and one or more human Ig light chain lambda J regions upstream of the host non-human mammal lambda constant region; respectively,
   (b) the insertion being such that the non-human mammal is able to produce a repertoire of chimaeric antibodies or heavy or light antibody chains having a non-human mammal constant region and a human variable region, wherein steps (a) and (b) can be carried out in either order and each of steps (a) and (b) can be carried out in a stepwise manner or as a single step.
51. A method for producing a non-human mammal or cell, the method comprising inserting into a non-human mammal cell genome;
   (a) a plurality of human Ig light chain kappa V regions and one or more human Ig light chain kappa J regions upstream of the host non-human mammal kappa constant region and/or a plurality of human Ig light chain lambda V regions and one or more human Ig light chain lambda J regions upstream of the host non-human mammal lambda constant region; and
   (b) optionally one or more human IgH V regions, one or more human D regions and one or more human J regions upstream of the host non-human mammal constant;
   the insertion being such that the non-human mammal is able to produce a repertoire of chimaeric antibodies or heavy or light antibody chains having a non-human mammal constant region and a human variable region, wherein steps (a) and (b) can be carried out in either order and each of steps (a) and (b) can be carried out in a stepwise manner or as a single step.
52. A method according to statement 50 or 51 wherein the non-human mammal genome is then modified to prevent expression of the native (fully host species specific) antibodies in the mammal, optionally by inversion of all or part of the non-human mammal VDJ or VJ region, optionally by insertion of one or more site specific recombinase sites into the genome and then use of these sites in recombinase-mediated excision or inversion of all or a part of the non-human mammal Ig locus.
53. A method according to statement 50 - 52 wherein the cell is an ES cell.
54. A method according to statement 50-53 wherein the insertion of human VDJ or VJ regions upstream of the host non-human mammal constant region is accomplished by step-wise insertion of multiple fragments by homologous recombination.
55. A method according to statement 50- 54 wherein the insertion process commences at a site where an initiation cassette has been inserted into the genome of an ES cell providing a unique targeting region.
56. A method according to statements 50-55 wherein one or more insertion events utilises site specific recombination.
57. A method according to statement 56 which utilises recombination mediated by, or involving, one or more of Frt sites, Flp recombinase: Dre recombinase, Rox sites, or PhiC31 recombinase.
58. A method according to statements 50-57 wherein the method comprises the steps of
   1. insertion of DNA forming an initiation cassette (also called a landing pad herein) into the genome of a cell;
   2. insertion of a first DNA fragment into the insertion site, the first DNA fragment comprising a first portion of a human DNA and a first vector portion containing a first selectable marker or generating a selectable marker upon insertion;
   3. optionally removal of part of the vector DNA-;
   4. insertion of a second DNA fragment into the vector portion of the first DNA fragment, the second DNA fragment containing a second portion of human DNA and a second vector portion, the second vector portion containing a second selectable marker, or generating a second selectable marker upon insertion;
   5. removal of any vector DNA to allow the first and second human DNA fragments to form a contiguous sequence; and67
   6. iteration of the steps of insertion of a part of the human V(D)J DNA and vector DNA removal, as necessary, to produce a cell with all or part of the human VDJ or VJ region sufficient to be capable of generating a chimaeric antibody in conjunction with a host constant region,
   wherein the insertion of at least one DNA fragment uses site specific recombination.
59. A method according to any of statements 50- 58 wherein the inserted landing pad sequence comprises SEQ ID NO 6 or is that of any of statements SEQ ID No. 2 or SEQ ID No. 3.
60. A method according to any of statements 50 - 59 wherein the landing pad is inserted into the mouse chromosome by homologous recombination between mouse J1-4 and mouse C mu sequences.
61. A method according to statements 50- 60 wherein the landing pad is recombined into the mouse chromosome by homologous recombination between mouse J1-4 and E mu sequences.
62. A method according to statements 50-61 wherein the landing pad comprises a non-endogenous S-mu, such as a rat S-mu switch.
63. A method, cell or mammal as stated in statements 1-60 wherein a human coding region DNA sequence is in a functional arrangement with a non-human mammal control sequence, such that transcription of the human DNA is controlled by the non-human mammal control sequence.
64. A method for producing an antibody or antibody heavy or light chain specific to a desired antigen, the method comprising immunizing a non-human mammal as claimed in statements 1,3, 5, 7, 9, 11, 13-48 with the desired antigen and recovering the antibody or antibody chain or recovering a cell producing the antibody or heavy or light chain.
65. A method for producing a fully humanised antibody or antibody chain comprising immunizing a non-human mammal according to statement 64 and then replacing the non-human mammal constant region of an antibody specifically reactive with the antigen with a human constant region, suitably by engineering of the nucleic acid encoding the antibody.
66. A humanised antibody or antibody chain produced according to statement 64 or 65.
67. Use of a humanised antibody or chain produced according to statement 64 or 65 in medicine.
68. A humanised antibody or antibody chain produced according to statement 64 or 65 for use in medicine.
69. A pharmaceutical composition comprising an antibody according to statement 64 or 65 and a pharmaceutical^ acceptable carrier or other excipient.
70. A chimaeric antibody derivative of a chimaeric antibody produced according to statement 64.
71. A mouse whose genome comprises an insertion of human IgH VDJ DNA between co-ordinates 114,667,090 and 114,665,190 of mouse chromosome 12, such as between coordinates 114,667,089 and 114667090, the insert comprising nucleotides 105,400,051 to 106,368,585 from human chromosome 14 (co-ordinates refer to NCBI36 for the human genome and NCB! m37, for the mouse C57BL/6J strain, or equivalent coordinates in another human chromosome 14 sequence or in another mouse genome respectively,), the insertion being upstream of the host non-human mammal constant region such that the mouse is able to produce a repertoire of chimaeric heavy chains having a non-human mammal constant region and a human variable region, wherein the mammal also comprises an insertion of the complete VJC human light chain region such that a fully human lambda or kappa human antibody chain may be generated which is able to form an antibody with a chimaeric heavy chain.
72. A mouse whose genome comprises an insertion of human IgH VDJ DNA between co-ordinates 114,667,090 and 114,667,091 of mouse chromosome 12, the insert comprising or consisting of nucleotides 105,400,051 to 106,368,585 from human chromosome 14 (co-ordinates refer to NCBI36 for the human genome and NCBI m37 for the mouse C57BL/6J strain, or equivalent coordinates in another human chromosome 14 sequence or in another mouse genome respectively), the insertion being upstream of the mouse constant region such that the mouse is able to produce a repertoire of chimaeric heavy chains having a mouse constant region and a human variable region, wherein the mouse also comprises an insertion of the complete VJC human light chain region such that a fully human lambda or kappa human antibody chain may be generated which is able to form an antibody with a chimaeric heavy chain.
73. A mouse whose genome comprises an insertion of human IgH VDJ DNA between co-ordinates 114,667,090 and 114,665,190 of mouse chromosome 12, where co-ordinates refer to NCBI m37, for the mouse C57BL/6J strain, or an insertion at an equivalent position in another mouse strain, the insert comprising or consisting of nucleotides 106,328,951-107,268,444 from human chromosome 14, where co-ordinates refer to the GRCH37/hgl9 sequence database for humans, or the same nucleotides from an equivalent position in another human chromosome 14 sequence, the insertion being upstream of the host mouse constant region such that the mouse is able to produce a repertoire of chimaeric heavy chains having a mouse constant region and a human variable region, wherein the mouse also comprises an insertion of the complete VJC human light chain region which is functional to generate a fully human lambda or kappa human antibody chain which forms an antibody with a chimaeric heavy chain.
74. A mouse according to statement 48 wherein the insertion is between co-ordinates 114,666,435 and 114,666,436 of mouse chromosome 12.
75. A non-human mammal or a cell whose genome comprises a plurality of human IgH V regions, one or more human D regions and one or more human J regions upstream of a host non-human mammal light chain constant region, such that the cell or mammal is able to express a chimaeric antibody chain.
76. A non-human mammal or a cell whose genome comprises a plurality of human Ig light chain V regions, and one or more human J regions upstream of a host non-human mammal heavy chain constant region, such that the cell or mammal is able to express a chimaeric antibody chain.
77. A cell or mammal according to statement 75 or 76 which is able to express an antibody having both heavy and light chains, including one chimaeric antibody chain.
78. A transgene encoding a plurality of human IgH V regions, one or more human D regions and one or more human J regions upstream of a host non-human mammal light chain constant region.
79. A transgene encoding a plurality of human Ig light chain V regions, and one or more human light chain J regions upstream of a host non-human mammal heavy chain constant region.
80. A polynucleotide landing pad sequence, the polynucleotide comprising nucleic acid regions homologous to regions of a target chromosome to allow for insertion by homologous recombination into the target chromosome, and comprising a nucleic acid site which permits recombinase-driven insertion of a nucleic acid into the landing pad, wherein the polynucleotide sequence comprises one or more of:
   (i) a non-human non-mouse sequence, such as a rat switch sequence, optionally the sequence of SEQ ID no 1;
   (ii) in a 5' to 3' direction, a mouse Elj sequence, a non-human, non-murine switch, and mouse C(j;
   (iii) a 3' homology arm having the sequence of SEQ ID no 6.
81. A cell or non-human mammal comprising a landing pad sequence according to statement 79 which has been inserted into the genome of the cell.
82. A cell, mammal or landing pad according to statement 80 or 81 wherein the rat switch sequence comprises 3, 4, 5, 6 or more contiguous repeats of the sequence GGGCT, optionally being SEQ ID no 1.
83. A cell, mammal or landing pad according to statement 80 - 82 wherein the landing pad sequence comprises the sequence of SEQ ID 2,
84. A cell, mammal or landing pad according to statement 80 - 82 wherein the landing pad sequence comprises the sequence of SEQ ID 3.
85. A non-human cell or non-human mammal comprising:
   a fully human lambda locus comprising substantially all the lambda VJC regions from a human,
   a chimaeric kappa locus comprising substantially all human kappa VJ regions operatively linked to a non-human host kappa constant region and
   a chimaeric heavy chain locus, having a human VDJ region operatively linked to a non-human host heavy chain constant region.
86. A chimaeric antibody comprising a human variable region and a non-human vertebrate or mammal (optionally a rat or mouse) constant region (optionally a C gamma or C mu),
   wherein the antibody is encoded by a nucleotide sequence corresponding to the nucleotide sequence of a chimaeric heavy chain locus of a cell (optionally a B-cell, ES cell or hybridoma), the locus comprising a non-human vertebrate or mammal constant region nucleotide sequence and a rearranged VDJ nucleotide sequence produced by the in-vivo rearrangement of a human V segment, a human D segment and a human J segment,
   the V region being selected from one of a V1-3 region, V2-5 region, V4-4 region, V1-2 region or V6-1 region from a human, optionally a Vl-3 or V6-1 segment.
87. A chimaeric antibody according to statement 86 wherein the J region is JH4 or JH6.
88. A chimaeric antibody according to statement 86 or 87 wherein the D region is D3-9, D3-10, D6-13 or D-19.
89. A chimaeric antibody according to any of statements 86 - 88 comprising: Vl-3 JH4, Vl-3 JH6, V6-1 D3-10, V6-1 D6-19, Vl-3 D3-9, Vl-3 D3-10, Vl-3 D6-19.
90. A non-human vertebrate or mammal cell (eg, a B-cell or ES cell or hybridoma) encoding a chimaeric antibody according to statements 86 - 89.
91. A non-human vertebrate or mammal (eg, a mouse or rat) having a genome encoding a chimaeric antibody according to any of statements 86 - 90.
92. A non-human vertebrate or mammal (eg, a mouse or rat) having a genome encoding a chimaeric antibody according to any of statements 86-91, the mammal expressing more Vl-3 antibodies than V2-5, V4-4, V1-2 or V6-1 antibodies.
93. A non-human vertebrate or mammal (eg, a mouse or rat) having a genome encoding a chimaeric antibody according to any *of* statements 86 - 92, the mammal expressing more VI-3 JH4 or VI-3 JH6 antibodies than any of, individually, V1-3 JH1, V1-3 JH2, VI-3 JH3 or VI-3 JH5 antibodies.
94. A non-human vertebrate or mammal (eg, a mouse or rat) having a genome encoding a chimaeric antibody according to any of statements 86 - 93, the mammal expressing more V6-1 JH4 or V6-1 JH6 antibodies than any of, individually V6-1 JH1, V6-1 JH2, V6-1 JH3 or V6-1 JH5 antibodies.
95. A non-human vertebrate or mammal (eg, a mouse or rat) having a genome encoding a chimaeric antibody according to any of statements 86 - 94, the mammal expressing a greater number of V1-3 DH10 antibodies than antibodies V1-3 with any other D region.
96. A chimaeric antibody comprising a human variable region and a non-human vertebrate or mammal (optionally a rat or mouse) constant region (optionally a light chain constant region), wherein the antibody is obtainable from a mammal (optionally a rat or mouse) whose genome comprises an antibody chain locus comprising a germline human kappa V1-8 and germline human kappa J1 sequence, and wherein the antibody is obtainable by *in vivo* recombination in said mammal of the VI-8 and J1 sequences and wherein the antibody has a variable region sequence which is different from that which is encoded by germline human kappa V1-8 and germline human kappa J1 sequences.
97. A chimaeric antibody according to statement 96 wherein the antibody sequence comprises, a X1X2 T F G Q, where X1X2= PR, RT, or PW; optionally aX1X2TFGQGTKVEIKRADA motif.
98. A chimaeric antibody comprising a human variable region and a non-human vertebrate or mammal (optionally a rat or mouse) constant region (optionally a light chain constant region), wherein the antibody is obtainable from a mammal (optionally a rat or mouse) whose genome comprises an antibody chain locus comprising a germline human kappa V1-6 and germline human kappa J1 sequence, and wherein the antibody is obtainable by *in vivo* recombination in said mammal of the VI-6 and J1 sequences and wherein the antibody has a variable region sequence which is different from that which is encoded by germline human kappa V1-6 and germline human kappa J1 sequences.
99. A chimaeric antibody according to statement 98 wherein the antibody sequence comprises a X3X4 T F G Q, where X3X4= PR or PW; optionally a X3X4 TFGQGTKVEIKRADA motif.
100. A chimaeric antibody comprising a human variable region and a non-human (optionally a rat or mouse) constant region (optionally a C gamma or C mu or a C kappa), wherein the antibody is obtainable from a mammal (optionally a rat or mouse) whose genome comprises an antibody chain locus comprising a germline human kappa V1-5 and germline human kappa J1 sequence, and wherein the antibody is obtainable by *in vivo* recombination in said mammal of the V1-5 and J1 sequences.
101. A chimaeric antibody comprising a human variable region and a non-human (optionally a rat or mouse) constant region (optionally a G gamma, or C mu or a C kappa), wherein the antibody is obtainable from a mammal (optionally a rat or mouse) whose genome comprises an antibody chain locus comprising a germline human kappa V1-5 and germline human kappa J4 sequence, and wherein the antibody is obtainable by in vivo recombination in said mammal of the V1-5 and J4 sequences.
102. A non-human vertebrate or mammal cell (eg, a B-cell or ES cell or hybridoma) whose genome comprises a chimaeric antibody chain locus of statement 100 or 101.
103. A non-human vertebrate or mammal (eg, a mouse or rat) whose genome comprises a chimaeric antibody chain locus of statement 100 or 101.

## Claims

1. A rodent whose genome comprises:
(a) a plurality of human IgH V regions, one or more human D regions and one or more human J regions upstream of the host rodent constant region;
wherein the rodent is able to produce a repertoire of chimaeric antibodies, or chimaeric heavy chains, having a rodent constant region and a human variable region; and
(b) one or more human Ig light chain kappa V regions and one or more human Ig light chain kappa J regions upstream of all or part of the human kappa constant region.

2. A rodent cell whose genome comprises:
(a) a plurality of human IgH V regions, one or more human D regions and one or more human J regions upstream of the host rodent constant region; and
(b) one or more human Ig light chain kappa V regions and one or more human Ig light chain kappa J regions upstream of all or part of the human kappa constant region.

3. A rodent whose genome comprises:
(a) a plurality of human IgH V regions, one or more human D regions and one or more human J regions upstream of the host rodent constant region;
wherein the rodent is able to produce a repertoire of chimaeric antibodies, or chimaeric heavy chains, having a rodent constant region and a human variable region; and
(b) one or more human Ig light chain lambda V regions and one or more human Ig light chain lambda J regions upstream of all or part of the human lambda constant region.

4. A rodent cell whose genome comprises:
(a) a plurality of human IgH V regions, one or more human D regions and one or more human J regions upstream of the host rodent constant region; and
(b) one or more human Ig light chain lambda V regions and one or more human Ig light chain lambda J regions upstream of all or part of the human lambda constant region.

5. The rodent or rodent cell of any preceding claim, wherein the genome comprises an insertion of the complete human light chain VJC region.

6. The rodent or rodent cell of any preceding claim, wherein there is no non-human coding sequence in the inserted light chain region.

7. The rodent or rodent cell of any preceding claim, wherein the VJC light chain DNA is inserted at the equivalent mouse locus.

8. The rodent or rodent cell of claim 1, 2, 5, or 6, wherein the human light chain kappa VJC DNA, or part thereof, is inserted immediately upstream or downstream of the mouse kappa VJC region.

9. The rodent or rodent cell of claim 3, 4, 5, or 6, wherein the human light chain lambda VJC DNA, or part thereof, is inserted immediately upstream or downstream of the mouse lambda VJC region.

10. The rodent or rodent cell of any preceding claim, wherein the rodent host VJC sequences are inactivated.

11. The rodent or rodent cell of any preceding claim, wherein:
(i) one or more rodent control sequences such as the enhancer sequence(s) is maintained upstream of the rodent Mu constant region, suitably in its native position with respect to the distance from the constant region; and/or
(ii) one or more rodent control sequences such as an enhancer sequence(s) are maintained downstream of the rodent Mu constant region, suitably in its native position with respect to the distance from the constant region; and/or
(iii) a rodent switch sequence, suitably the endogenous switch sequence, is maintained upstream of the rodent Mu constant region, suitably in its native position with respect to distance from the constant region.

12. A method for producing an antibody or antibody heavy or light chain specific to a desired antigen, the method comprising immunising a rodent as claimed in any one of claims 1, 3 and 5-11, with the desired antigen and recovering the antibody or antibody chain or recovering a cell producing the antibody or heavy or light chain.

13. A method for producing a fully humanised antibody or antibody chain comprising immunizing a rodent according to the method of claim 12 and then replacing the rodent constant region of an antibody specifically reactive with the antigen with a human constant region, suitably by engineering of the nucleic acid encoding the antibody.

14. A humanised antibody or antibody chain produced according to claim 12 or 13.

15. Use of a humanised antibody or chain produced according to claim 12 or 13 in medicine.

16. A pharmaceutical composition comprising an antibody according to claim 12 or 13 and a pharmaceutically acceptable carrier or other excipient.
